# EUROPEAN PATENT SPECIFICATION

(11) **EP 1 651 640 B1**
(45) Date of publication and mention of the grant of the patent: **14.02.2007**
(21) Application number: 04744026.8
(22) Date of filing: 13.07.2004
(51) Int. Cl.: C07D 409/12, A61P 11/00, A61P 37/00, A61K 31/4436, C07D 409/14, C07D 471/04, C07D 417/14, C07D 413/14

(54) **NICOTINAMIDE DERIVATIVES USEFUL AS PDE4 INHIBITORS**
NICOTINAMID-DERIVATE VERWENDBAR ALS PDE4 INHIBITOREN
DERIVES DE NICOTINAMIDE UTILES EN TANT QU'INHIBITEURS DE PDE4

(30) Priority: 25.07.2003 GB 0317498
(43) Date of publication of application: 03.05.2006
(73) Proprietor: Pfizer Limited, Sandwich, Kent CT13 9NJ (GB); PFIZER INC., New York, N.Y. 10017 (US)
(72) Inventor: BARBER, Christopher Gordon, Sandwich, Kent CT13 9NJ (GB); BUNNAGE, Mark Edward, Sandwich, Kent CT13 9NJ (GB); HARVEY, John Wilson, Sandwich, Kent CT13 9NJ (GB); MATHIAS, John Paul, Sandwich, Kent CT13 9NJ (GB)
(74) Representative: Laurent, Claire
(86) International application number: PCT/IB2004/002367
(87) International publication number: WO 2005/009994

(56) References cited:
- WO-A-01/57036
- DATABASE CHEMCATS Chemical Abstracts Service, Columbus, Ohio, USA; XP002253965 & "Akos (Lithuania)" 1 June 2003 (2003-06-01), AKOS CONSULTING AND SOLUTIONS GMBH , RIEHEN, CH-4125, SWITSERLAND

## Description

This invention relates to nicotinamide derivatives useful as PDE4 inhibitors and to processes for the preparation of, intermediates used in the preparation of, compositions containing and the uses of such derivatives.

The 3',5'-cyclic nucleotide phosphodiesterases (PDEs) comprise a large class of enzymes divided into at least eleven different families which are structurally, biochemically and pharmacologically distinct from one another. The enzymes within each family are commonly referred to as isoenzymes, or isozymes. A total of more than fifteen gene products is included within this class, and further diversity results from differential splicing and post-translational processing of those gene products. The present invention is primarily concerned with the four gene products of the fourth family of PDEs, i.e., PDE4A, PDE4B, PDE4C, and PDE4D. These enzymes are collectively referred to as being isoforms or subtypes of the PDE4 isozyme family.

The PDE4s are characterized by selective, high affinity hydrolytic degradation of the second messenger cyclic nucleotide, adenosine 3',5'-cyclic monophosphate (cAMP), and by sensitivity to inhibition by rolipram. A number of selective inhibitors of the PDE4s have been discovered in recent years, and beneficial pharmacological effects resulting from that inhibition have been shown in a variety of disease models (see, e.g., Torphy *et al., Environ. Health Perspect.* ,1994, 102 Suppl. 10, p. 79-84 ; Duplantier *et al., J. Med. Chem.,* 1996, 39, p. 120-125 ; Schneider *et al., Pharmacol. Biochem. Behav.,* 1995, 50, p. 211-217 ; Banner and Page, *Br. J. Pharmacol.,* 1995, 114, p. 93-98 ; Bamette *et al., J. Pharmacol. Exp. Ther.,* 1995, 273, p. 674-679 ; Wright *et al., Can. J. Physiol. Pharmacol.,* 1997, 75, p. 1001-1008 ; Manabe *et al., Eur. J. Pharmacol.,* 1997, 332, p. 97-107 and Ukita *et al., J. Med. Chem.,* 1999, 42, p. 1088-1099). Accordingly, there continues to be considerable interest in the art with regard to the discovery of further selective inhibitors of PDE4s.

Successful results have already been obtained in the art with the discovery and development of selective PDE4 inhibitors. In vivo, PDE4 inhibitors reduce the influx of eosinophils to the lungs of allergen-challenged animals while also reducing the bronchoconstriction and elevated bronchial responsiveness occurring after allergen challenge. PDE4 inhibitors also suppress the activity of immune cells (including CD4⁺ T-lymphocytes, monocytes, mast cells, and basophils), reduce pulmonary edema, inhibit excitatory nonadrenergic noncholinergic neurotransmission (eNANC), potentiate inhibitory nonadrenergic noncholinergic neurotransmission (iNANC), reduce airway smooth muscle mitogenesis, and induce bronchodilation. PDE4 inhibitors also suppress the activity of a number of inflammatory cells associated with the pathophysiology of COPD, including monocytes/macrophages, CD4⁺ T-lymphocytes, eosinophils and neutrophils. PDE4 inhibitors also reduce vascular smooth muscle mitogenesis and potentially interfere with the ability of airway epithelial cells to generate pro-inflammatory mediators. Through the release of neutral proteases and acid hydrolases from their granules, and the generation of reactive oxygen species, neutrophils contribute to the tissue destruction associated with chronic inflammation, and are further implicated in the pathology of conditions such as emphysema. Therefore, PDE4 inhibitors are particularly useful for the treatment of a great number of inflammatory, respiratory and allergic diseases, disorders or conditions and for wounds and some of them are in clinical development mainly for treatment of asthma, COPD, bronchitis and emphysema.

The effects of PDE4 inhibitors on various inflammatory cell responses can be used as a basis for profiling and selecting inhibitors for further study. These effects include elevation of cAMP and inhibition of superoxide production, degranulation, chemotaxis, and tumor necrosis factor alpha (TNFa) release in eosinophils, neutrophils and monocytes.

Some nicotinamide derivatives having a PDE4 inhibitory activity have already been made. For example, the patent application WO 98/45268 discloses nicotinamide derivatives having activity as selective inhibitors of PDE4D isozyme.

The patent applications WO 01/57036 and WO 03/068235 also disclose nicotinamide derivatives which are PDE4 inhibitors useful in the treatment of various inflammatory allergic and respiratory diseases and conditions.

However, there is still a huge need for additional PDE4 inhibitors that are good drug candidates. In particular, preferred compounds should bind potently to the PDE4 enzyme whilst showing little affinity for other receptors and enzymes. They should also possess favourable pharmacokinetic and metabolic activities, be non-toxic and demonstrate few side effects. Furthermore, it is also desirable that the ideal drug candidate will exist in a physical form that is stable and easily formulated.

The present invention therefore provides new nicotinamide derivatives of formula (I): 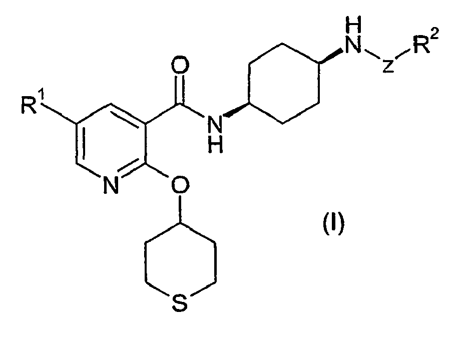 wherein :
R¹ is selected from H, halo and (C₁-C₄)alkyl;
Z is a linker group selected from CO and SO₂;
R² is selected from phenyl, benzyl, naphthyl, heteroaryl and (C₃-C₈)cycloalkyl, each of which is optionally substituted with 1 to 3 substituents each independently selected from halo, CN, CONR³R⁴, (C₁-C₆)alkyl, halo(C₁-C₆)alkyl, OH, hydroxy(C₁-C₆)alkyl, ((C₃-C₈)cycloalkyl)-(C₁-C₆)alkyl, phenyl (optionally substituted by OH and/or halo), (C₃-C₈)cycloalkyl and NR³R⁴; and
R³ and R⁴ are each independently selected from H, (C₁-C₄)alkyl, and SO₂(C₁-C₄)alkyl;
and pharmaceutically acceptable salts and solvates thereof.

In the here above general formula (I), halo denotes a halogen atom selected from the group consisting of fluoro (F), chloro (Cl), bromo (Br) and iodo (I) in particular fluoro or chloro. (C₁-C₄)alkyl or (C₁-C₆)alkyl or (C₂-C₆)alkyl radicals denote a straight-chain or branched group containing respectively 1 to 4 or 1 to 6 or 2 to 6 carbon atoms. This also applies if they carry substituents or occur as substituents of other radicals, for example in (C₁-C₆)alkoxy radicals, hydroxy(C₁-C₆)alkyl, hydroxy(C₂-C₆)alkoxy radicals and halo(C₁-C₆)alkyl radicals. Examples of suitable (C₁-C₄)alkyl and (C₁-C₆)alkyl radicals are methyl, ethyl, n-propyl, iso-propyl, n-butyl, iso-butyl, sec-butyl, tert-butyl, pentyl, hexyl etc. Examples of suitable (C₁-C₆)alkoxy and (C₂-C₆)alkoxy radicals are methoxy, ethoxy, n-propyloxy, iso-propyloxy, n-butyloxy, iso-butyloxy, sec-butyloxy, tert-butyloxy, pentyloxy, hexyloxy etc. Hydroxy(C₁-C₆)alkyl and hydroxy(C₂-C₆)alkoxy radicals may contain more than one hydroxy group (-OH). According to a preferred embodiment of said invention, such radicals contain one hydroxy substituent. Examples of suitable hydroxy(C₁-C₆)alkyl radicals are hydroxymethyl, 1-hydroxyethyl or 2-hydroxyethyl. Accordingly, halo(C₁-C₆)alkyl radicals may contain more than one halo group. According to a preferred embodiment of said invention, such radicals contain 1, 2 or 3 halo substituent. Examples of suitable halo(C₁-C₆)alkyl radicals are difluoromethyl, trifluoromethyl, difluoroethyl or trifluoroethyl.

In the hereabove general formula (I), "heteroaryl" means a monocyclic or polycyclic ring system comprising at least one aromatic ring, having 5 to 14 ring atoms, which ring system contains 1, 2, 3, 4 or 5 ring heteroatom(s) independently selected from N, O and S. Examples of suitable heteroaryl radicals are pyrrole, furan, furazan, thiophene, imidazole, pyrazole, oxazole, isoxazole, thiazole, isothiazole, tetrazole, triazine, pyridine, pyrazine, pyrimidine, pyridazine, indolizine, indole, isoindole, indazole, purine, naphthyridine, phthalazine, quinoline, isoquinoline, quinoxaline, quinazoline, cinnoline, benzofuran, thiadiazole, benzothiadiazole, oxadiazole, benzofuran, dihydrobenzofuran, benzoxadiazole, benzopyrimidine, benzothiophene, benzoxazole, benzothiazole, imidazopyridine, benzimidazole, pyrazolopyridine, pyrazolopyrimidine, etc. including, where a ring nitrogen atom is present, the corresponding N-oxides and quaternary salts.

Finally, (C₃-C₈)cycloalkyl radical means a 3-membered to 8-membered saturated carbocyclic ring. Examples of suitable (C₃-C₈)cycloalkyl radicals are cyclopropyl, cyclobutyl, cyclopentyl, cyclohexyl, cycloheptyl and cyclooctyl

It has been found that these nicotinamide derivatives are inhibitors of PDE4 isoenzymes, particularly useful for the treatment of inflammatory, respiratory and allergic diseases and conditions or for wounds.

In the general formula (I) according to the present invention, when a radical is mono- or poly-substituted, said substituent(s) can be located at any desired and chemically-feasible position(s). Also, when a radical is polysubstituted, said substituents can be identical or different, unless otherwise stated.

Preferably R¹ is H, halo, CH₃ or C₂H₅. More preferably R¹ is H, F, Cl or CH₃. Most preferably R¹ is F.

Preferably R² is selected from the group consisting of phenyl, imidazole, pyrazine, indazole, purine, quinoline, quinazoline, benzofuran, dihydrobenzofuran, benzothiadiazole, benzoxadiazole, pyrazole, imidazopyridine, benzimidazole, pyrazolopyridine, pyrazolopyrimidine, benzyl and cyclopropyl, each of which is optionally substituted with 1 to 3 substituents each independently selected from halo, CN, CONR³R⁴, (C₁-C₆)alkyl, halo(C₁-C₆)alkyl, OH, hydroxy(C₁-C₆)alkyl, ((C₃-C₈)cycloalkyl)-(C₁-C₆)alkyl, phenyl (optionally substituted by OH and/or halo), (C₃-C₈)cycloalkyl and NR³R⁴.

More preferably R² is phenyl, imidazole, indazole, quinoline, quinazoline, dihydrobenzofuran, benzothiadiazole, benzoxadiazole, pyrazole, imidazopyridine, benzimidazole, pyrazolopyridine, benzyl or cyclopropyl, each of which is optionally substituted by one or more substituents selected from CH₃, N(CH₃)SO₂CH₃, NHSO₂CH₂CH₃, NHSO₂CH(CH₃)₂, OH, CH₂OH, Cl, F, C₂H₅, CH(CH₃)₂, C₂H₄OH, CF₃.

Most preferably R² is as defined in the Examples.

Preferably Z is CO.

Preferably the compound is selected from any one of the Examples, or a pharmaceutically acceptable salt or solvate thereof.

Preferred compounds according to the present invention are the nicotinamide derivatives of formula (I) wherein :
R¹ is H, halo, CH₃ or C₂H₅, more preferably R¹ is H, F, Cl or CH₃, and most preferably R¹ is F, and
R² is selected from the group consisting of phenyl, imidazole, pyrazine, indazole, purine, quinoline, quinazoline, benzofuran, dihydrobenzofuran, benzothiadiazole, benzoxadiazole, pyrazole, imidazopyridine, benzimidazole, pyrazolopyridine, pyrazolopyrimidine, benzyl and cyclopropyl,
each of which is optionally substituted with 1 to 3 substituents each independently selected from halo, CN, CONR³R⁴, (C₁-C₆)alkyl, halo(C₁-C₆)alkyl, OH, hydroxy(C₁-C₆)alkyl, ((C₃-C₈)cycloalkyl)-(C₁-C₆)alkyl, phenyl (optionally substituted by OH and/or halo), (C₃-C₈)cycloalkyl and NR³R⁴.

More preferably, the compounds are selected from the nicotinamide derivatives of formula (I) as described in the here above paragraph wherein Z is CO.

Further preferred compounds according to the present invention are the nicotinamide derivatives of formula (I) wherein :
R¹ is H, halo, CH₃ or C₂H₅, more preferably R¹ is H, F, Cl or CH₃, and most preferably R¹ is F, and
R² is phenyl, imidazole, indazole, quinoline, quinazoline, dihydrobenzofuran, benzothiadiazole, benzoxadiazole, pyrazole, imidazopyridine, benzimidazole, pyrazolopyridine, benzyl or cyclopropyl,
each of which is optionally substituted by one or more substituents selected from CH₃, N(CH₃)SO₂CH₃, NHSO₂CH₂CH₃, NHSO₂CH(CH₃)₂, OH, CH₂OH, Cl, F, C₂H₅, CH(CH₃)₂, C₂H₄OH, CF₃.

Still more preferably, the compounds are selected from the nicotinamide derivatives of formula (I) as described in the here above paragraph wherein Z is CO.

Still more preferably, the compound is selected from any one of Examples 10, 11, 12, 13, 14, 15, 19, 20, 22, 23, 25, 27, 29, 33, 35, 37, 38, 41, 42, 43, 44, 45, 46, 51, 52, 53, 54, 57, 59, 60, 63, 64, 66 and 72 or a pharmaceutically acceptable salt or solvate thereof.

Yet more preferably, the compound is selected from any one of Examples 10, 15, 19, 20, 22, 23, 25, 27, 35, 37, 38, 53, 54, 57, 59 and 63, or a pharmaceutically acceptable salt or solvate thereof.

Another most preferred compound is that of Example 63 or a pharmaceutically acceptable salt or solvate thereof.

The nicotinamide derivatives of the formula (I) can be prepared using the Routes disclosed hereunder, and exemplified in the Examples and Preparations, in which the substituents R¹, R² and Z are as previously defined for the nicotinamide derivatives of the formula (I) unless otherwise stated. Other conventional methods may be used in accordance with the skilled person's knowledge.

Unless otherwise provided herein:
PyBOP® means Benzotriazol-1-yloxytris(pyrrolidino)phosphonium hexafluorophosphate;
PyBrOP® means bromo-tris-pyrrolidino-phosphonium hexafluorophosphate; CDI means N,N'-carbonyldiimidazole;
WSCDI means 1-(3-dimethylaminopropyl)-3-ethylcarbodiimide hydrochloride;
Mukaiyama's reagent means 2-chloro-1-methylpyridinium iodide;
HATU means O-(7-Azabenzotriazol-1-yl)-*N,N,N'N'*-tetramethyluronium hexafluorophosphate;
HBTU means *O*-Benzotriazol-1-yl-*N,N,N'N'*-tetramethyluronium hexafluorophosphate;
DCC means N,N'-dicyclohexylcarbodiimide;
CDI means N,N'-carbonyldiimidazole;
HOAT means 1-hydroxy-7-azabenzotriazole;
HOBT means 1-hydroxybenzotriazole hydrate;
Hünig's base means N-ethyldiisopropylamine;
Et₃N means triethylamine;
NMM means N-methylmorpholine;
NMP means 1-methyl-2-pyrrolidinone;
DMAP means 4-dimethylaminopyridine;
NMO means 4-methylmorpholine N-oxide;
KHMDS means potassium bis(trimethylsilyl)amide;
NaHMDS means sodium bis(trimethylsilyl)amide;
DIAD means diisopropyl azodicarboxylate;
DEAD means diethyl azodicarboxylate;
DIBAL means diisobutylammonium hydride;
Dess-Martin periodinane means 1,1,1-triacetoxy-1,1-dihydro-1,2-benziodoxol-3(1H)-one;
TBDMS-Cl means *tert*-butyldimethylchlorosilane;
TMS-Cl means chlorotrimethylsilane;
Boc means *tert*-butoxycarbonyl;
CBz means benzyloxycarbonyl;
MeOH means methanol, EtOH means ethanol, and EtOAc means ethyl acetate;
THF means tetrahydrofuran, DMSO means dimethyl sulphoxide, and DCM means dichloromethane; DMF means N,N-dimethylformamide;
AcOH means acetic acid, TFA means trifluoroacetic acid; rt means room temperature; 3° means tertiary; eq means equivalents; Me means methyl, Et means ethyl, Bn means benzyl; other abbreviations are used in accordance with standard synthetic chemistry practice.

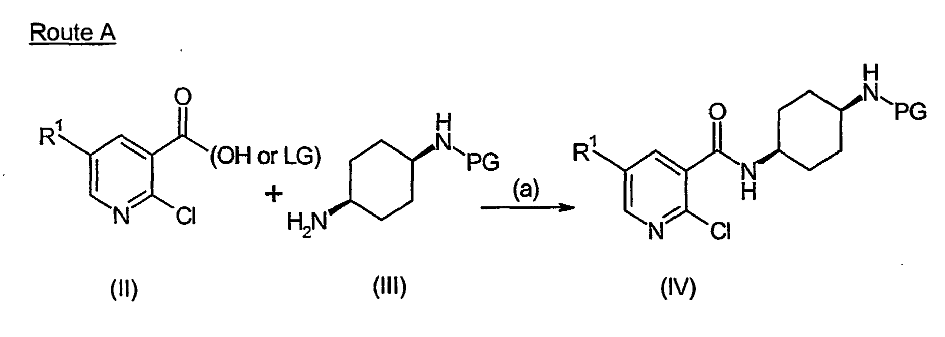

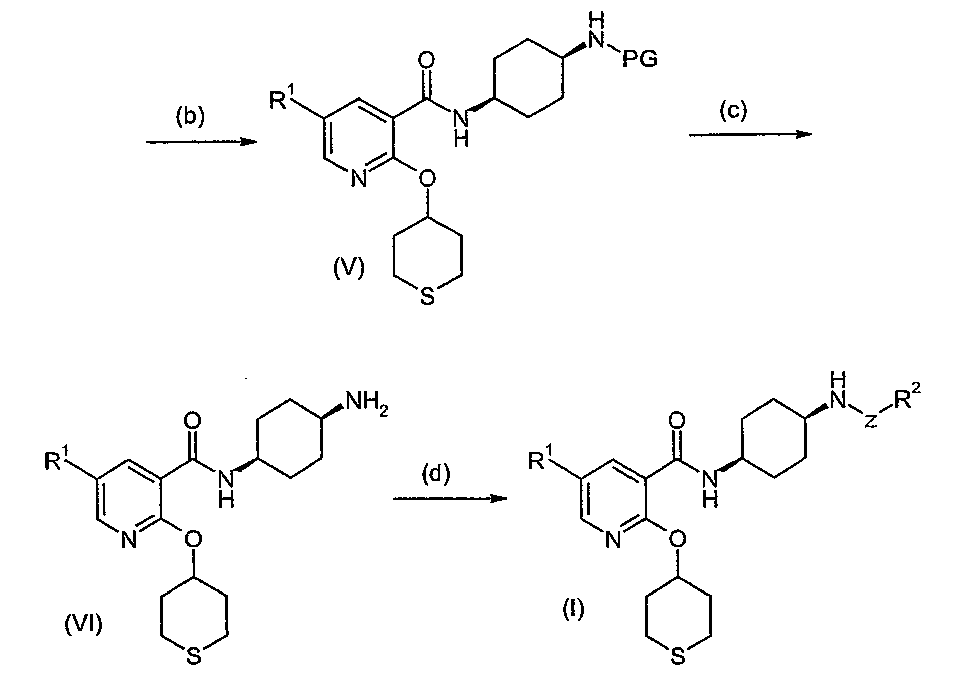

Nicotinic acids of formula (II) are either available commercially or may be obtained by analogy with the methods of Haylor et. al. (EP 0634413); and Marzi et. al. European J. Org. Chem. (2001), (7), 1371-1376.

The protected amines of formula (III) are either available commercially or may be prepared by analogy with the method of Oku et al (WO 99/54284).
In the scheme above, R¹, R² and Z are as previously defined, PG is a suitable amine protecting group, typically Boc, CBz or Bn, and preferably Boc, and LG is a suitable leaving group, typically halo, and preferably Cl.

Step (a)-Acid-amine coupling.
This acid/amine coupling may be undertaken by using either
(i) an acyl chloride derivative of acid (II) + amine (III), with an excess of acid acceptor in a suitable solvent, or
(ii) the acid (II) with a conventional coupling agent + amine (III), optionally in the presence of a catalyst, with an excess of acid acceptor in a suitable solvent.

Typically the conditions are as follows:
(i) acid chloride of acid (II) (generated in-situ), an excess of amine (III), optionally with an excess of 3° amine such as Et₃N, Hünig's base or NMM, in DCM or THF, without heating for 1 to 24 hrs,
   or
(ii) acid (II), WSCDI/DCC/CDI optionally in the presences of HOBT or HOAT, an excess of amine (III), with an excess of NMM, Et₃N, Hünig's base in THF, DCM or EtOAc, at rt. for 4 to 48 hrs; or, acid (II), PYBOP^{®}/PyBrOP^{®}/Mukaiyama's reagent/HATU/HBTU, an excess of amine (III), with an excess of NMM, Et₃N, Hünig's base in THF, DCM or EtOAc, at rt. for 4 to 24 hrs.

The preferred conditions are: acid chloride of acid (II) (generated in-situ), about 1.1 eq amine (III), in DCM at rt. for 18hrs,
Or, acid (II), 1.1 eq amine (III), CDI in DMF at rt. for up to 72 hrs.

### Step (b)-Ether formation

The chloride (IV) is treated with an excess of tetrahydrothiopyran-4-ol, in the presence of a suitable alkali metal base (NaH, K₂CO₃, Cs₂CO₃) in a suitable solvent (eg.MeCN, DMF), optionally in the presence of a catalyst (eg imidazole, DMAP) to provide the ether (V).

The preferred conditions are : chloride (IV), 1.5-2.5 eq tetrahydrothiopyran-4-ol, in the presence of an excess of Cs₂CO₃ in MeCN at about the reflux temperature of the reaction.

### Step (c)-Removal of protecting group

Deprotection of the N protecting group (PG) is undertaken using standard methodology, as described in "Protective Groups in Organic Synthesis" by T.W. Greene and P. Wutz.

When PG is Boc, the preferred conditions are: hydrochloric acid in dioxan and dichloromethane at rt for about 3 hrs.

### Step (d)-Reaction of amino group with Y-Z-R²

Compounds of the formula (I) may be prepared by reaction of amine (VI) with a suitable reagent of formula Y-Z-R², where Y represents OH or Cl.
When Z represents CO, and Y represents OH or Cl, compounds of formula (I) may be prepared by reaction of the amine of formula (VI) with R²CO₂H according to the general methods described previously for step (a).
The preferred conditions are: WSCDI, HOBT, amine (VI), R²CO₂H, an excess of 3° amine base (Hünig's base, Et₃N or NMM) in dichloromethane, N,N-dimethylformamide, NMP or DMA, at rt. for up to 36 hrs, or amine (VI), acid R²CO₂H, HBTU in the presence of an excess of 3° amine base (Hünig's base, Et₃N or NMM) in DMF for up to 24 hrs at rt.

When Z represents SO₂ and Y represents Cl, compounds of formula (I) may be prepared by reaction of the amine of formula (VI) with R²SO₂Cl by analogy with the general methods described in step (a).
The preferred conditions are: WSCDI, HOBT, amine (VI), R²SO₂Cl, an excess of 3° amine base (Hünig's base, Et₃N or NMM) in N,N-dimethylformamide, at rt. for 18 hrs, or amine (IV), R²SO₂Cl in the presence of excess Et₃N in dichloromethane at rt.

Compounds of formula R²ZY, are either commercially available, or may be obtained using standard methodology, or when R² is a heterocycle, by analogy with the methods described in Comprehensive Heterocyclic Chemistry I and II (Elsevier Science Ltd.) and references therein.

Step (d) is exemplified below in Examples 1-51, 54-57, 61-62 and 64-70.

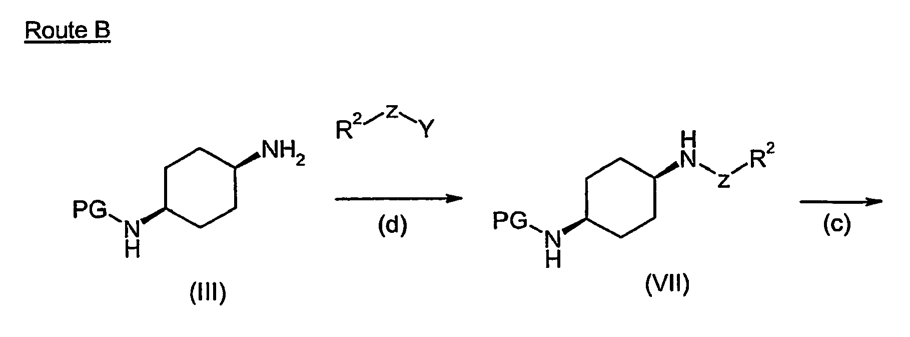

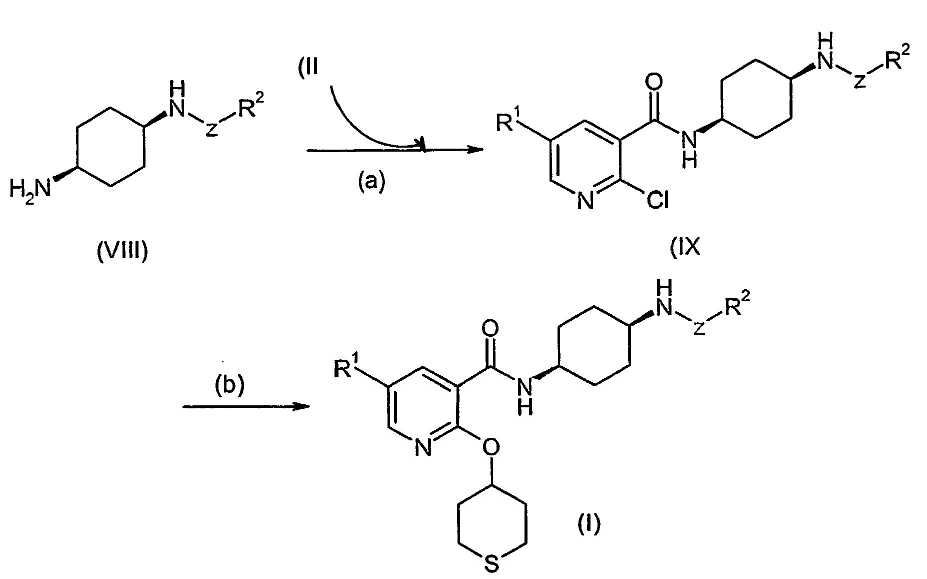

The compound of formula (VII) may be prepared from the amine (III) by reaction with R²ZY according to the methods described previously in step (d), Route A.
The compound of formula (VIII) may be prepared from the compound of formula (VII) by analogy to the methods described previously in step (c), Route A.
Compounds of formula (IX) may be prepared by reaction of the amine of formula (VIII) with the acid or acid derivative (II) according to the methods described previously in step (a), Route A.
Compounds of formula (I) may be prepared by reaction of compounds of formula (IX) with tetrahydrothipyran-4-ol as described previously in step (b), Route A.

The transformation (IX) to (I) is exemplified by Example 63.

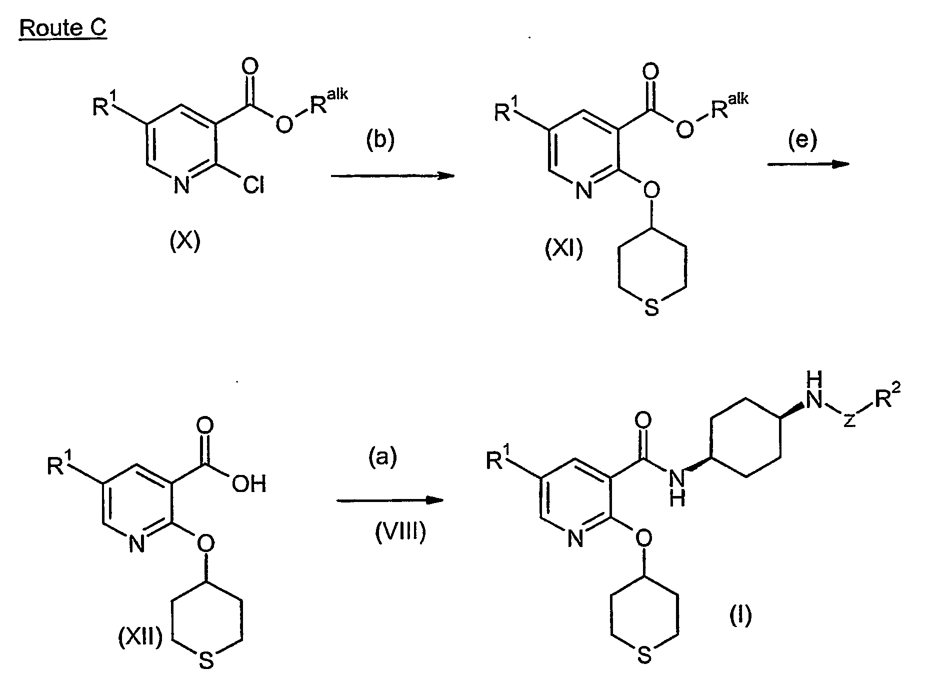

R^{alk} represents a C₁-C₄ alkyl group, preferably Me or Et.
Compounds of formula (X) are either available commercially or may be obtained from the compounds of formula (II), using standard esterification conditions.
Compounds of formula (XI) may be prepared by reaction of the ester (X) with tetrahydrothiopyran-4-ol, as described previously in step (b), Route A.

### Step (e)-Ester hydrolysis

Hydrolysis of the ester (XI) may be achieved in the presence of acid or base, in a suitable solvent, optionally at elevated temperature to afford the acid (XII).
Typically, the ester (XI) is treated with an alkali metal hydroxide (eg Li, Na, Cs) in aqueous solvent (MeOH, EtOH, dioxan, THF) at between rt and the reflux temperature of the reaction, to give the acid of formula (XII)

Reaction of the acid (XII) with the amine (VIII) as described previously in step (a) provides the compounds of formula (I).

### Further Routes

Certain R² groups may undergo further functional group interconversions (FGls) and transformations, such as alkylation of a phenol hydroxy group, using a suitable alkylbromide, in the presence of a suitable alkali metal base (such as K₂CO₃), optionally in the presence of a catalyst (eg Kl) in a suitable solvent such as acetonitrile and/or N,N-dimethylformamide at elevated temperature, or demethylation of a methoxy group by treatment with lithium iodide in pyridine or collidine, or by treatment with BBr₃ in dichloromethane (see ex 71-75).

For certain compounds of the description, a suitable protecting group strategy may be employed. For example, a hydroxyl group may be protected using a tetrahydropyran group, and deprotection may be achieved by treatment with a solution of acetic acid:water:tetrahydrofuran (4:1:2 by volume) at rt. for upto 18 hrs. (see e.g. Examples 52 to 57). Further, a benzyloxy group may be used and deprotected to give the corresponding hydroxyl compound, for example by using a reduction (e.g. with palladium black in acid).

FGI and protection/deprotection strategies are exemplified in Examples 52-53, 58-59 and 71-75.

All of the above reactions and the preparations of novel starting materials used in the preceding methods are conventional and appropriate reagents and reaction conditions for their performance or preparation as well as procedures for isolating the desired products will be well-known to those skilled in the art with reference to literature precedents and the examples and preparations hereto.

As mentioned above, use of protection/deprotection strategies are needed in some instances. Methods such as those described by T.W. GREENE (*Protective Groups in Organic Synthesis,* A. Wiley-Interscience Publication, 1981) or by McOMIE (*Protective Groups in Organic Chemistry,* Plenum Press, 1973), can be used.

Compounds of formula (I), as well as intermediate for the preparation thereof can be purified according to various well-known methods, such as for example crystallization or chromatography.

The nicotinamide derivatives of formula (I) may also be optionally transformed in pharmaceutically acceptable salts. In particular, these pharmaceutically acceptable salts of the nicotinamide derivatives of the formula (I) include the acid addition and the base salts (including disalts) thereof.

Suitable acid addition salts are formed from acids which form non-toxic salts. Examples include the acetate, aspartate, benzoate, besylate, bicarbonate/carbonate, bisulphate, camsylate, citrate, edisylate, esylate, fumarate, gluceptate, gluconate, glucuronate, hibenzate, hydrochloride/chloride, hydrobromide/bromide, hydroiodide/iodie, hydrogen phosphate, isethionate, D- and L-lactate, malate, maleate, malonate, mesylate, methylsulphate, 2-napsylate, nicotinate, nitrate, orotate, palmoate, phosphate, saccharate, stearate, succinate sulphate, D- and L-tartrate, 1-hydroxy-2-naphtoate, 3-hydroxy-2-naphthoate and tosylate saltes.

Suitable base salts are formed from bases which form non-toxic salts. Examples include the aluminium, arginine, benzathine, calcium, choline, diethylamine, diolamine, glycine, lysine, magnesium, meglumine, olamine, potassium, sodium, tromethamine and zinc salts.

For a review on suitable salts, see Stahl and Wermuth, Handbook of Pharmaceutical Salts: Properties, Selection and Use, Wiley-VCH, Weinheim, Germany (2002).

A pharmaceutically acceptable salt of a nicotinamide derivative of the formula (I) may be readily prepared by mixing together solutions of the nicotinamide derivative of formula (I) and the desired acid or base, as appropriate. The salt may precipitate from solution and be collected by filtration or may be recovered by evaporation of the solvent.

Pharmaceutically acceptable solvates in accordance with the invention include hydrates and solvates wherein the solvent of crystallization may be isotopically substituted, e.g. D₂O, d₆-acetone, d₆-DMSO.

Clathrates are drug-host inclusion complexes wherein, in contrast to the aforementioned solvates, the drug and host are present in non-stoichiometric amounts. For a review of such complexes, see J Pharm Sci, 64 (8), 1269-1288 by Haleblian (August 1975).

Hereinafter all references to nicotinamide derivatives of formula (I) include references to salts thereof and to solvates and clathrates of compounds of formula (I) and salts thereof.

The invention includes all polymorphs of the nicotinamide derivatives of formula (I).

Thus certain derivatives of nicotinamide derivatives of formula (I) which have little or no pharmacological activity themselves can, when metabolised upon administration into or onto the body, give rise to nicotinamide derivatives of formula (I) having the desired activity. Such derivatives are referred to as "prodrugs".

Prodrugs in accordance with the invention can, for example, be produced by replacing appropriate functionalities present in the nicotinamide derivatives of formula (I) with certain moieties known to those skilled in the art as "pro-moieties" as described, for example, in "Design of Prodrugs" by H Bundgaard (Elsevier, 1985).

Finally, certain nicotinamide derivatives of formula (I) may themselves act as prodrugs of other nicotinamide derivatives of formula (I).

Nicotinamide derivatives of formula (I) containing one or more asymmetric carbon atoms can exist as two or more optical isomers. Where a nicotinamide derivative of formula (I) contains an alkenyl or alkenylene group, geometric cis/trans (or Z/E) isomers are possible, and where the nicotinamide derivative contains, for example, a keto or oxime group, tautomeric isomerism ('tautomerism') may occur. It follows that, unless otherwise defined, a single nicotinamide derivative may exhibit more than one type of isomerism.

Included within the scope of the present invention are all optical isomers, geometric isomers and tautomeric forms of the nicotinamide derivatives of formula (I), including compounds exhibiting more than one type of isomerism, and mixtures of one or more thereof.

Cis/trans isomers may be separated by conventional techniques well known to those skilled in the art, for example, fractional crystallisation and chromatography.

Conventional techniques for the preparation/isolation of individual stereoisomers include the conversion of a suitable optically pure precursor, resolution of the racemate (or the racemate of a salt or derivative) using, for example, chiral HPLC, or fractional crystallisation of diastereoisomeric salts formed by reaction of the racemate with a suitable optically active acid or base, for example, tartaric acid.

The present invention also includes all pharmaceutically acceptable isotopic variations of a nicotinamide derivative of formula (I). An isotopic variation is defined as one in which at least one atom is replaced by an atom having the same atomic number, but an atomic mass different from the atomic mass usually found in nature.

Examples of isotopes suitable for inclusion in the nicotinamide derivatives of the invention include isotopes of hydrogen, such as ²H and ³H, carbon, such as ¹³C and ¹⁴C, nitrogen, such as ¹⁵N, oxygen, such as ¹⁷O and ¹⁸O, phosphorus, such as ³²P, sulphur, such as ³⁵S, fluorine, such as ¹⁸F, and chlorine, such as ³⁶Cl.

Substitution of the nicotinamide derivative of formula (I) isotopes such as deuterium, i.e. ²H, may afford certain therapeutic advantages resulting from greater metabolic stability, for example, increased in vivo half-life or reduced dosage requirements, and hence may be preferred in some circumstances.

Certain isotopic variations of the nicotinamide derivatives of formula (I), for example, those incorporating a radioactive isotope, are useful in drug and/or substrate tissue distribution studies. The radioactive isotopes tritium, i.e. ³H, and carbon-14, i.e. ¹⁴C, are particularly useful for this purpose in view of their ease of incorporation and ready means of detection.

Isotopic variations of the nicotinamide derivatives of formula (I) can generally be prepared by conventional techniques known to those skilled in the art or by processes analogous to those described in the accompanying Examples and Preparations using appropriate isotopic variations of suitable reagents.

According to a further aspect, the present invention concerns mixtures of nicotinamide derivatives of the formula (I), as well as mixtures with or of their pharmaceutically acceptable salts, solvates, polymorphs, isomeric forms and/or isotope forms.

According to the present invention, all the here above mentioned forms of the nicotinamide derivatives of formula (I) except the pharmaceutically acceptable salts (i.e. said solvates, polymorphs, isomeric forms and isotope forms), are defined as "derived forms" of the nicotinamide derivatives of formula (I) in what follows.

The nicotinamide derivatives of formula (I), their pharmaceutically acceptable salts and/or derived forms, are valuable pharmaceutical active compounds, which are suitable for the therapy and prophylaxis of numerous disorders in which the PDE4 enzymes are involved, in particular the inflammatory disorders, allergic disorders, respiratory diseases and wounds.

The nicotinamide derivatives of formula (I) and their pharmaceutically acceptable salts and derived forms as mentioned above can be administered according to the invention to animals, preferably to mammals, and in particular to humans, as pharmaceuticals for therapy or prophylaxis. They can be administered per se, in mixtures with one another or in combination with other drugs, or in the form of pharmaceutical preparations which permit enteral (gastric) or parenteral (non-gastric) administration and which as active constituent contain an efficacious dose of at least one nicotinamide derivative of the formula (I), its pharmaceutically acceptable salts and/or derived forms, in addition to customary pharmaceutically innocuous excipients and/or additives. The term "excipient" is used herein to describe any ingredient other than the compound of the invention. The choice of excipient will to a large extent depend on the particular mode of administration.

The nicotinamide derivatives of formula (I), their pharmaceutically acceptable salts and/or derived forms may be freeze-dried, spray-dried, or evaporatively dried to provide a solid plug, powder, or film of crystalline or amorphous material. Microwave or radio frequency drying may be used for this purpose.

### ORAL ADMINISTRATION

The nicotinamide derivatives of formula (I) their pharmaceutically acceptable salts and/or derived forms of the invention may be administered orally. Oral administration may involve swallowing, so that the compound enters the gastrointestinal tract, or buccal or sublingual administration may be employed by which the compound enters the blood stream directly from the mouth.

Formulations suitable for oral administration include solid formulations such as tablets, capsules containing particulates, liquids, or powders, lozenges (including liquid-filled), chews, multi- and nano-particulates, gels, films (including muco-adhesive), ovules, sprays and liquid formulations.

Liquid formulations include suspensions, solutions, syrups and elixirs. Such formulations may be employed as fillers in soft or hard capsules and typically comprise a carrier, for example, water, ethanol, propylene glycol, methylcellulose, or a suitable oil, and one or more emulsifying agents and/or suspending agents. Liquid formulations may also be prepared by the reconstitution of a solid, for example, from a sachet.

The nicotinamide derivatives of formula (I), their pharmaceutically acceptable salts and/or derived forms of the invention may also be used in fast-dissolving, fast-disintegrating dosage forms such as those described in Expert Opinion in Therapeutic Patents, 11 (6), 981-986 by Liang and Chen (2001).

The composition of a typical tablet in accordance with the invention may comprise:

| **Ingredient** | **% w/w** |
|---|---|
| Nicotinamide derivative of formula (I) | 10.00* |
| Microcrystalline cellulose | 64.12 |
| Lactose | 21.38 |
| Croscarmellose sodium | 3.00 |
| Magnesium stearate | 1.50 |

| | |
|---|---|
| * Quantity adjusted in accordance with drug activity. | |

A typical tablet may be prepared using standard processes known to a formulation chemist, for example, by direct compression, granulation (dry, wet, or melt), melt congealing, or extrusion. The tablet formulation may comprise one or more layers and may be coated or uncoated.

Examples of excipients suitable for oral administration include carriers, for example, cellulose, calcium carbonate, dibasic calcium phosphate, mannitol and sodium citrate, granulation binders, for example, polyvinylpyrrolidine, hydroxypropylcellulose, hydroxypropylmethylcellulose and gelatin, disintegrants, for example, sodium starch glycolate and silicates, lubricating agents, for example, magnesium stearate and stearic acid, wetting agents, for example, sodium lauryl sulphate, preservatives, antioxidants, flavours and colourants.

Solid formulations for oral administration may be formulated to be immediate and/or modified release. Modified release formulations include delayed-, sustained-, pulsed-, controlled dual-, targeted and programmed release. Details of suitable modified release technologies such as high energy dispersions, osmotic and coated particles are to be found in Verma et al, Pharmaceutical Technology On-line, 25(2), 1-14 (2001). Other modified release formulations are described in US Patent No. 6,106,864.

### PARENTERAL ADMINISTRATION

The nicotinamide derivatives of formula (I), their pharmaceutically acceptable salts and/or derived forms of the invention may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

Parenteral formulations are typically aqueous solutions which may contain excipients such as salts, carbohydrates and buffering agents (preferably to a pH of from 3 to 9), but, for some applications, they may be more suitably formulated as a sterile nonaqueous solution or as a dried form to be used in conjunction with a suitable vehicle such as sterile, pyrogen-free water.

The preparation of parenteral formulations under sterile conditions, for example, by lyophilisation, may readily be accomplished using standard pharmaceutical techniques well known to those skilled in the art.

The solubility of nicotinamide derivatives of formula (I) used in the preparation of parenteral solutions may be increased by suitable processing, for example, the use of high energy spray-dried dispersions (see WO 01/47495) and/or by the use of appropriate formulation techniques, such as the use of solubility-enhancing agents.

Formulations for parenteral administration may be formulated to be immediate and/or modified release. Modified release formulations include delayed-, sustained-, pulsed-, controlled dual-, targeted and programmed release.

### TOPICAL ADMINISTRATION

The nicotinamide derivatives of the invention may also be administered topically to the skin or mucosa, either dermally or transdermally. Typical formulations for this purpose include gels, hydrogels, lotions, solutions, creams, ointments, dusting powders, dressings, foams, films, skin patches, wafers, implants, sponges, fibres, bandages and microemulsions. Liposomes may also be used. Typical carriers include alcohol, water, mineral oil, liquid petrolatum, white petrolatum, glycerin and propylene glycol. Penetration enhancers may be incorporated - see, for example, J Pharm Sci, 88 (10), 955-958 by Finnin and Morgan (October 1999).

Other means of topical administration include delivery by iontophoresis, electroporation, phonophoresis, sonophoresis and needle-free or microneedle injection.

Formulations for topical administration may be formulated to be immediate and/or modified release. Modified release formulations include delayed-, sustained-, pulsed-, controlled dual-, targeted and programmed release. Thus nicotinamide derivatives of formula (I) may be formulated in a more solid form for administration as an implanted depot providing long-term release of the active compound.

### INHALED/INTRANASAL ADMINISTRATION

The nicotinamide derivatives of formula (I) can also be administered intranasally or by inhalation, typically in the form of a dry powder (either alone, as a mixture, for example, in a dry blend with lactose in anhydrous or monohydrate form, preferably monohydrate, mannitol, dextran, glucose, maltose, sorbitol, xylitol, fructose, sucrose or trehalose, or as a mixed component particle, for example, mixed with phospholipids) from a dry powder inhaler or as an aerosol spray from a pressurised container, pump, spray, atomiser (preferably an atomiser using electrohydrodynamics to produce a fine mist), or nebuliser, with or without the use of a suitable propellant, such as dichlorofluoromethane.

The pressurised container, pump, spray, atomizer, or nebuliser contains a solution or suspension of the active compound comprising, for example, ethanol (optionally, aqueous ethanol) or a suitable alternative agent for dispersing, solubilising, or extending release of the active, the propellant(s) as solvent and an optional surfactant, such as sorbitan trioleate or an oligolactic acid.

Prior to use in a dry powder or suspension formulation, the drug product is micronised to a size suitable for delivery by inhalation (typically less than 5 microns). This may be achieved by any appropriate comminuting method, such as spiral jet milling, fluid bed jet milling, supercritical fluid processing to form nanoparticles, high pressure homogenisation, or spray drying.

A suitable solution formulation for use in an atomiser using electrohydrodynamics to produce a fine mist may contain from 1µg to 20mg of the nicotinamide derivative of formula (I) per actuation and the actuation volume may vary from 1µl to 100µl. A typical formulation may comprise a nicotinamide derivative of formula (I), propylene glycol, sterile water, ethanol and sodium chloride. Alternative solvents which may be used instead of propylene glycol include glycerol and polyethylene glycol.

Capsules, blisters and cartridges (made, for example, from gelatin or HPMC) for use in an inhaler or insufflator may be formulated to contain a powder mix of the nicotinamide derivative of formula (I), a suitable powder base such as lactose or starch and a performance modifier such as I-leucine, mannitol, or magnesium stearate.

In the case of dry powder inhalers and aerosols, the dosage unit is determined by means of a valve which delivers a metered amount. Units in accordance with the invention are typically arranged to administer a metered dose or "puff" containing from 1 µg to 4000 µg of the nicotinamide derivative of formula (I). The overall daily dose will typically be in the range 1 µg to 20 mg which may be administered in a single dose or, more usually, as divided doses throughout the day.

Formulations for inhaled/intranasal administration may be formulated to be immediate and/or modified release. Modified release formulations include delayed-, sustained-, pulsed-, controlled dual-, targeted and programmed release. Sustained or controlled release can be obtained by using for example poly(D,L-lactic-co-glycolic acid).

Flavouring agents, such as methol and levomethol and/or sweeteners such as saccharing or saccharin sodium can be added to the formulation.

According to a preferred aspect, the nicotinamide derivatives of formula (I) of the present invention are administered intranasally or by inhalation.

### RECTAL/INTRAVAGINAL ADMINISTRATION

The nicotinamide derivatives of formula (I) may be administered rectally or vaginally, for example, in the form of a suppository, pessary, or enema. Cocoa butter is a traditional suppository base, but various alternatives may be used as appropriate.

Formulations for rectal/vaginal administration may be formulated to be immediate and/or modified release. Modified release formulations include delayed-, sustained-, pulsed-, controlled dual-, targeted and programmed release.

### OCULAR/ANDIAL ADMINISTRATION

The nicotinamide derivatives of formula (I) may also be administered directly to the eye or ear, typically in the form of drops of a micronised suspension or solution in isotonic, pH-adjusted, sterile saline. Other formulations suitable for ocular and andial administration include ointments, biodegradable (e.g. absorbable gel sponges, collagen) and non-biodegradable (e.g. silicone) implants, wafers, lenses and particulate or vesicular systems, such as niosomes or liposomes. A polymer such as crossed-linked polyacrylic acid, polyvinylalcohol, hyaluronic acid, a cellulosic polymer, for example, hydroxypropylmethylcellulose, hydroxyethylcellulose, or methyl cellulose, or a heteropolysaccharide polymer, for example, gelan gum, may be incorporated together with a preservative, such as benzalkonium chloride. Such formulations may also be delivered by iontophoresis.

Formulations for ocular/andial administration may be formulated to be immediate and/or modified release. Modified release formulations include delayed-, sustained-, pulsed-, controlled dual-, targeted, or programmed release.

### ENABLING TECHNOLOGIES

The nicotinamide derivatives of formula (I) may be combined with soluble macromolecular entities such as cyclodextrin or polyethylene glycol-containing polymers to improve their solubility, dissolution rate, taste-masking, bioavailability and/or stability.

Drug-cyclodextrin complexes, for example, are found to be generally useful for most dosage forms and administration routes. Both inclusion and non-inclusion complexes may be used. As an alternative to direct complexation with the drug, the cyclodextrin may be used as an auxiliary additive, i.e. as a carrier, diluent, or solubiliser. Most commonly used for these purposes are alpha-, beta- and gamma-cyclodextrins, examples of which may be found in International Patent Applications Nos. WO 91/11172, WO 94/02518 and WO 98/55148.

### DOSAGE

For administration to human patients, the total daily dose of the nicotinamide derivatives of formula (I) is typically in the range 0.001 mg/kg to 100 mg/kg depending, of course, on the mode of administration. The total daily dose may be administered in single or divided doses. The physician will readily be able to determine doses for subjects depending on age, weight, health state and sex or the patient as well as the severity of the disease.

According to another embodiment of the present invention, the nicotinamide derivatives of the formula (I), their pharmaceutically acceptable salts and/or their derived forms, can also be used as a combination with one or more additional therapeutic agents to be co-administered to a patient to obtain some particularly desired therapeutic end result. The second and more additional therapeutic agents may also be a nicotinamide derivatives of the formula (I), their pharmaceutically acceptable salts and/or their derived forms, or one or more PDE4 inhibitors known in the art. More typically, the second and more therapeutic agents will be selected from a different class of therapeutic agents.

As used herein, the terms "co-administration", "co-administered" and "in combination with", referring to the nicotinamide derivatives of formula (I) and one or more other therapeutic agents, is intended to mean, and does refer to and include the following :
■ simultaneous administration of such combination of nicotinamide derivative(s) and therapeutic agent(s) to a patient in need of treatment, when such components are formulated together into a single dosage form which releases said components at substantially the same time to said patient,
■ substantially simultaneous administration of such combination of nicotinamide derivative(s) and therapeutic agent(s) to a patient in need of treatment, when such components are formulated apart from each other into separate dosage forms which are taken at substantially the same time by said patient, whereupon said components are released at substantially the same time to said patient,
■ sequential administration of such combination of nicotinamide derivative(s) and therapeutic agent(s) to a patient in need of treatment, when such components are formulated apart from each other into separate dosage forms which are taken at consecutive times by said patient with a significant time interval between each administration, whereupon said components are released at substantially different times to said patient; and
■ sequential administration of such combination of nicotinamide derivative(s) and therapeutic agent(s) to a patient in need of treatment, when such components are formulated together into a single dosage form which releases said components in a controlled manner whereupon they are concurrently, consecutively, and/or overlappingly administered at the same and/or different times by said patient.

Suitable examples of other therapeutic agents which may be used in combination with the nicotinamide derivatives of the formula (I), their pharmaceutically acceptable salts and/or their derived forms include, but are by no mean limited to :
(a) 5-Lipoxygenase (5-LO) inhibitors or 5-lipoxygenase activating protein (FLAP) antagonists,
(b) Leukotriene antagonists (LTRAs) including antagonists of LTB4, LTC4, LTD4, and LTE4,
(c) Histaminic receptor antagonists including H1, H3 and H4 antagonists,
(d) α1- and α2-adrenoceptor agonist vasoconstrictor sympathomimetic agents for decongestant use,
(e) Muscarinic M3 receptor antagonists or anticholinergic agents,
(f) β2-adrenoceptor agonists,
(g) Theophylline,
(h) Sodium cromoglycate,
(i) COX-1 inhibitors (NSAIDs) and COX-2 selective inhibitors,
(j) Oral or inhaled Glucocorticosteroids,
(k) Monoclonal antibodies active against endogenous inflammatory entities,
(l) Anti-tumor necrosis factor (anti-TNF-a) agents,
(m) Adhesion molecule inhibitors including VLA-4 antagonists,
(n) Kinin-B1 - and B2 -receptor antagonists,
(o) Immunosuppressive agents,
(p) Inhibitors of matrix metalloproteases (MMPs),
(q) Tachykinin NK1, NK2 and NK3 receptor antagonists,
(r) Elastase inhibitors,
(s) Adenosine A2a receptor agonists,
(t) Inhibitors of urokinase,
(u) Compounds that act on dopamine receptors, e.g. D2 agonists,
(v) Modulators of the NFkb pathway, e.g. IKK inhibitors,
(w) Agents that can be classed as mucolytics or anti-tussive,
(x) antibiotics, and
(y) p38 MAP kinase inhibitors

According to the present invention, combination of the nicotinamide derivatives of formula (I) with:
■ muscarinic M3 receptor agonists or anticholinergic agents including in particular ipratropium salts, namely bromide, tiotropium salts, namely bromide, oxitropium salts, namely bromide, perenzepine, and telenzepine,
■ β2-adrenoceptor agonists including albutarol, salbutamol, formoterol and salmeterol,
■ p38 MAP kinase inhibitors,
■ H3 anatgonists,
■ glucocorticosteroids, in particular inhaled glucocorticosteroids with reduced systemic side effects, including prednisone, prednisolone, flunisolide, triamcinolone acetonide, beclomethasone dipropionate, budesonide, fluticasone propionate, and mometasone furoate,
■ or adenosine A2a receptor agonists,
are preferred.

It is to be appreciated that all references herein to treatment include curative, palliative and prophylactic treatment. The description which follows concerns the therapeutic applications to which the nicotinamide derivatives of formula (I) may be put.

The nicotinamide derivatives of formula (I) inhibit the PDE4 isozyme and thereby have a wide range of therapeutic applications, as described further below, because of the essential role, which the PDE4 family of isozymes plays in the physiology of all mammals. The enzymatic role performed by the PDE4 isozymes is the intracellular hydrolysis of adenosine 3',5'-monophosphate (cAMP) within pro-inflammatory leukocytes. cAMP, in turn, is responsible for mediating the effects of numerous hormones in the body, and as a consequence, PDE4 inhibition plays a significant role in a variety of physiological processes. There is extensive literature in the art describing the effects of PDE inhibitors on various inflammatory cell responses, which in addition to cAMP increase, include inhibition of superoxide production, degranulation, chemotaxis and tumor necrosis factor (TNF) release in eosinophils, neutrophils and monocytes.

Therefore, a further aspect of the present invention relates to the use of the nicotinamide derivatives of formula (I), their pharmaceutically acceptable salts and/or derived forms, in the treatment of diseases, disorders, and conditions in which the PDE4 isozymes are involved. More specifically, the present invention also concerns the use of the nicotinamide derivatives of formula (I), their pharmaceutically acceptable salts and/or derived forms, in the treatment of diseases, disorders, and conditions selected from the group consisting of :
■ asthma of whatever type, etiology, or pathogenesis, in particular asthma that is a member selected from the group consisting of atopic asthma, non-atopic asthma, allergic asthma, atopic bronchial IgE-mediated asthma, bronchial asthma, essential asthma, true asthma, intrinsic asthma caused by pathophysiologic disturbances, extrinsic asthma caused by environmental factors, essential asthma of unknown or inapparent cause, non-atopic asthma, bronchitic asthma, emphysematous asthma, exercise-induced asthma, allergen induced asthma, cold air induced asthma, occupational asthma, infective asthma caused by bacterial, fungal, protozoal, or viral infection, non-allergic asthma, incipient asthma and wheezy infant syndrome,
■ chronic or acute bronchoconstriction, chronic bronchitis, small airways obstruction, and emphysema,
■ obstructive or inflammatory airways diseases of whatever type, etiology, or pathogenesis, in particular an obstructive or inflammatory airways disease that is a member selected from the group consisting of chronic eosinophilic pneumonia, chronic obstructive pulmonary disease (COPD), COPD that includes chronic bronchitis, pulmonary emphysema or dyspnea associated therewith, COPD that is characterized by irreversible, progressive airways obstruction, adult respiratory distress syndrome (ARDS) and exacerbation of airways hyper-reactivity consequent to other drug therapy
■ pneumoconiosis of whatever type, etiology, or pathogenesis, in particular pneumoconiosis that is a member selected from the group consisting of aluminosis or bauxite workers' disease, anthracosis or miners' asthma, asbestosis or steam-fitters' asthma, chalicosis or flint disease, ptilosis caused by inhaling the dust from ostrich feathers, siderosis caused by the inhalation of iron particles, silicosis or grinders' disease, byssinosis or cotton-dust asthma and talc pneumoconiosis;
■ bronchitis of whatever type, etiology, or pathogenesis, in particular bronchitis that is a member selected from the group consisting of acute bronchitis, acute laryngotracheal bronchitis, arachidic bronchitis, catarrhal bronchitis, croupus bronchitis, dry bronchitis, infectious asthmatic bronchitis, productive bronchitis, staphylococcus or streptococcal bronchitis and vesicular bronchitis,
■ bronchiectasis of whatever type, etiology, or pathogenesis, in particular bronchiectasis that is a member selected from the group consisting of cylindric bronchiectasis, sacculated bronchiectasis, fusiform bronchiectasis, capillary bronchiectasis, cystic bronchiectasis, dry bronchiectasis and follicular bronchiectasis,
■ seasonal allergic rhinitis or perennial allergic rhinitis or sinusitis of whatever type, etiology, or pathogenesis, in particular sinusitis that is a member selected from the group consisting of purulent or nonpurulent sinusitis, acute or chronic sinusitis and ethmoid, frontal, maxillary, or sphenoid sinusitis,
■ rheumatoid arthritis of whatever type, etiology, or pathogenesis, in particular rheumatoid arthritis that is a member selected from the group consisting of acute arthritis, acute gouty arthritis, chronic inflammatory arthritis, degenerative arthritis, infectious arthritis, Lyme arthritis, proliferative arthritis, psoriatic arthritis and vertebral arthritis,
■ gout, and fever and pain associated with inflammation,
■ an eosinophil-related disorder of whatever type, etiology, or pathogenesis, in particular an eosinophil-related disorder that is a member selected from the group consisting of eosinophilia, pulmonary infiltration eosinophilia, Loffler's syndrome, chronic eosinophilic pneumonia, tropical pulmonary eosinophilia, bronchopneumonic aspergillosis, aspergilloma, granulomas containing eosinophils, allergic granulomatous angiitis or Churg-Strauss syndrome, polyarteritis nodosa (PAN) and systemic necrotizing vasculitis,
■ atopic dermatitis, allergic dermatitis, contact dermatitis, or allergic or atopic eczema,
■ urticaria of whatever type, etiology, or pathogenesis, in particular urticaria that is a member selected from the group consisting of immune-mediated urticaria, complement-mediated urticaria, urticariogenic material-induced urticaria, physical agent-induced urticaria, stress-induced urticaria, idiopathic urticaria, acute urticaria, chronic urticaria, angioedema, cholinergic urticaria, cold urticaria in the autosomal dominant form or in the acquired form, contact urticaria, giant urticaria and papular urticaria,
■ conjunctivitis of whatever type, etiology, or pathogenesis, in particular conjunctivitis that is a member selected from the group consisting of actinic conjunctivitis, acute catarrhal conjunctivitis, acute contagious conjunctivitis, allergic conjunctivitis, atopic conjunctivitis, chronic catarrhal conjunctivitis, purulent conjunctivitis and vernal conjunctivitis,
■ uveitis of whatever type, etiology, or pathogenesis, in particular uveitis that is a member selected from the group consisting of inflammation of all or part of the uvea, anterior uveitis, iritis, cyclitis, iridocyclitis, granulomatous uveitis, nongranulomatous uveitis, phacoantigenic uveitis, posterior uveitis, choroiditis; and chorioretinitis,
■ psoriasis;
■ multiple sclerosis of whatever type, etiology, or pathogenesis, in particular multiple sclerosis that is a member selected from the group consisting of primary progressive multiple sclerosis and relapsing remitting multiple sclerosis,
■ autoimmune/inflammatory diseases of whatever type, etiology, or pathogenesis, in particular an autoimmune/inflammatory disease that is a member selected from the group consisting of autoimmune hematological disorders, hemolytic anemia, aplastic anemia, pure red cell anemia, idiopathic thrombocytopenic purpura, systemic lupus erythematosus, polychondritis, scleroderma, Wegner's granulomatosis, dermatomyositis, chronic active hepatitis, myasthenia gravis, Stevens-Johnson syndrome, idiopathic sprue, autoimmune inflammatory bowel diseases, ulcerative colitis, endocrin opthamopathy, Grave's disease, sarcoidosis, alveolitis, chronic hypersensitivity pneumonitis, primary biliary cirrhosis, juvenile diabetes or diabetes mellitus type I, keratoconjunctivitis sicca, epidemic keratoconjunctivitis, diffuse interstitial pulmonary fibrosis or interstitial lung fibrosis, idiopathic pulmonary fibrosis, cystic fibrosis, glomerulonephritis with and without nephrotic syndrome, acute glomerulonephritis, idiopathic nephrotic syndrome, minimal change nephropathy, inflammatory/hyperproliferative skin diseases, benign familial pemphigus, pemphigus erythematosus, pemphigus foliaceus, and pemphigus vulgaris,
■ prevention of allogeneic graft rejection following organ transplantation,
■ inflammatory bowel disease (IBD) of whatever type, etiology, or pathogenesis, in particular inflammatory bowel disease that is a member selected from the group consisting of collagenous colitis, colitis polyposa, transmural colitis, ulcerative colitis and Crohn's disease (CD),
■ septic shock of whatever type, etiology, or pathogenesis, in particular septic shock that is a member selected from the group consisting of renal failure, acute renal failure, cachexia, malarial cachexia, hypophysial cachexia, uremic cachexia, cardiac cachexia, cachexia suprarenalis or Addison's disease, cancerous cachexia and cachexia as a consequence of infection by the human immunodeficiency virus (HIV),
■ liver injury,
■ pulmonary hypertension of whatever type, etiology or pathogenesis including primary pulmonary hypertension / essential hypertension, pulmonary hypertension secondary to congestive heart failure, pulmonary hypertension secondary to chronic obstructive pulmonary disease, pulmonary venous hypertension, pulmonary arterial hypertension and hypoxia-induced pulmonary hypertension,
■ bone loss diseases, primary osteoporosis and secondary osteoporosis,
■ central nervous system disorders of whatever type, etiology, or pathogenesis, in particular a central nervous system disorder that is a member selected from the group consisting of depression, Alzheimers disease, Parkinson's disease, learning and memory impairment, tardive dyskinesia, drug dependence, arteriosclerotic dementia and dementias that accompany Huntington's chorea, Wilson's disease, paralysis agitans, and thalamic atrophies,
■ infection, especially infection by viruses wherein such viruses increase the production of TNF-α in their host, or wherein such viruses are sensitive to upregulation of TNF-α in their host so that their replication or other vital activities are adversely impacted, including a virus which is a member selected from the group consisting of HIV-9, HIV-2, and HIV-3, cytomegalovirus (CMV), influenza, adenoviruses and Herpes viruses including *Herpes zoster* and *Herpes simplex,*
■ yeast and fungus infections wherein said yeast and fungi are sensitive to upregulation by TNF-α or elicit TNF-α production in their host, e.g., fungal meningitis, particularly when administered in conjunction with other drugs of choice for the treatment of systemic yeast and fungus infections, including but are not limited to, polymixins, e.g. Polymycin B, imidazoles, e.g. clotrimazole, econazole, miconazole, and ketoconazole, triazoles, e.g. fluconazole and itranazole as well as amphotericins, e.g. Amphotericin B and liposomal Amphotericin B,
■ ischemia-reperfusion injury, ischemic heart disease, autoimmune diabetes, retinal autoimmunity, chronic lymphocytic leukemia, HIV infections, lupus erythematosus, kidney and ureter disease, urogenital and gastrointestinal disorders and prostate diseases,
■ reduction of scar formation in the human or animal body, such as scar formation in the healing of acute wounds, and
■ psoriasis, other dermatological and cosmetic uses, including antiphlogistic, skin-softening, skin elasticity and moisture-increasing activities.

According to one aspect the present invention relates in particular to the treatment of a respiratory disease, such as adult respiratory distress syndrome (ARDS), bronchitis, chronic bronchitis, chronic obstructive pulmonary disease (COPD), cystic fibrosis, asthma, emphysema, bronchiectasis, chronic sinusitis and rhinitis.

According to another aspect the present invention relates in particular to the treatment of gastrointestinal (Gl) disorders, in particular inflammatory bowel diseases (IBD) such as Crohn's disease, ileitis, collagenous colitis, colitis polyposa, transmural colitis and ulcerative colitis.

A still further aspect of the present invention also relates to the use of the nicotinamide derivatives of formula (I), their pharmaceutically acceptable salts and/or derived forms, for the manufacture of a drug having a PDE4 inhibitory activity. In particular, the present inventions concerns the use of the nicotinamide derivatives of formula (I), their pharmaceutically acceptable salts and/or derived forms, for the manufacture of a drug for the treatment of inflammatory, respiratory, allergic and scar-forming diseases, disorders, and conditions, and more precisely for the treatment of diseases, disorders, and conditions that are listed above.

As a consequence, the present invention provides a particularly interesting method of treatment of a mammal, including a human being, with a PDE4 inhibitor including treating said mammal with an effective amount of a nicotinamide derivative of formula (I), its pharmaceutically acceptable salts and/or derived forms. More precisely, the present invention provides a particularly interesting method of treatment of a mammal, including a human being, to treat an inflammatory, respiratory, allergic and scar-forming disease, disorder or condition, including treating said mammal with an effective amount of a nicotinamide derivative of formula (I), its pharmaceutically acceptable salts and/or derived forms.

Further aspects of the invention are mentioned in the claims.

The following Examples illustrate the preparation of the nicotinamide derivatives of the formula (I) :

### Examples 1 to 5

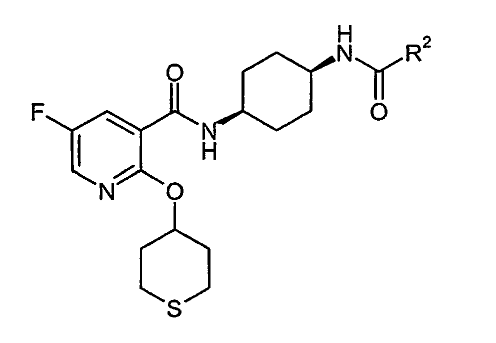

1-(3-Dimethylaminopropyl)-3-ethylcarbodiimide hydrochloride (1.67eq) was added to a solution of the amine hydrochloride from preparation 15a (1.2eq), 1-hydroxybenzotriazole hydrate (1.2eq), the appropriate acid (1eq) and N-ethyldiisopropylamine (3.3eq) in dichloromethane (7.5mlmmol⁻¹), and the reaction stirred at room temperature for 18 hours. The mixture was partitioned between dichloromethane and 1N hydrochloric acid, and the layers separated, adding a minimum volume of methanol to prevent precipitation where necessary. The organic phase was concentrated under reduced pressure, the residue triturated with hot isopropyl acetate, and the resulting solid filtered off and dried to give the title compounds.

| Ex no | R² | Yield (%) | Data |
|---|---|---|---|
| 1 | 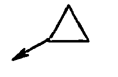 | 96 | ¹HNMR (DMSO-d₆, 400MHz) δ: 0.64 (m, 4H), 1.50-1.80 (m, 9H), 1.84-1.94 (m, 2H), 2.26 (m, 2H), 2.62-2.82 (m, 4H), 3.64 (m, 1H), 3.90 (m, 1H), 5.16 (m, 1H), 7.90 (d, 1H), 7.96 (m, 1H), 8.08 (d, 1H), 8.26 (m, 1H) |
| | | | LRMS: m/z ES⁺ 444 [MNa]⁺ |
| | | | Microanalysis found; C, 59.53; H, 6.55; N, 10.01, C₂₁H₂₈FN₃O₃S; requires C, 59.84; H, 6.70; N, 9.97%. |
| 2 | 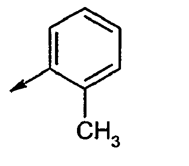 | 68 | ¹HNMR (DMSO-d₆, 400MHz) δ: 1.60-1.96 (m, 10H), 2.24-2.34 (m, 5H), 2.64-2.80 (m, 4H), 3.84 (m, 1H), 3.94 (m, 1H), 5.16 (m, 1H), 6.14-6.32 (m, 4H), 7.96 (m, 1H), 8.08 (m, 2H), 8.26 (m, 1H) |
| | | | LRMS: m/z APCl⁺ 472 [MH]⁺ |
| | | | Microanalysis found; C, 61.25; H, 6.30; N, 8.88, C₂₅H₃₀FN₃O₃S; 1.0H₂O requires C, 61.33; H, 6.52; N, 8.58%. |
| 3 | 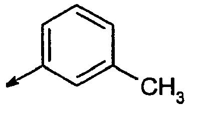 | 50 | ¹HNMR (DMSO-d₆, 400MHz) δ: 1.60-1.96 (m, 10H), 2.24-2.36 (m, 5H), 2.64-2.80 (m, 4H), 3.82 (m, 1H), 3.96 (m, 1H), 5.18 (m, 1H), 7.30 (m, 2H), 7.60 (m, 2H), 7.98 (m, 1H), 8.08 (m, 2H), 8.26 (m, 1H) |
| | | | LRMS: m/z APCl⁺ 472 [MH]⁺ |
| | | | Microanalysis found; C, 62.69; H, 6.43; N, 9.13, C₂₅H₃₀FN₃O₃S; 0.4H₂O requires C, 62.71; H, 6.48; N, 8.78%. |
| 4 | 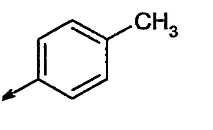 | 67 | ¹HNMR (CD₃OD, 400MHz) δ: 1.70-2.06 (m, 10H), 2.36-2.46 (m, 5H), 2.72-2.86 (m, 4H), 3.98 (m, 1H), 4.16 (m, 1H), 5.16 (m, 1H), 7.26 (d, 2H), 7.70 (d, 2H), 8.06 (m, 1H), 8.18 (d, 1H) |
| | | | LRMS: m/z ES⁺ 494 [MNa]⁺ |
| | | | Microanalysis found; C, 62.98; H, 6.37; N, 8.93, C₂₅H₃₀FN₃O₃S; 0.3H₂O requires C, 62.95; H, 6.47; N, 8.81%. |
| 5^{1A} | 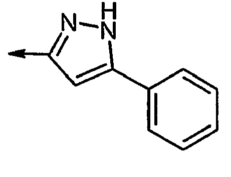 | 21 | ¹HNMR (DMSO-d₆, 400MHz) δ: 1.80-1.95 (m, 10H), 2.25 (m, 2H), 2.60-2.70 (m, 2H), 2.70-2.85 (m, 2H), 3.85 (m, 1H), 3.95 (m, 1H), 5.15 (m, 1H), 7.15 (s, 1H), 7.25-7.60 (m, 4H), 7.75 (m, 2H), 7.95 (dd, 1H), 8.10 (d, 1H), 8.25 (m, 1H), 13.58 (br s, 1H) |
| | | | LRMS: m/z ES⁺ 546 [MNa]⁺ |
| | | | Microanalysis found; C, 60.58; H, 5.84; N, 13.18, C₂₇H₃₀FN₅O₃S;0.6H₂O; requires C, 60.68; H, 5.88, N, 13.10%. |

| | | | |
|---|---|---|---|
| A= 5-phenyl-pyrazole-3-carboxylic acid was obtained from Cambridge Major 1= N-methylmorpholine was used as the base instead of N-ethyldiisopropylamine, and the product was triturated from methanol. | | | |

### Examples 6 to 14

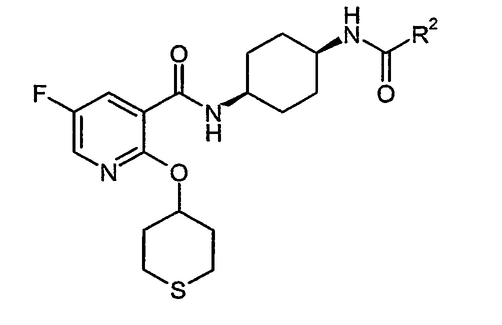

1-(3-Dimethylaminopropyl)-3-ethylcarbodiimide hydrochloride (1.5eq) was added to a solution of the amine hydrochloride from preparation 15a (1eq), 1-hydroxybenzotriazole hydrate (1.2eq), the appropriate acid (1eq) and N-ethyldiisopropylamine (4eq) in N,N-dimethylacetamide (7mlmmol⁻¹), and the reaction stirred at room temperature for 18 hours. The mixture was partitioned between ethyl acetate and 10% aqueous citric acid and the layers separated. The organic phase was washed with sodium bicarbonate solution and brine, then dried (MgSO₄) and concentrated under reduced pressure. The crude products were purified either by trituration with isopropyl acetate to give the products as solids (A), or by column chromatography on silica gel using an elution gradient of dichloromethane:methanol:0.88 ammonia (99:1:0.1 to 96:4:0.4) (B).

| Ex no | R² | Yield (%) | Data |
|---|---|---|---|
| 6 B | 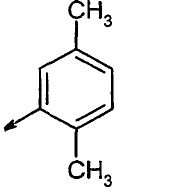 | 58 | ¹HNMR (DMSO-d₆, 400MHz) δ: 1.60-1.90 (m, 10H), 2.24-2.34 (m, 8H), 2.60-2.80 (m, 4H), 3.82 (m, 1H), 3.96 (m, 1H), 5.18 (m, 1H), 7.08 (m, 3H), 7.98 (m, 1H), 8.10 (m, 2H), 8.28 (d, 1H) |
| | | | LRMS: m/z ES⁺ 508 [MNa]⁺ |
| | | | Microanalysis found; C, 63.16; H, 6.58; N, 8.47, C₂₆H₃₂FN₃O₃S; 0.5H₂O requires C, 63.14; H, 6.72; N, 8.50%. |
| 7^{1c} B | 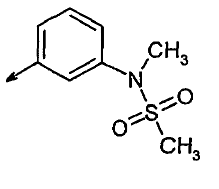 | 98 | ¹HNMR (CDCl₃, 400MHz) δ: 1.60-2.10 (m, 10H), 2.40 (m, 2H), 2.72-2.86 (m, 4H), 2.88 (s, 3H), 3.36 (s, 3H), 4.10-4.30 (m, 2H), 5.46 (m, 1H), 6.22 (d, 1H), 7.48 (m, 1H), 7.54 (m, 1H), 7.70 (d, 1H), 7.84 (s, 1H), 8.06 (d, 1H), 8.10 (d, 1H), 8.28 (m, 1H) |
| | | | LRMS: m/z ES⁺ 587 [MNa]⁺ |
| | | | Microanalysis found; C, 55.18; H, 5.89; N, 9.93, C₂₆H₃₃FN₄O₅S₂; requires C, 55.30; H, 5.89; N, 9.92 %. |
| 8^{2c} B | 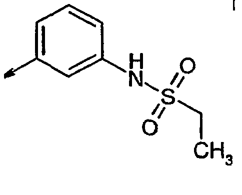 | 47 | ¹HNMR (DMSO-d₆, 400MHz) δ: 1.18 (t, 3H), 1.60-2.00 (m, 10H), 2.28 (m, 2H), 2.62-2.80 (m, 4H), 3.08 (q, 2H), 3.80 (m, 1H), 3.96 (m, 1H), 5.28 (m, 1H), 7.28-7.40 (m, 2H), 7.52 (d, 1H), 7.60 (s, 1H), 8.00 (m, 1H), 8.14 (m, 2H), 8.28 (d, 1H), 9.92 (s, 1H) |
| | | | LRMS: m/z ES⁺ 587 [MNa]⁺ |
| | | | Microanalysis found; C, 55.11; H, 5.87; N, 9.87, C₂₆H₃₃FN₄O₅S₂; requires C, 55.30; H, 5.89; N, 9.92 %. |
| 9^{3c} B | 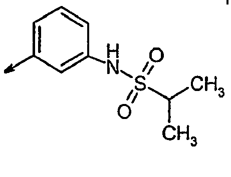 | 76 | ¹HNMR (DMSO-d₆, 400MHz) δ: 1.20 (d, 6H), 1.60-1.96 (m, 10H), 2.28 (m, 2H), 2.62-2.80 (m, 4H), 3.20 (m, 1H), 3.80 (m, 1H), 3.96 (m, 1H), 5.28 (m, 1H), 7.38 (m, 2H), 7.50 (m, 1H), 7.60 (m, 1H), 8.00 (m, 1H), 8.14 (m, 2H), 8.30 (d, 1H), 9.90 (s, 1H) |
| | | | LRMS: m/z ES⁺ 601 [MNa]⁺ |
| | | | Microanalysis found; C, 55.76; H, 6.12; N, 9.55, C₂₇H₃₅FN₄O₅S₂; requires C, 56.04; H, 6.10; N, 9.68 %. |
| 10^{4d} A | 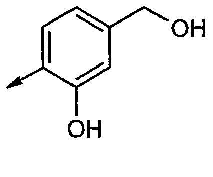 | 46 | ¹HNMR (CD₃OD, 400MHz) δ: 1.70-2.04 (m, 10H), 2.36-2.45 (m, 2H), 2.73-2.81 (m, 4H), 4.05 (m, 1H), 4.16 (m, 1H), 4.57 (s, 2H), 5.34 (m, 1H), 6.87 (d, 1H), 6.91 (s, 1H), 7.82 (m, 1H), 8.11 (m, 2H), 8.36 (m, 1H) |
| | | | LRMS: m/z APCl⁺ 504 [MH]⁺ |
| | | | Microanalysis found; C, 58.78; H, 6.07; N, 8.12. C₂₅H₃₀FN₃O₅S ;0.4 H₂O requires C, 58.79; H, 6.08; N, 8.23%. |
| 11 A | 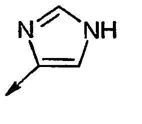 | 48 | ¹HNMR (DMSO-d₆, 400MHz) δ: 1.60-1.80 (m, 8H), 1.80-1.96 (m, 2H), 2.20-2.32 (m, 2H), 2.64 (m, 2H), 2.78 (m, 2H), 3.88 (m, 1H), 3.98 (m, 1H), 5.16 (m, 1H), 7.42 (m, 1H), 7.60 (s, 1H), 7.70 (s, 1H), 7.86 (m, 1H), 8.10 (m, 1H), 8.26 (d, 1H), 12.50 (m, 1H) |
| | | | LRMS: m/z ES⁺ 470 [MNa]⁺ |
| | | | Microanalysis found; C, 55.62; H, 5.96; N, 14.98, C₂₁H₂₆FN₅O₃S; 0.5H₂O requires C, 55.25; H, 5.96; N, 15.34%. |
| 12⁵ B | 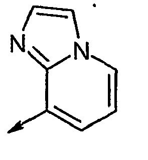 | 20 | ¹HNMR (CDCl₃, 400MHz) δ: 1.80-2.10 (m, 10H), 2.44 (m, 2H), 2.76 (m, 4H), 4.20 (m, 1H), 4.32 (m, 1H), 5.26 (m, 1H), 6.98 (m, 1H), 7.64-7.70 (m, 2H), 8.04 (d, 1H), 8.16 (d, 1H), 8.20-8.30 (m, 3H), 10.58 (m, 1H) |
| | | | LRMS: m/z ES⁺ 520 [MNa]⁺ |
| | | | Microanalysis found; C, 58.58; H, 5.78; N, 13.49, C₂₅H₂₈FN₅O₃S; 0.8H₂O requires C, 58.65; H, 5.83; N, 13.68 %. |
| 13⁶ B | 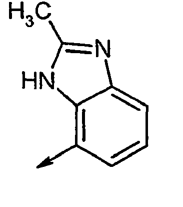 | 15 | ¹HNMR (CDCl₃, 400MHz) δ: 1.50-2.10 (m, 11H), 2.40-2.50 (m, 2H), 2.64-2.82 (m, 7H), 4.16-4.34 (m, 2H), 5.28 (m, 1H), 7.24-7.34 (m, 3H), 7.62 (m, 1H), 8.04 (d, 1H), 8.10 (m, 1H), 8.28 (m, 1H) |
| | | | LRMS: m/z ES⁺ 534 [MNa]⁺ |
| | | | Microanalysis found; C, 60.35; H, 5.88; N, 13.37, C₂₆H₃₀FN₅O₃S; 0.3H₂O requires C, 60.40; H, 5.97; N, 13.55 %. |
| 14⁷ B | 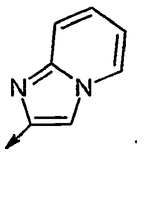 | 38 | ¹HNMR (CDCl₃, 400MHz) δ: 1.66-2.10 (m, 10H), 2.40-2.50 (m, 2H), 2.80-2.90 (m, 4H), 4.16-4.30(m, 2H), 5.30 (m, 1H), 6.86 (m, 1H), 7.26 (m, 1H), 7.48 (m, 1H), 7.48 (d, 1H), 8.06 (d, 1H), 8.12 (m, 3H), 8.26 (m, 1H) |
| | | | LRMS: m/z ES⁺ 520 [MNa]⁺ |
| | | | Microanalysis found; C, 59.91; H, 5.67; N, 13.84, C₂₅H₂₈FN₅O₃S; 0.2H₂O requires C, 59.91; H, 5.71; N, 13.97 %. |

| | | | |
|---|---|---|---|
| 1= 3-Methanesulphonylmethylamino-benzoic acid was prepared as described in preparation 48 2= 3-Ethanesulphonylamino-benzoic acid was prepared as described in preparation 49 3= 3-Isopropylsulphonylamino-benzoic acid was prepared as described in preparation 50 4= 2-Hydroxy-4-hydroxymethylbenzoic acid was prepared as described in preparation 65 5= Imidazo[1,2-a]pyridine-8-carboxylic acid was prepared as described in preparation 23 6 = 2-Methyl-1H-benzoimidazole-4-carboxylic acid was prepared as described in J. Med. Chem. 2000; 43(22); 4084. 7 = Imidazo[1,2-a]pyridine-2-carboxylic acid hydrobromide was prepared as described in preparation 21 C= N,N-dimethylformamide was used as the reaction solvent D= the product was triturated with diisopropyl ether | | | |

### Example 15

### Syn-N-[4-(4-Chloro-2-hydrox-benzoylamino)-cyclohexyl]-5-fluoro-2-(tetrahy dro-thiopyran-4-yloxy)-nicotinamide

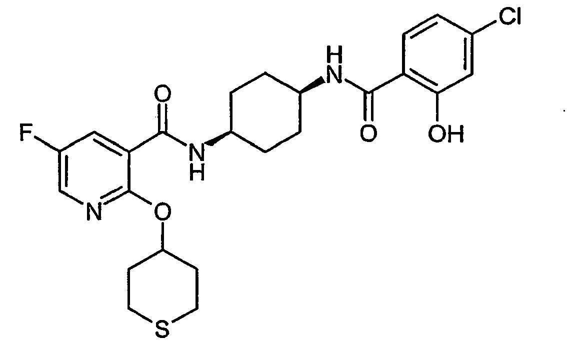

1-(3-Dimethylaminopropyl)-3-ethylcarbodiimide hydrochloride (125mg, 0.65mmol) was added to a solution of the amine from preparation 15b (177mg, 0.5mmol), 1-hydroxybenzotriazole hydrate (88mg, 0.65mmol), 4-chlorosalicylic acid (78mg, 0.45mmol) and N-ethyldiisopropylamine (260µl, 1.5mmol) in N,N-dimethylformamide (10ml), and the reaction stirred at room temperature for 18 hours. The mixture was partitioned between ethyl acetate (15ml) and water (10ml), and the layers separated.

The organic phase was washed with 1N citric acid (20ml), sodium bicarbonate solution (20ml) and brine (20ml), then dried (MgSO₄) and evaporated under reduced pressure. The product was crystallised from ether and dried *in vacuo* to afford the title compound, 17mg.
¹HNMR (CD₃OD, 400MHz) δ: 1.74-2.03 (m, 10H), 2.37-2.43 (m, 2H), 2.70-2.83 (m, 4H), 4.04 (m, 1H), 4.13 (m, 1H), 5.35 (m, 1H), 6.93 (m, 2H), 7.83 (d, 1H), 8.06 (m, 1H), 8.16 (d, 1H).
LRMS : m/z ES⁺ 530 [MNa]⁺
Microanalysis found: C, 56.44; H, 5.19; N, 7.93. C₂₄H₂₇ ClFN₃O₄S;0.15H₂O requires C, 56.44; H, 5.39; N, 8.23%.

### Examples 16 to 24

The following examples of general structure: 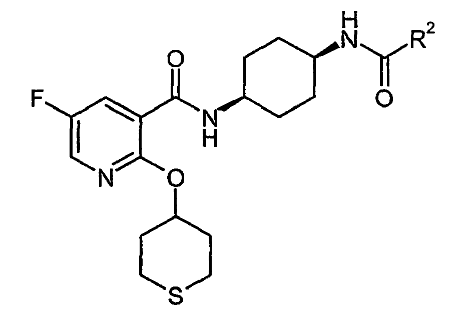 were prepared from the amine from preparation 15b, and the appropriate acid, following a similar procedure to that described in example 15.

| Ex no | R² | Yield (%) | Data |
|---|---|---|---|
| 16 | 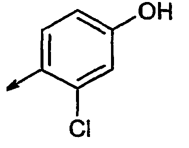 | 37 | ¹HNMR (CD₃OD, 400MHz) δ: 1.74-2.02 (m, 10H), 2.38-2.42 (m, 2H), 2.72-2.81 (m, 4H), 4.00 (m, 1H), 4.10 (m, 1H), 5.32 (m, 1H), 6.76 (d, 1H), 6.84 (s, 1H), 7.33 (d, 1H), 8.07 (m, 1H), 8.16 (d, 1H). |
| | | | LRMS: m/z ES⁺ 530 [MNa]⁺ |
| | | | Microanalysis found; C, 56.40; H, 5.38; N, 8.10, C₂₄H₂₇ CIFN₃O₄S; 0.16H₂O requires C, 56.42; H, 5.39; N, 8.22%. |
| 17 | 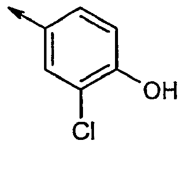 | 74 | ¹HNMR (DMSO-d₆, 400MHz) δ: 1.70 (m, 6H), 1.81 (m, 2H), 1.92 (m, 2H), 2.30 (m, 2H), 2.72 (m, 4H), 3.79 (m, 1H), 3.96 (m, 1H), 5.19 (m, 1H), 6.97 (d, 1H), 7.64 (d, 1H), 7.84 (s, 1H), 8.00 (m, 2H), 8.10 (d, 1H), 8.28 (d, 1H), 10.72 (brs,1H). |
| | | | LRMS : m/z APCl⁺ 508, 510 [MH⁺] |
| | | | Microanalysis found: C, 56.33; H, 5.43; N, 7.96. C₂₄H₂₇ClFN₃O₄S requires C, 56.74; H, 5.36; N, 8.27%. |
| 18 | 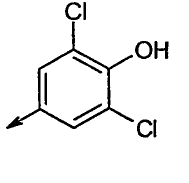 | 73 | ¹HNMR (DMSO-d₆, 400MHz) δ: 1.69 (m, 6H), 1.80 (m, 2H), 1.88 (m, 2H), 2.28 (m, 2H), 2.71 (m, 4H), 3.79 (m, 1H), 3.95 (m, 1H), 5.19 (m, 1H), 7.84 (s, 2H), 7.98 (dd, 1H), 8.11 (d, 1H), 8.17 (d, 1H), 8.28 (d, 1H), 10.73 (brs, 1H). |
| | | | LRMS : m/z APCl⁻ 540, 542, 544 [M-H] |
| | | | Microanalysis found: C, 52.98; H, 4.94; N, 7.58. C₂₄H₂₆Cl₂FN₃O₄S requires C, 53.14; H, 4.83; N, 7.75%. |
| 19^{A1} | 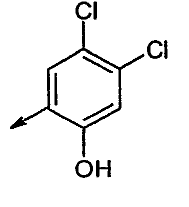 | 20 | ¹HNMR (CD₃OD, 400MHz) δ: 1.74-2.03 (m, 10H), 2.37-2.44 (m, 2H), 2.71-2.83 (m, 4H), 4.03 (m, 1H), 4.13 (m, 1H), 5.35 (m, 1H), 7.08 (s, 1H), 8.00 (s, 1H), 8.07 (m, 1H), 8.15 (d, 1H). |
| | | | LRMS : m/z APCl⁺542 [MH⁺] |
| 20 | 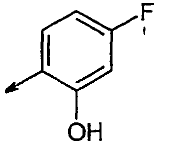 | 31 | ¹HNMR (CD₃OD, 400MHz) δ: 1.76-2.03 (m, 10H), 2.40 (m, 2H), 2.70-2.82 (m, 4H), 4.04 (m, 1H), 4.13 (m, 1H), 5.34 (m, 1H), 6.60-6.64 (m, 2H), 7.88 (m, 1H), 8.06 (m, 1H), 8.17 (d, 1H). |
| | | | LRMS: m/z ES⁺ 514 [MNa]⁺ |
| 21 | 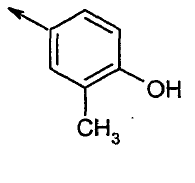 | 74 | ¹HNMR (DMSO-d₆, 400MHz) δ: 1.70 (m, 2H), 1.82 (m, 2H), 1.98 (m, 6H), 2.30 (s, 3H), 2.42 (m, 2H), 2.80 (m, 4H), 4.15 (m, 1H), 4.25 (m, 1H), 5.44 (m, 1H), 5.63 (brs, 1H), 6.05 (d, 1H), 6.82 (d, 1H), 7.54 (dd, 1H), 7.59 (s, 1H), 8.12 (d, 1H), 8.27 (dd, 1H). |
| | | | LRMS : m/z APCl⁺ 488 [MH⁺] |
| | | | Microanalysis found: C, 61.17; H, 6.23; N, 8.64. C₂₅H₃₀FN₃O₄S requires C, 61.58; H, 6.20; N, 8.62%. |
| 22^{B} | 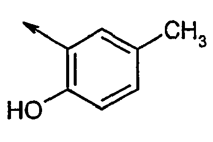 | 96 | ¹HNMR (CDCl₃, 400MHz) δ : 1.73 (m, 2H), 1.83 (m, 2H), 1.99 (m, 6H), 2.31 (s, 3H), 2.39 (m, 2H), 2.76 (m, 2H), 2.84 (m, 2H), 4.15 (m, 1H), 4.27 (m, 1H), 5.49 (m, 1H), 6.35 (d, 1H), 6.90 (d, 1H), 7.19 (s, 1H), 7.21 (d, 1H), 8.07 (d, 1H), 8.11 (d, 1H), 8.28 (dd, 1H), 11.99 (brs, 1H). |
| | | | LRMS : m/z APCl⁺ 488 [MH⁺] |
| 23² | 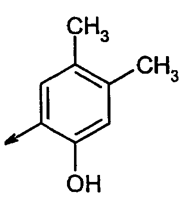 | 61 | ¹HNMR (DMSO-d₆, 400MHz) δ: 1.63-1.93 (m, 10H), 2.13 (s, 3H), 2.16 (s, 3H), 2.25-2.32 (m, 2H), 2.63-2.79 (m, 4H), 3.87 (m, 1H), 3.98 (m, 1H), 5.18 (m, 1H), 6.68 (s, 1H), 7.63 (s, 1H), 7.97 (m, 1H), 8.13 (d, 1H), 8.28 (d, 1H), 8.32 (m, 1H), 12.05 (brs, 1H). |
| | | | LRMS : m/z APCl⁺ 502 [MH⁺] |
| 24 | 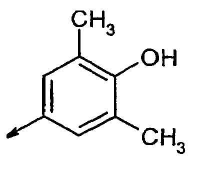 | 19 | ¹HNMR (CD₃OD, 400MHz) δ: 1.75 (m, 2H), 1.83-2.05 (m, 8H), 2.24 (s, 6H), 2.42 (m, 2H), 2.73-2.85 (m, 4H), 3.95 (m, 1H), 4.15 (m, 1H), 5.35 (m, 1H), 7.43 (s, 2H), 8.06 (m, 1H), 8.16 (d, 1H). |
| | | | LRMS : m/z APCl⁺ 502 [MH⁺] |

| | | | |
|---|---|---|---|
| 1= 4,5-dichloro-2-hydroxybenzoic acid was prepared as described in (US 2703332) 2= 4,5-dimethyl-2-hydroxybenzoic acid was prepared as described in Bull. Soc. Chim. Fr. 1963; 1813 A= 2.6eq of 1-(3-dimethylaminopropyl)-3-ethylcarbodiimide hydrochloride was used, and the product was purified by column chromatography on silica gel using methanol:dichloromethane (3:97). B= the amine hydrochloride from preparation 15a was used,1-methyl-2-pyrrolidinone was used as the solvent, and the reaction was stirred at 40°C | | | |

### Example 25

### Syn-5-Fluoro-N-[4-(5-fluoro-2-hydroxy-benzoylamino)-cyclohexyl]-2-(tetrahydrothiopyran-4-yloxy)-nicotinamide

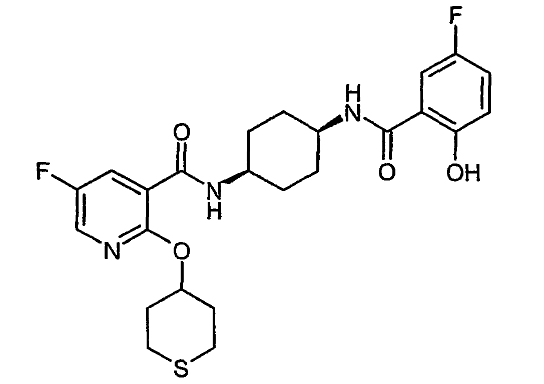

1-(3-Dimethylaminopropyl)-3-ethylcarbodiimide hydrochloride (111.2mg, 0.58mmol) was added to a mixture of 5-fluorosalicylic acid (70.2mg, 0.45mmol), the amine from preparation 15b (176mg, 0.5mmol), 1-hydroxybenzotriazole hydrate (68mg, 0.5mmol) and N-ethyldiisopropylethylamine (142mg, 1.1mmol) in 1-methyl-2-pyrrolidinone (3ml), and the reaction stirred at room temperature for 48 hours. TLC analysis showed starting material remaining, so additional 5-fluorosalicylic acid (31 mg, 0.2mmol), 1-hydroxybenzotriazole hydrate (30mg, 0.22mmol), N-ethyldiisopropylamine (28.5mg, 0.22mmol) and 1-(3-dimethylaminopropyl)-3-ethylcarbodiimide hydrochloride (48mg, 0.25mmol) were added and the reaction stirred for a further 3 days. The mixture was partitioned between ethyl acetate (100ml) and water (100ml), and the pH adjusted to 2 using 2N hydrochloric acid. The layers were separated, and the organic phase washed with water (3x75ml), dried (MgSO₄) and concentrated under reduced pressure.The residue was purified by column chromatography on silica gel using an elution gradient of dichloromethane:methanol (99:1 to 96:4), and the product crystallised from isopropyl acetate to give the title compound as a solid, 69mg.
¹HNMR (DMSO-d₆, 400MHz) δ: 1.70 (m, 8H), 1.87 (m, 2H), 2.25 (m, 2H), 2.66 (m, 2H), 2.95 (m, 2H), 3.87 (m, 1H), 3.98 (m, 1H), 5.16 (m, 1H), 6.92 (m, 1H), 7.25 (m, 1H), 7.70 (m, 1H), 7.93 (m, 1H), 8.11 (m, 1H), 8.25 (m, 1H), 8.48 (m, 1H), 12.00 (s, 1H).
LRMS : m/z ES⁺ 514 [MNa⁺]
Microanalysis found: C, 58.64; H, 5.54; N, 8.55. C₂₄H₂₇F₂N₃O₄S requires C, 58.32; H, 5.52; N, 8.42%.

### Example 26

### Syn-N-14-(3,5-Dichloro-2-hydroxy-benzoylamino)-cydohexyl]-5-fluoro-2-(tetrahydrothiopyran-4-yloxy)-nicotinamide

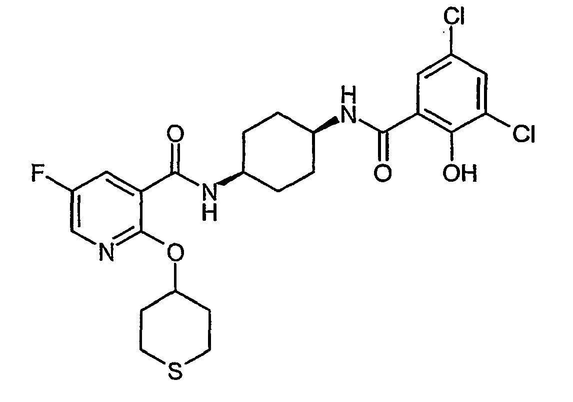

The title compound was obtained as a white solid in 12% yield from the compound from preparation 15b and 3,5-dichlorosalicylic acid, following the procedure described in example 25.
¹HNMR (DMSO-d₆, 400MHz) δ: 1.70 (m, 8H), 1.87 (m, 2H), 2.28 (m, 2H), 2.66 (m, 2H), 2.75 (m, 2H), 3.86 (m, 1H), 3.97 (m, 1H), 5.16 (m, 1H), 7.74 (m, 1H), 7.97 (m, 1H), 8.05 (m, 1H), 8.12 (m, 1H), 8.28 (m, 1H), 8.80 (m, 1H), 13.65 (s, 1H).
LRMS : m/z APCl⁺ 542, 544, 546 [MH⁺]

### Example 27

### Syn-N-[4-(5-Chloro-2-hydroxy-benzoylamino)-cyclohexyl]-5-fluoro-2-(tetrahydrothiopyran-4-yloxy)-nicotinamide

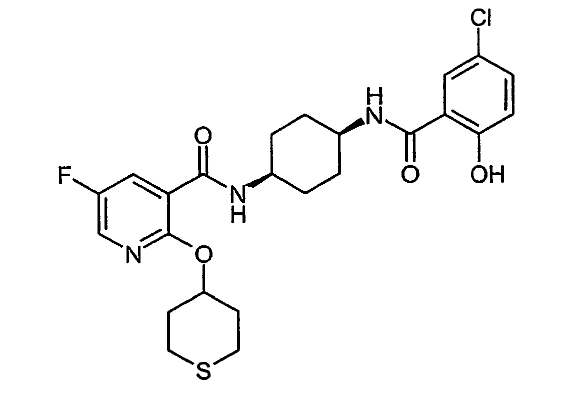

1-(3-Dimethylaminopropyl)-3-ethylcarbodiimide hydrochloride (125mg, 0.65mmol) was added to a mixture of 5-chlorosalicylic acid (78mg, 0.45mmol), the amine from preparation 15b (177mg, 0.5mmol), 1-hydroxybenzotriazole hydrate (88mg, 0.65mmol) and N-ethyldiisopropylethylamine (0.26m1, 1.5mmol) in N,N-dimethylformamide (10ml), and the reaction stirred at room temperature for 18 hours. The mixture was partitioned between ethyl acetate (15ml) and 1 N citric acid solution (15ml), and the layers separated. The organic phase was washed with sodium bicarbonate solution (15ml), then dried (MgSO₄) and evaporated under reduced pressure. The product was suspended in tolene (5ml), n-butylamine (0.05ml, 0.5mmol) added and the solution stirred at room temperature for 18 hours. The solution was concentrated under reduced pressure and the residue dissolved in ethyl acetate (with a minimum volume of dichloromethane), washed with 1 N citric acid, saturated sodium carbonate solution, dried (MgSO₄) and evaporated under reduced pressure to give the title compound, as a white solid, 28mg.
¹HNMR (CD₃OD, 400MHz) δ: 1.74-2.03 (m, 10H), 2.37-2.42 (m, 2H), 2.71-2.83 (m, 4H), 4.04 (m, 1H), 4.13 (m, 1H), 5.34 (m, 1H), 6.90 (d, 1H), 7.33 (m, 1H), 7.85 (s, 1H), 8.06 (m, 1H), 8.16 (d, 1H).
LRMS : m/z APCl⁺ 508 [MH⁺]

### Example 28

### Syn-5-Fluoro-N-[4-(2-hydroxy-3-methyl-benzoylamino)-cyclohexyl]-2-(tetrahydrothiopyran-4-yloxy)-nicotinamide

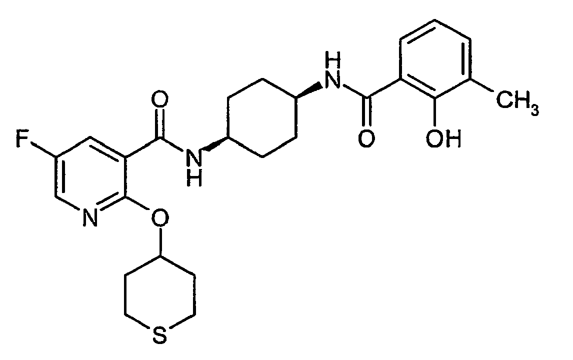

A solution of 3-methylsalicylic acid (32mg, 0.21 mmol) in N,N-dimethylformamide (0.5ml) was added to a mixture of 1-(3-dimethylaminopropyl)-3-ethylcarbodiimide hydrochloride (44mg, 0.23mmol), the amine from preparation 15a (82mg, 0.21 mmol), 1-hydroxybenzotriazole hydrate (31 mg, 0.23mmol) and N-methylmorpholine (48µl, 0.44mmol) in N,N-dimethylformamide (4ml), and the reaction stirred at room temperature for 18 hours. The mixture was evaporated under reduced pressure and the residue suspended in tetrahydrofuran (1ml) and 1N sodium hydroxide solution (1ml), and stirred at room temperature for 72 hours. The mixture was concentrated under reduced pressure, the aqueous solution acidified by the addition of 2N hydrochloric acid (1ml), and extracted with dichloromethane (5ml, 2ml). The combined organic extracts were washed with water (2ml), dried (MgSO₄) and evaporated under reduced pressure. The residue was crystallised from methanol to afford the title compound.
¹HNMR (CDCl₃, 400MHz) δ: 1.72 (m, 2H), 1.81 (m, 2H), 1.89-2.05 (m, 6H), 2.27 (s, 3H), 2.72-2.88 (m, 4H), 4.16 (m, 1H), 4.26 (m, 1H), 5.46 (m, 1H), 6.32 (d, 1H), 6.76 (m, 1H), 7.27 (m, 2H), 8.06 (m, 1H), 8.10 (m, 1H), 8.28 (dd, 1H), 12.50 (brs, 1H).
LRMS : m/z APCl⁺ 510 [MNa⁺]

### Example 29

### Syn-5-Fluoro-N-[4-(2-hydroxy-benzoylamino)-cyclohexyl]-2-(tetrahydrothiopyran-4-yloxy)-nicotinamide

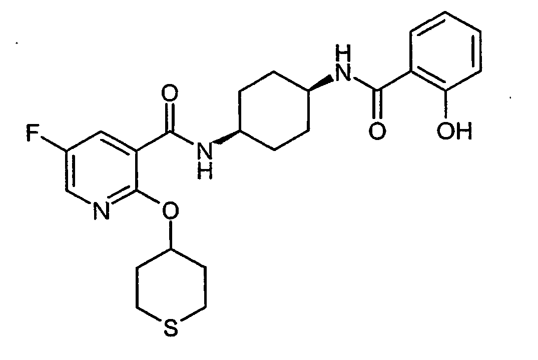

The title compound was obtained in 86% yield from the amine from preparation 15a and 2-hydroxybenzoic acid, following the procedure described in example 28.
¹HNMR (CDCl₃, 400MHz) δ: 1.72(m, 2H), 1.81 (m, 2H), 1.97 (m, 6H), 2.39 (m, 2H), 2.80 (m, 4H), 4.15 (m, 1H), 4.26 (m, 1H), 5.47 (m, 1H), 6.37 (m, 1H), 6.87 (t, 1H), 6.99 (d, 1H), 7.42 (m, 2H), 8.06 (d, 1H), 8.11 (m, 1H), 8.28 (m, 1H), 12.22 (brs, 1H)
LRMS : m/z ES⁺ 496 [MNa]⁺
Microanalysis found; C, 60.60; H, 5.96; N, 8.71, C₂₄H₂₈FN₃O₄S; requires C, 60.87; H, 5.96, N, 8.87%.

### Example 30

### Syn-2-Hydroxy-quinoline-4-carboxylic acid (4-{[5-fluoro-2-(tetrahydro-thiopyran-4-yloxy)-pyridine-3-carbonyl]-amino}-cyclohexyl)-amide

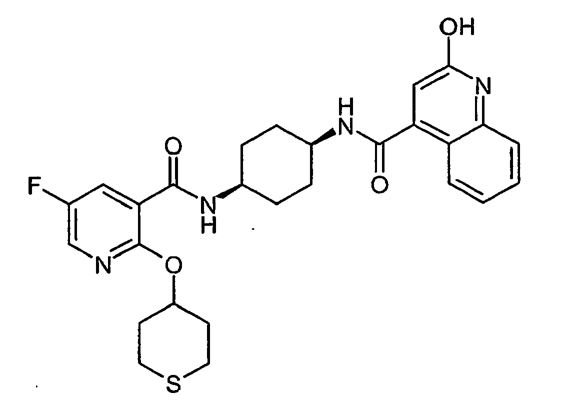

1-(3-Dimethylaminopropyl)-3-ethylcarbodiimide hydrochloride (96mg, 0.5mmol) was added to a solution of the amine hydrochloride from preparation 15a (150mg, 0.38mmol), 1-hydroxybenzotriazole hydrate (77mg, 0.50mmol), 2-hydroxy-4-quinoline carboxylic acid (73mg, 0.38mmol) and N-ethyldiisopropylamine (0.27ml, 1.6mmol) in dichloromethane (5ml) and N,N-dimethylformamide (10ml) and the reaction stirred at room temperature for 18 hours. The mixture was evaporated under reduced pressure and the residue triturated with methanol to afford the title compound as an off-white solid, 168mg.
¹HNMR (DMSO-d₆, 400MHz) δ: 1.68-1.92 (m, 10H), 2.26 (m, 2H), 2.61-2.78 (m, 4H), 3.84-3.97 (m, 2H), 5.16 (m, 1H), 6.44 (s, 1H), 7.18 (m, 1H), 7.33 (d, 1H), 7.50 (m, 1H), 7.63 (d, 2H), 7.96 (m, 1H), 8.10 (d, 1H), 8.61 (d, 1H), 11.85 (s, 1H).
Microanalysis found: C, 59.44; H, 5.70; N, 10.52. C₂₇H₂₉FN₄O₄S;H₂O requires C, 59.76; H, 5.70; N, 10.32%.

### Example 31

### Syn-6-Hydroxy-2-methyl-quinoline-4-carboxylic acid (4-{[5-fluoro-2-(tetrahydrothiopyran-4-yloxy)-pyridine-3-carbonyl]-amino}-cyclohexyl)-amide

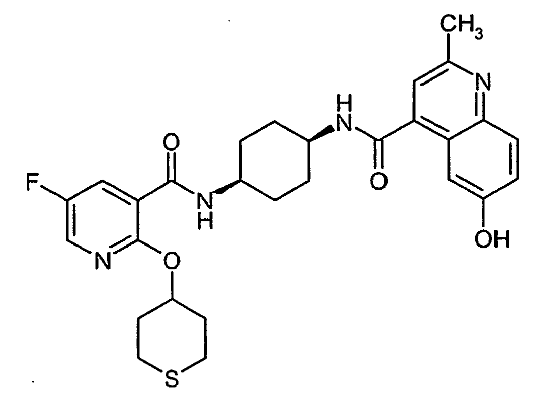

The title compound was obtained as an off-white solid in 80% yield from the amine from preparation 15a and 6-hydroxy-2-methylquinoline-4-carboxylic acid (obtained from SPECS), following the procedure described in example 30.
¹HNMR (DMSO-d₆, 400MHz) δ: 1.72-1.92 (m, 10H), 2.28 (m, 2H), 2.60 (s, 3H), 2.63-2.77 (m, 4H), 3.96 (m, 2H), 5.15 (m, 1H), 7.24 (m, 3H), 7.77 (d, 1H), 7.96 (dd, 1H), 8.11 (d, 1H), 8.26 (d, 1H), 8.54 (d, 1H), 9.89 (s, 1H).
LRMS : m/z APCl⁺ 561 [MNa]⁺
Microanalysis found: C, 60.88; H, 5.89; N, 10.21. C₂₈H₃₁FN₄O₄S;0.6H₂O requires C, 61.21; H, 5.91; N, 10.20%.

### Example 32

### Syn-8-Hydroxy-quinoline-4-carboxylic acid (4-{[5-fluoro-2-(tetrahydro-thiopyran-4-yloxy)-pyridine-3-carbonyl]-amino}-cyclohexyl)-amide

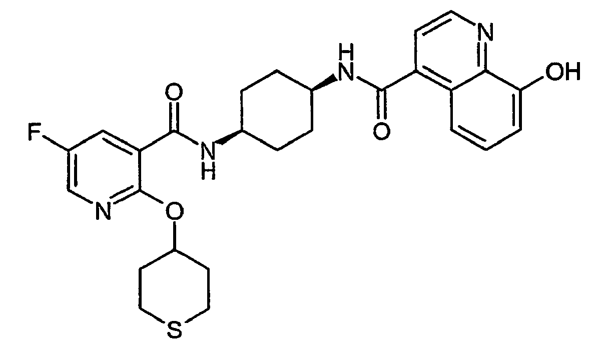

The title compound was obtained as a yellow solid 60% yield from the amine from preparation 15a and 8-hydroxyquinoline-4-carboxylic acid (obtained from Bader), following the procedure described in example 30.
¹HNMR (DMSO-d₆, 400MHz) δ: 1.75 (m, 10H), 2.25 (m, 2H), 2.66 (m, 4H), 3.95 (m, 2H), 5.14 (m, 1H), 7.08 (d, 1H), 7.48 (m, 3H), 7.96 (d, 1H), 8.11 (d, 1H), 8.26 (s, 1H), 8.62 (d, 1H), 8.86 (d, 1H), 9.87 (m, 1H).
LRMS : m/z APCl⁺ 525 [MH]⁺
Microanalysis found: C, 61.34; H, 5.60; N, 10.64. C₂₇H₂₉FN₄O₄S;0.1H₂O requires C, 61.60; H, 5.59; N, 10.64%.

### Example 33

### Syn-1H-Indazole-7-carboxylic acid (4-{[5-fluoro-2-(tetrahydro-thiopyran-4-yloxy)-pyridine-3-carbonyl]-amino}-cyclohexyl)-amide

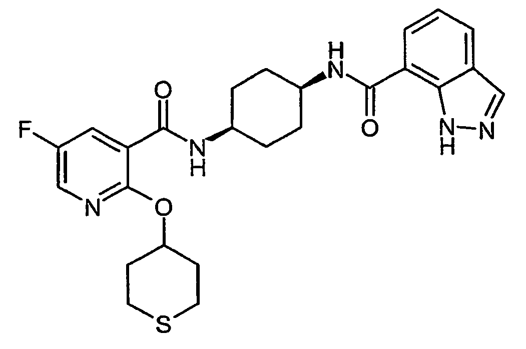

1-(3-Dimethylaminopropyl)-3-ethylcarbodiimide hydrochloride (64mg, 0.33mmol) was added to a solution of the amine hydrochloride from preparation 15a (130mg, 0.33mmol), 1-hydroxybenzotriazole hydrate (46mg, 0.33mmol), the acid from preparation 87 (45mg, 0.27mmol) and N-methylmorpholine (0.22ml, 1.11mmol) in dichloromethane (4ml) and the reaction stirred at room temperature for 18 hours. The mixture was diluted with ethyl acetate (10ml), washed with 2N hydrochloric acid (10ml) and extracted with dichloromethane (10ml). The organic solution was dried (MgSO₄) and evaporated under reduced pressure. The colourless oil was trituated with ethyl acetate and the resulting solid then purified by HPLC using an elution gradient of acetonitrile:0.1% aqueous trifluoroacetic acid (5:95 to 95:5). The product was azeotroped with dichloromethane to give the title compound.
¹HNMR (CD₃OD, 400MHz) δ: 1.75-2.05 (m, 10H), 2.35-2.50 (m, 2H), 2.65-2.85 (m, 4H), 4.05-4.25 (m, 2H), 5.28-5.38 (m, 1H), 7.25 (m, 1H), 7.90 (d, 1H), 7.95 (d, 1H), 8.08 (dd, 1H), 8.15 (m, 2H), 8.38 (br d, 1H)
LRMS: m/z APCl⁺ 498 [MH]⁺
Microanalysis found; C, 57.52; H, 5.97; N, 12.53, C₂₅H₂₈FN₅O₃S;0.4CH₂Cl₂; requires C, 57.52; H, 5.97, N, 12.53%.

### Example 34

### Syn-1H-Indazole-6-carboxylic acid (4-{[5-fluoro-2-(tetrahydro-thiopyran-4-yloxy)-pyridine-3-carbonyl]-amino}-cyclohexyl)-amide

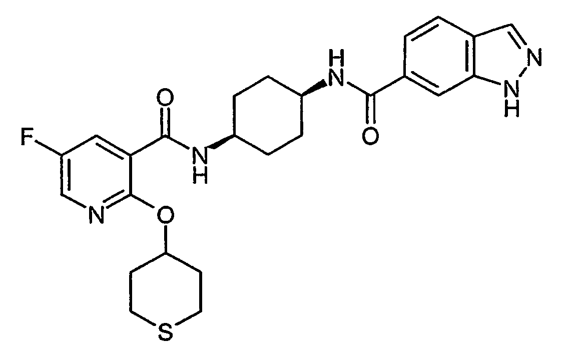

The title compound was obtained as a white solid from the amine from preparation 15a and 1H-indazole-6-carboxylic acid (WO 98/09961, ex 1(A)) following the procedure described in example 33.
¹HNMR (CD₃OD 400MHz) δ: 1.75-2.05 (m, 10H), 2.38-2.50 (m, 2H), 2.70-2.90 (m, 4H), 4.05 (m, 1H), 4.15 (m, 1H), 5.35 (m, 1H), 7.55 (d, 1H), 7.85 (d, 1H), 8.01 (s, 1H), 8.05 (dd, 1H), 8.10 (s, 1H), 8.18 (m, 1H), 8.38 (br d, 1H)
LRMS: m/z APCl⁺ 498 [MH]⁺
Microanalysis found; C, 58.57; H, 5.71; N, 13.33, C₂₅H₂₈FN₅O₃S;0.2CH₂Cl₂; requires C, 58.82; H, 5.56, N, 13.61%.

### Examples 35 to 47

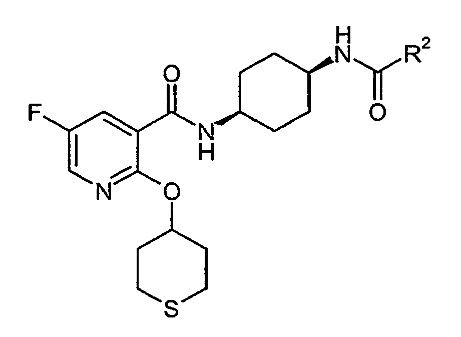

The amine hydrochloride from preparation 15a was dissolved in dichloromethane, the solution washed with 1N sodium hydroxide solution, then dried (MgSO₄) and evaporated under reduced pressure.
1-(3-Dimethylaminopropyl)-3-ethylcarbodiimide hydrochloride (200mg, 1.05mmol) was added to a solution of the freshly prepared amine (200mg, 0.57mmol), 1-hydroxybenzotriazole hydrate (93mg, 0.69mmol), the appropriate acid (0.52mmol) and N-ethyldiisopropylamine (480µl, 2.28mmol) in N,N-dimethylformamide (3ml), and the reaction stirred at room temperature for 18 hours. The mixture was partitioned between ethyl acetate and 2N hydrochloric acid and the layers separated. The organic phase was washed with additional 2N hydrochloric acid, sodium bicarbonate solution, water and brine, then dried (MgSO₄) and concentrated under reduced pressure. The crude products were purified by column chromatography on silica gel using an elution gradient of ethyl acetate:pentane (30:70 to 100:0) or using acetonitrile:dichloromethane (1:99 to 50:50). The products were then azeotroped with dichloromethane:diisopropyl ether and triturated with diisopropyl ether to afford the title compounds as white solids.

| Ex no | R² | Yield (%) | Data |
|---|---|---|---|
| 35 | 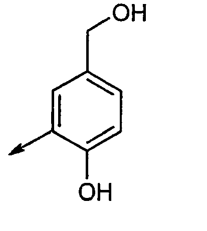 | 27 | ¹HNMR (CD₃OD, 400MHz) δ: 1.75-2.06 (m, 10H), 2.40 (m, 2H), 2.72-2.85 (m, 4H), 4.05 (m, 1H), 4.15 (m, 1H), 4.54 (s, 2H), 5.61 (m, 1H), 6.93 (d, 1H), 7.37 (d, 1H), 7.83 (s, 1H), 8.04 (m, 1H), 8.16 (d, 1H) |
| | | | LRMS: m/z APCl⁺ 504 [MH]⁺ |
| | | | Microanalysis found; C, 59.64; H, 6.09; N, 8.28. C₂₅H₃₀FN₃O₅S requires C, 59.63; H, 6.00; N, 8.34%. |
| 36 | 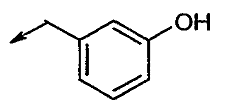 | 16 | ¹HNMR (CD₃OD, 400MHz) δ: 1.62-2.03 (m, 10H), 2.40 (m, 2H), 2.71-2.84 (m, 4H), 3.45 (s, 3H), 3.82 (m, 1H), 4.03 (m, 1H), 5.32 (m, 1H), 6.64 (d, 2H), 6.75 (m, 2H), 7.11 (m, 1H), 8.05 (m, 1H), 8.17 (d, 1H) |
| | | | LRMS: m/z APCl⁺ 488 [MH]⁺ |
| | | | Microanalysis found; C, 61.58; H, 6.37; N, 8.16. C₂₅H₃₀FN₃O₄S; 0.1 H₂O; 0.05 [(CH₃)₂CH]₂O requires C, 61.45; H, 6.30; N, 8.50%. |
| 37 ^{1A} | 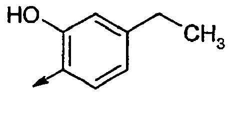 | 36 | ¹HNMR (CDCl₃, 400MHz) δ: 1.24 (t, 3H), 1.60-2.08 (m, 10H), 2.40 (m, 2H), 2.62 (q, 2H), 2.72-2.92 (m, 4H), 4.16 (m, 1H), 4.26 (m, 1H), 5.46 (m, 1H), 6.28 (m, 1H), 6.70 (d, 1H), 6.82 (s, 1H), 7.34 (d, 1H), 8.04-8.16 (m, 2H), 8.28 (m, 1H), 12.22 (m, 1H) |
| | | | LRMS: m/z ES⁺ 524 [MNa]⁺ |
| | | | Microanalysis found; C, 62.12; H, 6.36; N, 8.34, C₂₆H₃₂FN₃O₄S; requires C, 62.26; H, 6.43; N, 8.34%. |
| 38² | 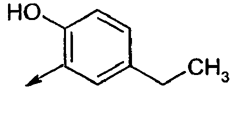 | 47 | ¹HNMR (CDCl₃, 400MHz) δ: 1.24 (t, 3H), 1.60-2.10 (m, 10H), 2.40 (m, 2H), 2.62 (q, 2H), 2.70-2.92 (m, 4H), 4.16 (m, 1H), 4.26 (m, 1H), 5.48 (m, 1H), 6.38 (m, 1H), 6.94 (d, 1H), 7.18 (s, 1H), 7.26(m, 1H), 8.06 (d, 1H), 8.14 (m, 1H), 8.28 (m, 1H), 12.00 (m, 1H) |
| | | | LRMS: m/z ES⁺ 524 [MNa]⁺ |
| | | | Microanalysis found; C, 62.03; H, 6.38; N, 8.33, C₂₆H₃₂FN₃O₄S; requires C, 62.26; H, 6.43; N, 8.38%. |
| 39^{3A} | 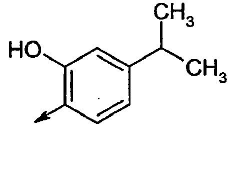 | 44 | ¹HNMR (CDCl₃, 400MHz) δ: 1.24 (m, 6H), 1.60-2.08 (m, 10H), 2.40 (m, 2H), 2.74-2.82 (m, 5H), 4.16 (m, 1H), 4.26 (m, 1H), 5.46 (m, 1H), 6.30 (m, 1H), 6.74 (d, 1H), 6.86 (s, 1H), 7.36 (d, 1H), 8.04-8.16 (m, 2H), 8.28 (m, 1H), 12.20 (m, 1H) |
| | | | LRMS: m/z ES⁺ 538 [MNa]⁺ |
| | | | Microanalysis found; C, 62.68; H, 6.61; N, 8.07, C₂₇H₃₄FN₃O₄S; requires C, 62.89; H, 6.65; N, 8.15%. |
| 40^{4A} | 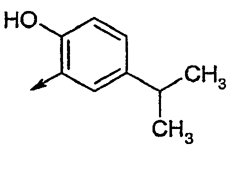 | 42 | ¹HNMR (CDCl₃, 400MHz) δ: 1.22 (m, 6H), 1.60-2.08 (m, 10H), 2.40 (m, 2H), 2.74-2.82 (m, 5H), 4.16 (m, 1H), 4.26 (m, 1H), 5.46 (m, 1H), 6.30 (m, 1H), 6.74 (d, 1H), 6.86 (s, 1H), 7.36 (d, 1H), 8.04-8.16 (m, 2H), 8.28 (m, 1H), 12.20 (brs, 1H) |
| | | | LRMS: m/z ES⁺ 538 [MNa]⁺ |
| | | | Microanalysis found; C, 62.58; H, 6.61; N, 8.18, C₂₇H₃₄FN₃O₄S; requires C, 62.89; H, 6.65; N, 8.15%. |
| 41 | 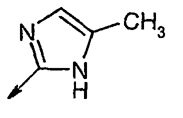 | 16 | ¹HNMR (CD₃OD, 400MHz) δ: 1.79-2.15 (m, 10H), 2.23 (brs, 3H), 2.38 (m, 2H), 2.68-2.84 (m, 4H), 4.01-4.12 (m, 2H), 5.31 (m, 1H), 6.72-6.99 (m, 1H), 8.03 (m, 1H), 8.14 (d, 1H) |
| | | | LRMS: m/z APCl⁺ 462 [MH]⁺ |
| | | | Microanalysis found; C, 57.03; H, 6.13; N, 14.83. C₂₂H₂₈FN₅O₃S; 0.15 H₂O requires C, 56.92; H, 6.14; N, 15.08%. |
| 42 | 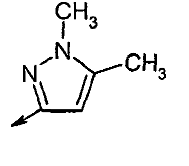 | 67 | ¹HNMR (CD₃OD, 400MHz) δ: 1.71-2.06 (m, 10H), 2.30 (s, 3H), 2.4 (m, 2H), 2.70-2.83 (m, 4H), 3.80 (s, 3H), 4.00 (m, 1H), 4.21 (m, 1H), 5.34 (m, 1H), 6.46 (s, 1H), 8.03 (m, 1H), 8.17 (d, 1H) |
| | | | LRMS: m/z APCl⁺ 476 [MH]⁺ |
| | | | Microanalysis found; C, 57.99; H, 6.44; N, 14.43. C₂₃H₃₀FN₅O₃S requires C, 58.09; H, 6.36; N, 14.73%. |
| 43⁶ | 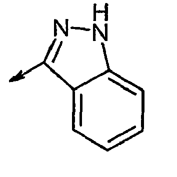 | 27 | ¹HNMR (CD₃OD, 400MHz) δ: 1.74-2.06 (m, 10H), 2.42 (m, 2H), 2.71-2.87 (m, 4H), 4.15 (m, 2H), 5.35 (m, 1H), 7.23 (m, 1H), 7.39 (m, 1H), 7.52 (d, 1H), 7.63 (s, 1H), 8.11 (m, 2H), 8.22 (d, 1H) |
| | | | LRMS: m/z APCl⁺ 498 [MH]⁺ |
| | | | Microanalysis found; C, 59.07; H, 5.66; N, 13.67. C₂₅H₂₈FN₅O₃S; 0.5 H₂O requires C, 59.27; H, 5.77; N, 13.82%. |
| 44⁷ | 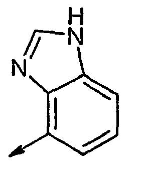 | 15 | ¹HNMR (CD₃OD, 400MHz) δ: 1.81-2.03 (m, 10H), 2.41 (m, 2H), 2.72 (m, 4H), 4.14 (m, 1H), 4.25 (m, 1H), 5.30 (m, 1H), 7.37 (m, 1H), 7.72 (d, 1H), 7.78 (s, 1H), 7.97 (m, 1H), 8.12 (m, 2H), 8.32 (m, 1H), 8.39 (m, 1H) |
| | | | LRMS: m/z APCl⁺ 498 [MH]⁺ |
| | | | Microanalysis found; C, 60.00; H, 5.88; N, 13.58. C₂₅H₂₈FN₅O₃S; 0.25 H₂O requires C, 59.80; H, 5.72; N, 13.95%. |
| 45⁸ | 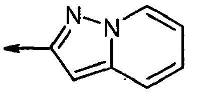 | 69 | ¹HNMR (CD₃OD, 400MHz) δ: 1.79-2.14 (m, 10H), 2.39 (m, 2H), 2.71-2.83 (m, 4H), 4.08 (m, 1H), 4.14 (m, 1H), 5.36 (m, 1H), 6.98 (t, 1H), 7.02 (s, 1H), 7.24 (t, 1H), 7.70 (d, 1H), 8.04 (m, 1H), 8.17 (d, 1H), 8.56 (d, 1H) |
| | | | LRMS: m/z APCl⁺ 498 [MH]⁺ |
| | | | Microanalysis found; C, 60.18; H, 5.72; N, 13.93. C₂₅H₂₈FN₅O₃S requires C, 60.35; H, 5.67; N, 14.07%. |
| 46⁹ | 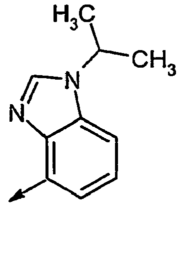 | 50 white solid | ¹HNMR (CD₃OD, 400MHz) δ: 1.65 (d, 6H), 1.82-2.03 (m, 10H), 2.39 (m, 2H), 2.68 (m, 4H), 4.12 (m, 1H), 4.23 (m, 1H), 4.82 (m, 1H), 5.31 (m, 1H), 7.41 (m, 1H), 7.82 (d, 1H), 7.99 (d, 1H), 8.08 (m, 1H), 8.16 (m, 1H), 8.43 (s, 1H) |
| | | | LRMS: m/z APCl⁺ 540 [MH]⁺ |
| | | | Microanalysis found; C, 61.80; H, 6.40; N, 12.85. C₂₈H₃₄FN₅O₃S; 0.25 H₂O requires C, 61.80; H, 6.39; N, 12.87%. |

| | | | |
|---|---|---|---|
| 1 = 4-ethyl-2-hydroxybenzoic acid was prepared as described in US 4012407 2= 5-ethyl-2-hydroxybenzoic acid was prepared as described in J. Med. Chem. 14; 1971; 265. 3= 2-hydroxy-4-isopropyl-benzoic acid was prepared as described in preparation 68 4= 2-hydroxy-5-isopropyl-benzoic acid was prepared as described in preparation 67 5= 4-Hydroxy-2-methoxy-benzoic acid was prepared as described in preparation 74 6= 1H-indazole-3-carboxylic acid was prepared as decribed in J. Amer. Chem. Soc. 1952;2009;74 7= 1H-Benzoimidazole-4-carboxylic acid was prepared as described in preparation 28 8= Pyrazolo[1,5-a]pyridine-2-carboxylic acid was prepared as described in J. Med. Chem, 2001; 44; 2691 9= 1-Isopropyl-1H-benzoimidazole-4-carboxylic acid was prepared as described in preparation 30 A=the compounds were isolated by trituration from ether | | | |

### Examples 47 to 50

The following compounds of general formula: 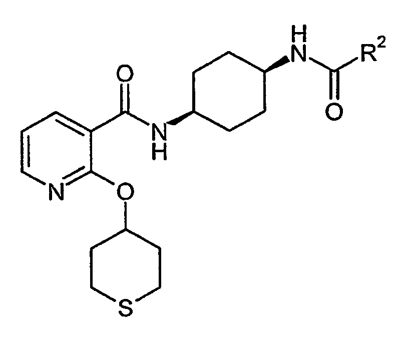 were prepared from the amine from preparation 15a and the appropriate acids, following a similar procedure to that described in example 35 to 46, and the compounds were purified by HPLC using acetonitrile:0.1%aqueous trifluoroacetic acid (5:95 to 95:5), and the product azeotroped with dichloromethane to give the title compounds.

| Ex no | R² | Yield (%) | Data |
|---|---|---|---|
| 47 | 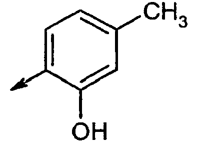 | 12 | ¹HNMR (CDCl₃, 400MHz) δ: 1.64-1.88 (m, 4H), 1.90-2.08 (m, 6H), 2.33 (s, 3H), 2.42 (m, 2H), 2.74-2.90 (m, 4H), 4.15 (brs, 1H), 4.27 (brs, 1H), 5.60 (m, 1H), 6.32 (d, 1H), 6.68 (d, 1H), 6.79 (s, 1H), 7.11 (m, 1H), 7.32 (d, 1H), 8.05 (d, 1H), 8.26 (d, 1H), 8.58 (d, 1H) |
| | | | LRMS : m/z ES⁺ 470 [MH]⁺ |
| | | | Microanalysis found; C, 60.11; H, 6.46; N, 7.97. C₂₅H₃₁N₃O₄S;0.4CH₂Cl₂ requires C, 60.58; H, 6.37; N, 8.34%. |
| 48 | 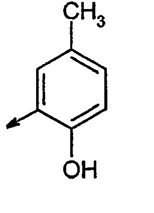 | 14 | ¹HNMR (CDCl₃, 400MHz) δ: 1.64-1.90 (m, 4H), 1.91-2.09 (m, 6H), 2.30 (t, 3H), 2.41 (m, 2H), 2.74-2.92 (m, 4H), 4.16 (brs, 1H), 4.28 (brs, 1H), 5.62 (m, 1H), 6.43 (d, 1H), 6.88 (d, 1H), 7.09 (m, 1H), 7.19 (m, 2H), 8.09 (d, 1H), 8.24 (d, 1H), 8.57 (d, 1H) |
| | | | LRMS : m/z ES⁺ 470 [MH]⁺ |
| | | | Microanalysis found; C, 61.69; H, 6.53; N, 8.35. C₂₅H₃₁N₃O₄S;0.25CH₂Cl₂ requires C, 61.79; H, 6.47; N, 8.56%. |
| 49 | 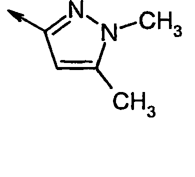 | 7 | ¹HNMR (CDCl₃, 400MHz) δ: 1.74-2.08 (m, 10H), 2.40-2.52 (m, 2H), 2.68 (s, 3H), 2.82 (m, 4H), 3.80 (s, 3H), 4.08-4.21 (m, 2H), 5.41 (m, 1H), 6.59 (s, 1H), 7.06 (q, 1H), 7.13 (d, 1H), 8.10 (d, 1H), 8.22 (d, 1H), 8.50 (d, 1H) |
| | | | LRMS : m/z ES⁺ 480 [MNa]⁺ |
| | | | Microanalysis found; C, 55.67; H, 6.19; N, 13.40. C₂₃H₃₁N₅O₃S;0.6CH₂Cl₂ requires C, 55.74; H, 6.38; N, 13.77%. |
| 50¹ | 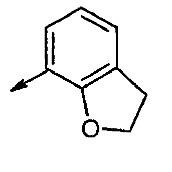 | 38 | ¹HNMR (CDCl₃, 400MHz) δ: 1.62-1.86 (m, 6H), 1.88-2.02 (m, 4H), 2.41-2.53 (m, 2H), 2.72-2.88 (m, 4H), 3.28 (t, 2H), 4.20 (brs, 2H), 4.78 (t, 2H), 5.40 (m, 1H), 6.98 (t, 1H), 7.07 (m, 1H), 7.34 (d, 1H), 7.74 (d, 1H), 7.96 (d, 1H), 8.02 (d, 1H), 8.22 (d, 1H), 8.57 (d, 1H) |
| | | | LRMS : m/z ES⁺ 482 [MH]⁺ |
| | | | Microanalysis found; C, 62.98; H, 6.45; N, 8.73. C₂₆H₃₁N₃O₄S;0.2CH₂Cl₂ requires C, 63.11; H, 6.35; N, 8.43%. |

| | | | |
|---|---|---|---|
| 1 =purified by crystallisation from methanol | | | |

### Example 51

### Syn-3-Hydroxymethyl-imidazo[1,2-a]pyridine-8-carboxylic acid (4-{[5-fluoro -2-(tetrahydro-thiopyran-4-yloxy)-pyridine-3-carbonyl]-amino}-cyclohexyl)-amide

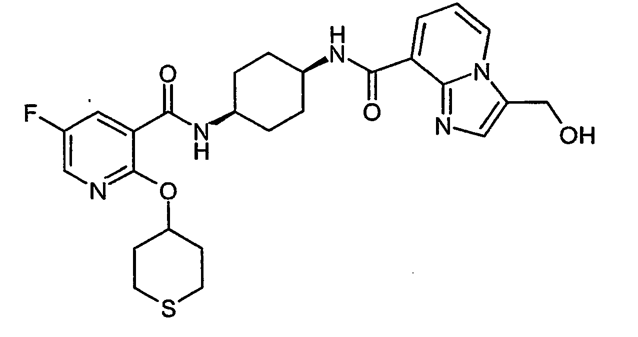

A mixture of the amine hydrochloride form preparation 15a (1.2g, 3.1mmol) and triethylamine (1.4ml, 10mmol) in N,N-dimethylformamide (10ml) was stirred at 60°C for 45 minutes, then cooled to room temperature. O-(1H-Benzotriazol-1-yl)-N,N,N'N'-tetramethyluronium hexafluorophosphate (610mg, 2.6mmol) and the acid from preparation 27 (500mg, 2.6mmol) were added and the reaction stirred at room temperature for 48 hours. The solution was concentrated under reduced pressure and the residue diluted with sodium bicarbonate solution (40ml), then extracted with ethyl acetate (4x40ml). The combined organic extracts were washed with brine, then dried (MgSO₄) and evaporated under reduced pressure. The crude product was triturated with hot methanol, the resulting solid filtered off and dried to afford the title compound as a white solid, 500mg.
¹HNMR (DMSO-d₆, 400MHz) δ: 1.69-1.90 (m, 10H), 2.24 (m, 2H), 2.58-2.75 (m, 4H), 4.00(m, 1H), 4.05 (m, 1H), 4.82 (d, 2H), 5.16 (m, 1H), 5.32 (m, 1H), 7.15 (m, 1H), 7.62 (s, 1H), 7.94 (dd, 1H), 8.04 (d, 1H), 8.26 (m, 2H), 8.60 (d, 1H), 10.38 (d, 1H).
LRMS: m/z ES⁺ 529 [MH]⁺

### Example 52

### Syn-5-Fluoro-N-(4-{[1-(2-hydroxy-ethyl)-5-methyl-1H-pyrazole-3-carbonyl]-amino}-cyclohexyl)-2-(tetrahydro-thiopyran-4-yloxy)-nicotinamide

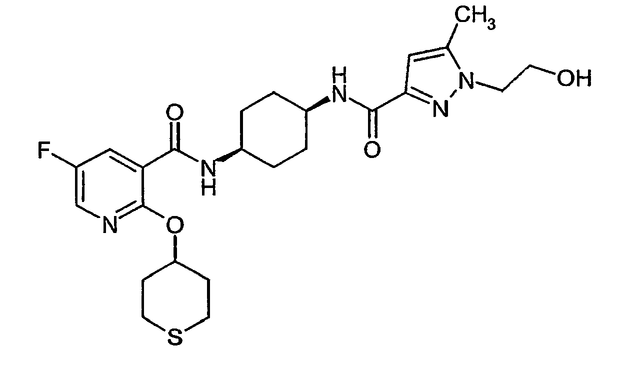

A mixture of the compound from preparation 82 (270mg, 0.53mmol), acetic acid (4ml), water (1ml) and tetrahydrofuran (2ml) was stirred at 75°C for 18 hours. The cooled reaction was diluted with water (5ml) and basified using solid potassium carbonate. The mixture was extracted into ethyl acetate and the combined organic extracts were washed with water, brine, then dried (MgSO₄) and evaporated under reduced pressure. The residue was purified by column chromatography on silica gel using an elution gradient of dichloromethane:methanol:0.88 ammnoia (99.5:0.5:0 to 95:5:0.5) and the product triturated with diisopropyl ether to give the title compound, 74mg.
¹HNMR (CDCl₃, 400MHz) δ: 1.63-2.03 (m, 10H), 2.33 (s, 3H), 2.43 (m, 2H), 2.72-2.83 (m, 4H), 4.03 (t, 2H), 4.06-4.27 (m, 4H), 5.33 (m, 1H), 6.60 (s, 1H), 6.87 (d, 1H), 8.03-8.13 (m, 2H), 8.27 (m, 1H)
LRMS: m/z APCl⁺ 506 [MH]⁺
Microanalysis found; C, 57.06; H, 6.50; N, 13.56. C₂₄H₃₂FN₅O₄S requires C, 57.01; H, 6.38; N, 13.85%.

### Example 53

### Syn-1-(2-Hydroxy-ethyl)-1H-indazole-3-carboxylic acid (4-{[5-fluoro-2-(tetrahydro-thiopyran-4-yloxy)-pyridine-3-carbonyl]-amino}-cyclohexyl)-amide

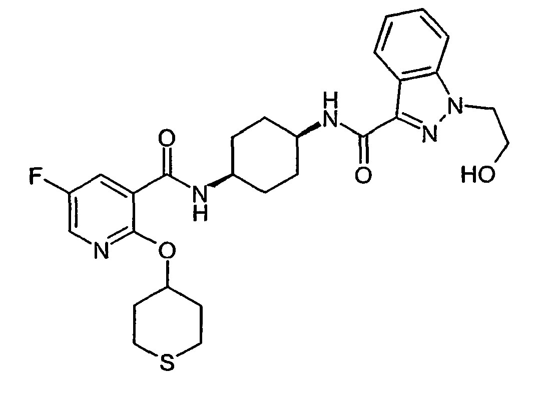

The title compound was obtained from the compound of preparation 81, following a similar procedure to that described in example 52.
¹HNMR (DMSO-d₆, 400MHz) δ: 1.74 (m, 8H), 1.92 (m, 2H), 2.27 (m, 2H), 2.65 (m, 2H), 2.78 (m, 2H), 3.81 (q, 2H), 3.96 (m, 1H), 4.02 (m, 1H), 4.45 (t, 2H), 4.85 (t, 1H), 5.19 (m, 1H), 7.20 (m, 1H), 7.40 (m, 1H), 7.60 (d, 1H), 7.71 (d, 1H), 7.94 (m, 1H), 8.08 (m, 2H).
LRMS : m/z ES⁺ 564 [MNa⁺]

### Example 54

### Syn-N-(4-{[5-Ethyl-1-(2-hydroxy-ethyl)-1H-pyrazole-3-carbonyl]-amino}-cyclohexyl)-5-fluoro-2-(tetrahydro-thiopyran-4-yloxy)-nicotinamide

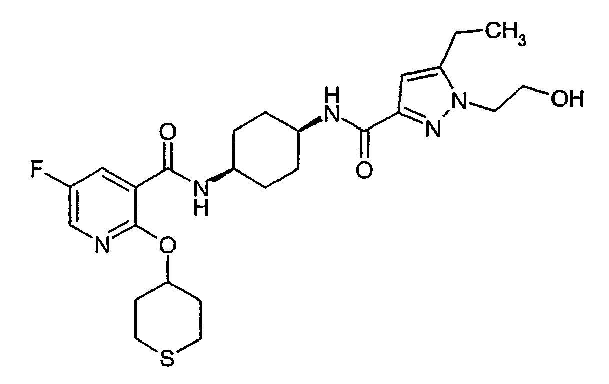

1-(3-Dimethylaminopropyl)-3-ethylcarbodiimide hydrochloride (215mg, 1.12mmol) was added to a solution of the amine hydrochloride from preparation 15a (300mg, 0.77mmol), 1-hydroxybenzotriazole hydrate (100mg, 0.57mmol), the acid from preparation 41 (300mg, 1.12mmol) and triethylamine (260µl, 1.87mmol) in N,N-dimethylformamide (3ml), and the reaction stirred at room temperature for 18 hours. The mixture was partitioned between ethyl acetate and 10% citric acid solution, and the layers separated. The organic phase was washed with water, sodium bicarbonate solution, dried (MgSO₄) and evaporated under reduced pressure. The residue was dissolved in tetrahydrofuran (3ml), acetic acid (4ml) and water (2ml) added, and the solution stirred at 80°C for 5 hours. The mixture was partitioned between ethyl acetate and water, the layers separated, and the organic phase washed with sodium bicarbonate solution, water and brine, then dried (MgSO₄) and evaporated under reduced pressure. The crude product was purified by column chromatography on silica gel using an elution gradient of dichloromethane:methanol (99:1 to 92:8), and the product triturated with diisopropyl ether to afford the title compound as a white solid.
¹HNMR (CD₃OD, 400MHz) δ: 1.29 (t, 3H), 1.70-2.04 (m, 10H), 2.40 (m, 2H), 2.69-2.87 (m, 6H), 3.91 (t, 2H), 4.02 (m, 1H), 4.15 (m, 1H), 4.20 (t, 2H), 5.35 (m, 1H), 6.52 (s, 1H), 8.04 (m, 1H), 8.16 (m, 1H)
LRMS: m/z APCl⁺ 520 [MH]⁺
Microanalysis found; C, 57.76; H, 6.69; N, 13.22. C₂₅H₃₄FN₅O₄S requires C, 57.79; H, 6.60; N, 13.48%.

### Example 55

### Syn-N-(4-{[5-Ethyl-2-(2-hydroxy-ethyl)-2H-pyrazole-3-carbonyl]-amino}-cyclohexyl)-5-fluoro-2-(tetrahydro-thiopyran-4-yloxy)-nicotinamide

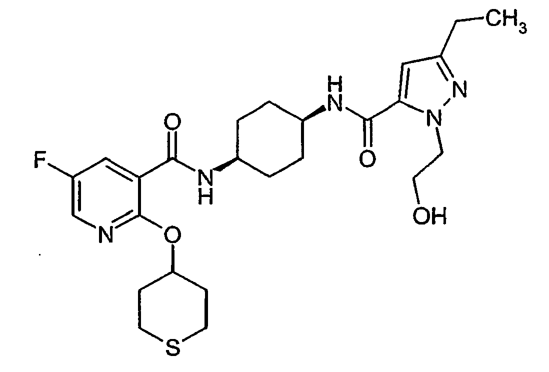

The title compound was obtained as a white solid in 40% yield from the amine from preparation 15a and the acid from preparation 40 following the procedure described in example 54.
¹HNMR (CD₃OD, 400MHz) δ: 1.23 (t, 3H), 1.71-2.04 (m, 10H),
2.41 (m, 2H), 2.61 (q, 2H), 2.75-2.87 (m, 4H), 3.84 (t, 2H), 3.97 (m, 1H), 4.14 (m, 1H), 4.52 (t, 2H), 5.34 (m, 1H), 6.57 (s, 1H), 8.06 (m, 1H), 8.16 (m, 1H)
LRMS: m/z APCl⁺ 520 [MH]⁺
Microanalysis found; C, 57.05; H, 6.55; N, 13.16. C₂₅H₃₄FN₅O₄S; 0.33 H₂O requires C, 57.13; H, 6.65; N, 13.32%.

### Example 56

### Syn-5-Fluoro-N-(4-{[2-(2-hydroxy-ethyl)-5-isopropyl-2H-pyrazole-3-carbonyl ]-amino}-cyclohexyl)-2-(tetrahydro-thiopyran-4-yloxy)-nicotinamide

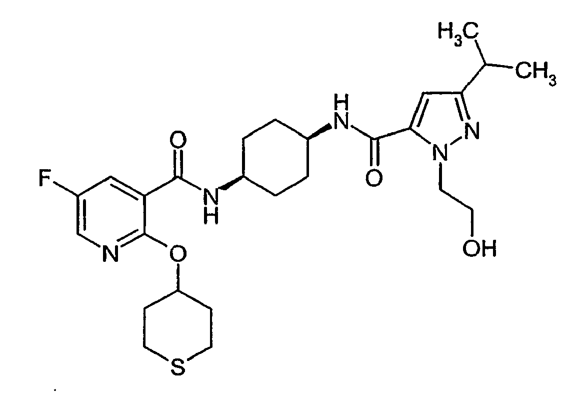

1-(3-Dimethylaminopropyl)-3-ethylcarbodiimide hydrochloride (250mg, 1.3mmol) was added to a solution of the amine hydrochloride from preparation 15a (390mg, 1.0mmol), 1-hydroxybenzotriazole hydrate (162mg, 1.2mmol), the acid from preparation 42 (282mg, 1.0mmol) and N-ethyldiisopropylamine (388mg, 3.0mmol) in N,N-dimethylformamide (1ml) and dichloromethane (10ml), and the reaction stirred at room temperature for 18 hours. The mixture was diluted with ethyl acetate and washed with 0.2N citric acid solution, sodium bicarbonate solution and brine. The organic phase was dried (MgSO₄) and evaporated under reduced pressure. The residue was dissolved in tetrahydrofuran (2ml), acetic acid (4ml) and water (1ml) added, and the solution stirred at 60°C for 4 hours. The mixture was partitioned between ethyl acetate and water, the layers separated, and the organic phase washed with 0.88 ammonia solution and brine, then dried (MgSO₄) and evaporated under reduced pressure. The crude product was purified by column chromatography on silica gel using an elution gradient of dichloromethane:ethyl acetate (100:0 to 0:100) to afford the title compound as a solid, 356mg.
¹HNMR (CDCl₃, 400MHz) δ: 1.29 (d, 6H), 1.68 (m, 2H), 1.83 (m, 2H), 1.96 (m, 6H), 2.41 (m, 2H), 2.81 (m, 4H), 3.00 (m, 1H), 4.04 (t, 2H), 4.11 (m, 1H), 4.25 (m, 1H), 4.62 (t, 2H), 5.46 (m, 1H), 6.43 (s, 1H), 6.61 (d, 1H), 8.06 (d, 1H), 8.11 (d, 1H), 8.28 (dd, 1H).
LRMS: m/z ES⁺ 556 [MNa]⁺
Microanalysis found: C, 57.88; H, 6.82; N, 12.55. C₂₆H₃₆FN₅O₄S;0.4H₂O requires C, 57.74; H, 6.86; N, 12.95%.

### Example 57

### Syn-5-Fluoro-N-(4-{[1-(2-hydroxy-ethyl)-5-isopropyl-1H-pyrazole-3-carbonyl ]-amino}-cyclohexyl)-2-(tetrahydro-thiopyran-4-yloxy)-nicotinamide

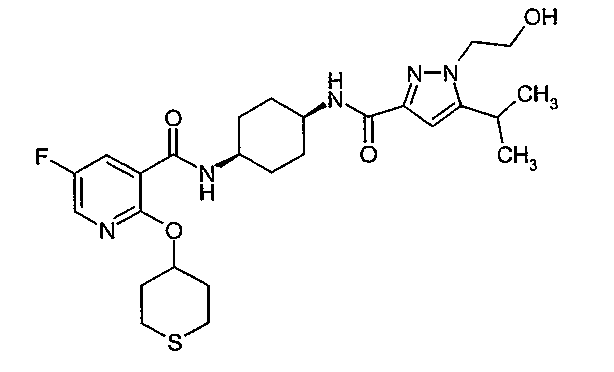

The title compound was obtained as a solid in 42% yield, after crystallisation from ether, from the amine from preparation 15a and the acid from preparation 43, following the procedure described in example 56.
¹HNMR (CDCl₃, 400MHz) δ: 1.27 (d, 6H), 1.68 (m, 2H), 1.81 (m, 2H), 1.94 (m, 6H), 2.43 (m, 2H), 2.82 (m, 4H), 2.98 (m, 1H), 4.04 (t, 2H), 4.13 (m, 1H), 4.21 (m, 3H), 5.33 (m, 1H), 6.65 (s, 1H), 6.87 (d, 1H), 8.05 (d, 1H), 8.08 (d, 1H), 8.26 (dd, 1H).
LRMS: m/z ES⁺ 534 [MH]⁺
Microanalysis found: C, 58.38; H, 6.83; N, 13.03. C₂₆H₃₆FN₅O₄S requires C, 58.52; H, 6.80; N, 13.12%.

### Example 58

### Syn-N-[4-(2,4-Dihydroxy-benzoylamino)-cyclohexyl]-5-fluoro-2-(tetrahydro-thiopyran-4-yloxy)-nicotinamide

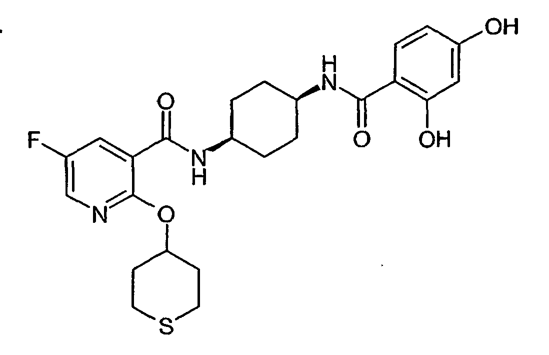

Palladium black (130mg) was added to a solution of the compound from Preparation 88 (70mg, 0.12mmol) in formic acid (10ml, 4.4% in ethanol) and N,N-dimethylformamide (5ml), and the reaction stirred under nitrogen for 4 hours. The mixture was filtered through Arbocel® and the filtrate basified using sodium bicarbonate, and evaporated under reduced pressure. The residue was partitioned between water and ethyl acetate and the layers separated. The organic phase was washed with water and brine, then dried (MgSO₄) and concentrated under reduced pressure. The crude product was purified by column chromatography on silica gel using an elution gradient of dichloromethane:methanol (99:1 to 97:3) to afford the title compound, 41 mg.
¹HNMR (DMSO-d₆, 400MHz) δ: 1.60-2.00 (m, 10H), 2.28 (m, 2H), 2.60-2.80 (m, 4H), 3.78-4.00 (m, 2H), 5.18 (m, 1H), 6.20-6.30 (m, 2H), 7.74 (m, 1H), 7.98 (m, 1H), 8.14 (m, 2H), 8.28 (d, 1H), 10.00 (s, 1H), 12.60 (s, 1H)
LRMS: m/z ES⁺ 512 [MNa]⁺
Microanalysis found; C, 58.85; H, 5.79; N, 8.51, C₂₄H₂₈FN₃O₅S; requires C, 58.88; H, 5.76; N, 8.58 %.

### Example 59

### Syn-5-Fluoro-N-[4-(2-hydroxy-5-trifluoromethyl-benzoylamino)-cyclohexyl]-2 -(tetrahydro-thiopyran-4-yloxy)-nicotlnamide

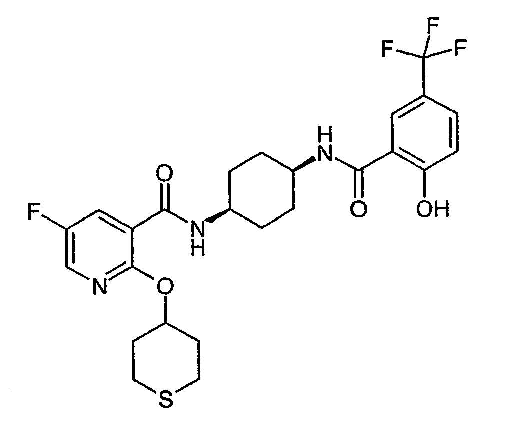

Palladium black (500mg) was added to a solution of the compound from preparation 79 (704mg, 1.11mmol) in formic acid (2.2g), ethanol (47.8g) and N,N-dimethylformamide (50ml) and the mixture stirred under nitrogen for 24 hours. The mixture was filtered through Arbocel®, washing through with additional ethanol (150ml), and the filtrate neutralised by the addition of saturated sodium bicarbonate solution (75ml). The mixture was concentrated under reduced pressure, diluted with water (50ml) and this aqueous solution extracted with ethyl acetate (3x100ml). The combined organic extracts were washed with water (2x100ml), brine (150m1), then dried (MgSO₄) and evaporated under reduced pressure. The residue was triturated with ether to give the title compound as a solid, 303mg.
¹HNMR (CD₃OD, 400MHz) δ: 1.77-2.03 (m, 10H), 2.36-2.42 (m, 2H), 2.69-2.82 (m, 4H), 4.06-4.15 (m, 2H), 5.33 (m, 1H), 7.03 (d, 1H), 7.60 (d, 1H), 8.05 (m, 1H), 8.16 (d, 1H), 8.20 (s, 1H).
LRMS : m/z APCl⁺ 542 [MH⁺]

### Example 60

### Syn-3-Methyl-imidazo[1,2-a]pyridine-8-carboxylic acid (4-{[5-fluoro-2-(tetrahydrothiopyran-4-yloxy)-pyridine-3-carbonyl]-amino} -cyclohexyl)-amide

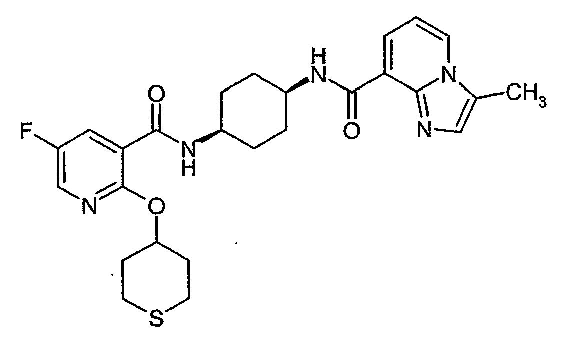

Imidazole (36mg, 0.53mmol), triphenylphosphine (139mg, 0.53mmol) and iodine (134mg, 0.53mmol) were added to a solution of the compound from example 54 (200mg, 0.4mmol) in dichloromethane (10ml), and the suspension stirred at room temperature for 48 hours. Sodium methylmercaptide (84mg, 1.2mmol) was added and the reaction stirred at room temperature for a further 24 hours. The reaction was quenched by the addition of sodium bicarbonate solution, and extracted with dichloromethane (3x25ml). The combined organic extracts were dried (MgSO₄) and evaporated under reduced pressure. The crude product was purified by column chromatography on silica gel using an elution gradient of ethyl acetate:pentane (10:90 to 100:0) to afford the title compound.
¹HNMR (CDCl₃, 400MHz) δ: 1.60-2.05 (m, 10H), 2.46 (m, 2H), 2.53 (s, 3H), 2.74 (m, 4H), 4.21 (m, 1H), 4.31 (m, 1H), 5.25 (m, 1H), 7.06 (m, 1H), 7.48 (m, 2H), 7.65 (m, 1H), 8.05 (m, 1H), 8.15 (m, 1H), 8.28 (m, 1H), 10.58 (m, 1H).

### Example 61

### Syn-5-Chloro-N-[4-(2-hydroxy-5-hydroxymethyl-benzoylamino)-cyclohexyl]-2-(tetrahydro-thiopyran-4-yloxy)-nicotinamide

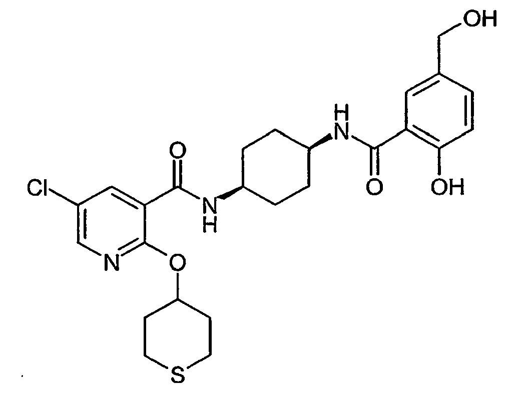

1-(3-Dimethylaminopropyl)-3-ethylcarbodiimide hydrochloride (74mg, 0.39mmol) was added to a solution of the amine hydrochloride from preparation 16 (111mg, 0.26mmol), 1-hydroxybenzotriazole hydrate (35mg, 0.20mmol), the acid from preparation 65 (400mg, 2.6mmol) and N-ethyldiisopropylamine (170µl, 0.98mmol) in N,N-dimethylformamide (3ml), and the reaction stirred at room temperature for 18 hours. The mixture was partitioned between dichloromethane (10ml) and 2N hydrochloric acid (15ml), and the layers separated. The organic phase was evaporated under reduced pressure and the residue was purified by column chromatography on silica gel to give the title compound.
¹HNMR (CD₃OD, 400MHz) δ: 1.77-2.04 (m, 10H), 2.38 (m, 2H), 2.69-2.83 (m, 4H), 4.05 (m, 1H), 4.14 (m, 1H), 4.53 (s, 2H), 5.37 (m, 1H), 6.88 (d, 1H), 7.39 (m, 1H), 7.82 (m, 1H), 8.21 (m, 2H)
HRMS: m/z ES⁺ 520.1662, [C₂₅H₃₀ClN₃O₅SH]⁺ requires 520.1668

### Example 62

### Syn-N-[4-(2-Hydroxy-5-hydroxymethyl-benzoylamino)-cyclohexyl]-5-methyl-2-(tetrahydro-thiopyran-4-yloxy)-nicotinamide

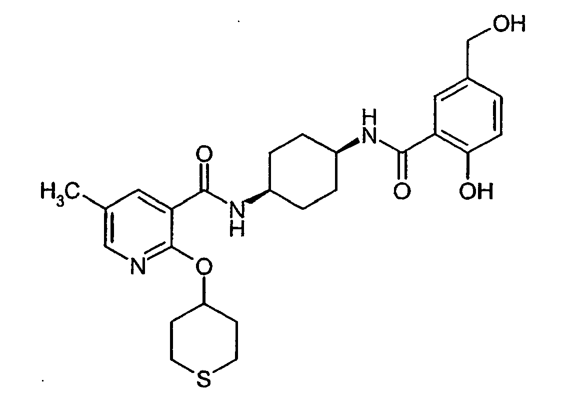

The title compound was obtained from the amine from preparation 17 and the acid from preparation 65, following the procedure described in example 86.
¹HNMR (CD₃OD, 400MHz) δ: 1.76-2.03 (m, 10H), 2.31 (s, 3H), 2.39 (m, 2H), 2.69-2.83 (m, 4H), 4.05 (m, 1H), 4.14 (m, 1H), 4.53 (s, 2H), 5.37 (m, 1H), 6.88 (d, 1H), 7.39 (m, 1H), 7.82 (m, 1H), 8.11 (m, 2H)
HRMS: m/z ES⁺ 500.2209, [C₂₆H₃₃N₃O₅SH]⁺ requires 500.2214

### Example 63

### Syn-5-Fluoro-N-[4-(2-hydroxy-4-methyl-benzoylamino)-cyclohexyl]-2-(tetrahydro-thiopyran-4-yloxy)-nicotinamide

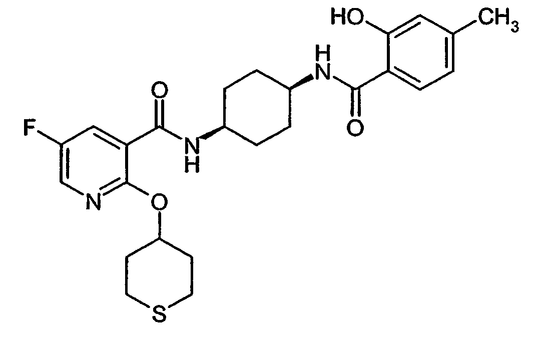

A mixture of the amine from preparation 78 (150mg, 0.37mmol), tetrahydrothiopyran-4-ol (WO 94/14793, pg 77) (200mg, 1.69mmol), and cesium carbonate (603mg, 1.85mmol) in acetonitrile (5ml) was stirred at 90°C for 42 hours. The cooled mixture was diluted with 2N hydrochloric acid and extracted with ethyl acetate. The combined organic extracts were washed with water then brine, dried (MgSO₄) and evaporated under reduced pressure. The crude product was purified by column chromatography on silica gel using an elution gradient of ethyl acetate:cyclohexane (10:90 to 60:40) to give the title compound, 130mg.
¹HNMR (CDCl₃, 400MHz) δ: 1.60-2.10 (m, 10H), 2.30-2.50 (m, 5H), 2.70-2.94 (m, 4H), 4.06-4.34 (m, 2H), 5.46 (m, 1H), 6.28 (m, 1H), 6.68 (1H, d), 6.80 (s, 1H), 7.32 (d, 1H), 8.00-8.18 (m, 2H), 8.28 (m, 1H), 12.20 (brs, 1H)
LRMS: m/z ES⁺ 510 [MNa]⁺
Microanalysis found; C, 61.31; H, 6.18; N, 8.56, C₂₅H₃₀FN₃O₄S requires C, 61.58; H, 6.20; N, 8.62%.

### Alternative method:

N-Methylmorpholine (11.16ml, 101.7mmol), 1-hydroxybenzotriazole hydate (7.49g, 55.5mmol) and 1-(3-dimethylaminopropyl)-3-ethylcarbodiimide hydrochloride (10.63g, 55.5mmol) were added portionwise to a suspension of the amine from preparation (18g, 46.2mmol) in N,N-dimethylformamide (180ml). A solution of 4-Methyl salicylic acid (8.43g, 55.5mmol) in N,N-dimethylformamide (40ml) was added dropwise over 90 minutes, and once addition was complete, the reaction was stirred at room temperature for 72 hours. The mixture was concentrated under reduced pressure and the residue suspended in a mixture of tetrahydrofuran and 1 N sodium hydroxide solution, and the mixture stirred at room temperature for 1 hour. The tetrahydrofuran was removed *in vacuo* and the residual aqueous solution was diluted with water (750ml), and extracted with dichloromethane (2L in total). The combined organic solutions were washed with 2N hydrochloric acid (150ml), dried (MgSO₄) and evaporated under reduced pressure. The residue was suspended in methanol (250ml), the suspension stirred at room temperature for 18 hours. The resulting solid was filtered off, washed with methanol and dried *in vacuo* to give the title compound, 20.1 g.
¹H-NMR (CDCl₃, 400MHz) δ: 1.71 (m, 2H), 1.81 (m, 2H), 1.88-2.06 (m, 6H), 2.33 (s, 3H), 2.40 (m, 2H), 2.77 (m, 2H), 2.84 (m, 2H), 4.15 (m, 1H), 4.26 (m, 1H), 5.47 (m, 1H), 6.28 (m, 1H), 6.67 (m, 1H), 6.80 (s, 1H), 7.31 (d, 1H), 8.07 (d, 1H), 8.10 (d, 1H), 8.29 (dd, 1H), 12.30 (brs, 1H).
LRMS : m/z (ES⁺) 510 [MNa⁺]
Microanalysis found: C, 61.39; H, 6.18; N, 8.89. C₂₅H₃₀FN₃O₄S requires C, 61.58; H, 6.20; N, 8.62%.

### Examples 64 to 68

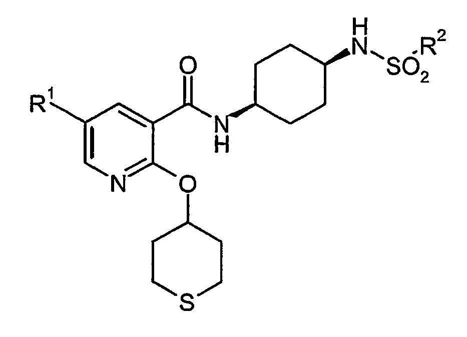

A mixture of the appropriate amine hydrochloride from preparations 15a and 18 (1eq), the appropriate sulphonyl chlorides (1.3eq) and triethylamine (3eq) in dichloromethane (25mlmmol⁻¹) was stirred at room temperature for 18 hours. The solution was washed with 10% citric acid solution then evaporated under reduced pressure. The product was crystallised from isopropyl acetate, to afford the title compounds as solids.

| Ex no | R¹ | R² | Yield (%) | Data |
|---|---|---|---|---|
| 64 | F | 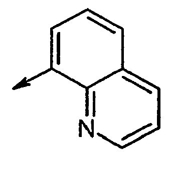 | 86 | ¹HNMR (DMSO-d₆, 400MHz) δ: 1.36 (m, 4H), 1.50 (m, 2H), 1.58 (m, 2H), 1.84 (m, 1H), 1.86 (m, 1H), 2.23 (m, 2H), 2.65 (m, 2H), 2.74 (m, 2H), 3.28 (m, 1H), 3.74 (m, 1H), 5.14 (m, 1H), 7.08 (d, 1H), 7.72 (m, 2H), 7.90 (dd, 1H), 7.98 (d, 1H), 8.25 (d, 1H), 8.31 (dd, 1H), 8.55 (d, 1H), 9.08 (m, 1H). |
| | | | | LRMS : m/z (APCl⁺) 567 [MNa]⁺ |
| | | | | Microanalysis found: C, 56.83; H, 5.37; N, 9.97. C₂₆H₂₉FN₄O₄S₂;0.1H₂O requires C, 57.15; H, 5.39; N, 10.25%. |
| 65^{A} | H | 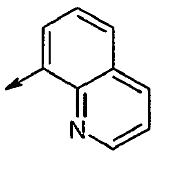 | 54 | ¹HNMR (CDCl₃, 400MHz) δ: 1.48-1.82 (m, 8H), 1.92-2.06 (m, 2H), 2.38-2.54 (m, 2H), 2.76-2.93 (m, 4H), 3.28-3.40 (m, 1H), 3.90-4.03 (m, 1H), 5.35-5.45 (m, 1H), 6.62 (brs, 1H), 7.04 (m, 1H), 7.58-7.66 (m, 1H), 7.70 (m, 1H), 7.95 (d, 1H), 8.08 (d, 1H), 8.21 (d, 1H), 8.36 (d, 1H), 8.48 (m, 2H), 9.12 (m, 1H) |
| | | | | LRMS: m/z ES⁺ 527 [MH]⁺, 549 [MNa]⁺ |
| 66 | F | 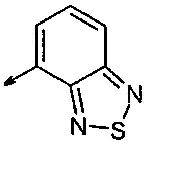 | 80 | ¹HNMR (DMSO-d₆, 400MHz) δ: 1.43 (m, 4H), 1.53 (m, 2H), 1.63 (m, 2H), 1.83 (m, 2H), 2.25 (m, 2H), 2.66 (m, 2H), 2.73 (m, 2H), 3.45 (m, 1H), 3.77 (m, 1H), 5.13 (m, 1H), 7.83-7.95 (m, 3H), 8.10 (d, 1H), 8.21 (dd, 1H), 8.27 (d, 1H), 8.38 (d, 1H). |
| | | | | LRMS : m/z (APCl⁺) 574 [MNa]⁺ |
| | | | | Microanalysis found: C, 49.80; H, 4.79; N, 12.39. C₂₃H₂₆FN₅O₄S₃ requires C, 50.08; H, 4.75; N, 12.69%. |
| 67^{A} | H | 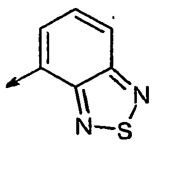 | | ¹HNMR (CDCl₃, 400MHz) δ: 1.50-1.68 (m, 6H), 1.70-1.86 (m, 2H), 1.90-2.08 (m, 2H), 2.39-2.54 (m, 2H), 2.80-2.97 (m, 4H), 3.37 (brs, 1H), 4.23 (brs, 1H), 5.49 (m, 2H), 7.09 (m, 1H), 7.73 (m, 1H), 7.89 (d, 1H), 8.19-8.33 (m, 3H), 8.54 (d, 1H) |
| | | | | LRMS : m/z ES⁺ 534 [MH]⁺ |
| 68 | F | 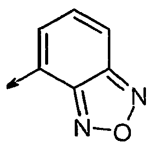 | 88 | ¹HNMR (DMSO-d₆, 400MHz) δ: 1.51 (m, 4H), 1.57 (m, 2H), 1.64 (m, 2H), 1.84 (m, 2H), 2.25 (m, 2H), 2.61-2.77 (m, 4H), 3.41 (m, 1H), 3.80 (m, 1H), 5.14 (m, 1H), 7.75 (dd, 1H), 7.93 (dd, 1H), 8.01 (d, 1H), 8.08 (d, 1H), 8.26-8.35 (m, 3H). |
| | | | | LRMS : m/z APCl⁺ 558 [MNa]⁺ |
| | | | | Microanalysis found: C, 51.00; H, 5.22; N, 12.60. C₂₃H₂₆FN₅O₅S₂;0.3H₂O requires C, 51.06; H, 4.96; N, 12.94%. |

| | | | | |
|---|---|---|---|---|
| A= compound recrystallised from methanol B= compound additionally purified by column chromatography on silica gel using dichloromethane:methanol (99:1). | | | | |

### Example 69

### Syn-5-Fluoro-2-(tetrahydro-thiopyran-4-yloxy)-N-[4-(toluene-4-sulfonylamino)-cyclohexyl]-nicotinamide

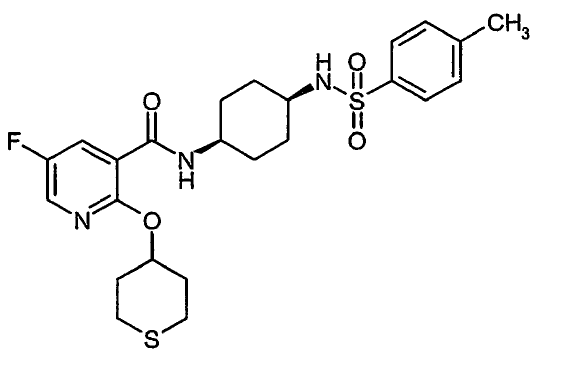

p-Toluenesulphonyl chloride (110mg, 0.58mmol) was added to a solution of the amine from preparation 15a (150mg, 0.39mmol) and N-ethyldiisopropylamine (335µl, 1.93mmol) in dichloromethane (3ml) and the solution stirred at room temperature for 18 hours. The mixture was partitioned between dichloromethane and sodium bicarbonate solution, the layers separated, and the organic phase washed with 2N hydrochloric acid, dried (MgSO₄) and evaporated under reduced pressure. The crude product was purified by column chromatography on silica gel using an elution gradient of ethyl acetate:pentane (5:95 to 100:0) to afford the title compound, 149mg. ¹HNMR (CDCl₃, 400MHz) δ: 1.54-2.00 (m, 10H), 2.36-2.44 (m, 5H), 2.70-2.80 (m, 4H), 3.36 (m, 1H), 4.02 (m, 1H), 4.96 (d, 1H), 5.28 (m, 1H), 7.10 (d, 2H), 7.78 (d, 2H), 7.96 (d, 1H), 8.04 (d, 1H), 8.22 (m, 1H)
LRMS: m/z ES⁺ 530 [MNa]⁺
Microanalysis found; C, 56.42; H, 5.94; N, 8.09, C₂₄H₃₀FN₃O₄S₂; 0.7H₂O requires C, 56.78; H, 5.96; N, 8.28 %.

### Example 70

### Syn-5-Fluoro-2-(tetrahydro-thiopyran-4-yloxy)-N-[4-(toluene-2-sulfonylamino)-cyclohexyl]-nicotinamide

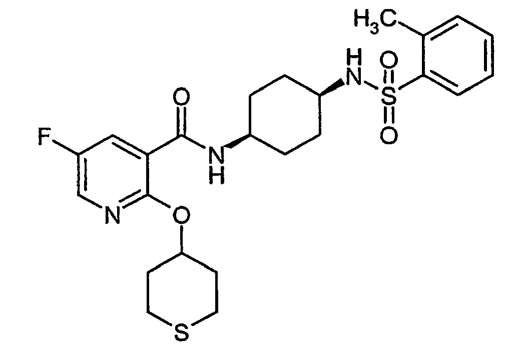

The title compound was obtained in 70% yield from the amine from preparation 15a and o-toluene sulphonyl chloride, following the procedure described in example 69. 1HNMR (CDCl₃, 400MHz) δ: 1.54-2.00 (m, 10H), 2.36-2.44 (m, 2H), 2.48 (s, 3H), 2.74-2.86 (m, 4H), 3.36 (m, 1H), 4.02 (m, 1H), 4.78 (d, 1H), 5.34 (m, 1H), 7.34 (m, 2H), 7.48 (t, 1H), 7.96-8.04 (m, 3H), 8.22 (m, 1H)
LRMS: m/z ES⁺ 530 [MNa]⁺
Microanalysis found; C, 56.43; H, 5.95; N, 8.23, C₂₄H₃₀FN₃O₄S₂; requires C, 56.78; H, 5.96; N, 8.28 %.

### Example 71

### Syn-N-[4-(2-hydroxy-5-methyl-sulfonylamino)-cyclohexyl]-2-(tetrahydro-thiopyran-4-yloxy)-nicotinamide

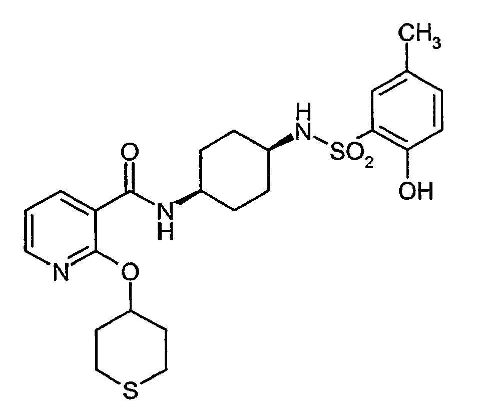

Boron tribromide (1.15ml, 1M in dichloromethane, 1.15mmol) was added to an ice-cooled solution of the ether from preparation 91 (150mg, 0.29mmol) in dichloromethane (10ml), and the reaction stirred at 0°C for 3 hours. The reaction was quenched by the addition of saturated sodium carbonate solution (10ml), then acidified using 2N hydrochloric acid (15ml). The layers were separated, and the organic phase was evaporated under reduced pressure. The crude product was recrystallised from methanol to afford the title compound as white crystals, 53mg. ¹HNMR (CDCl₃, 400MHz) δ: 1.45-1.61 (m, 6H), 1.63-1.75 (m, 2H), 1.84-1.95 (m, 2H), 2.21 (s, 3H), 2.25-2.35 (m, 2H), 2.63-2.81 (m, 4H), 3.08 (brs, 1H), 3.78 (brs, 1H), 5.23 (m, 1H), 6.87 (d, 2H), 7.11 (t, 1H), 7.22 (d, 1H), 7.45 (s, 1H), 7.94 (d, 1H), 8.10 (d, 1H), 8.27 (d, 1H)
LRMS : m/z ES⁺ 528 [MNa]⁺

### Example 72

### Syn-5-Fluoro-N-[4-(7-hydroxy-quinoline-8-sulfonylamino)-cyclohexyl]-2-(tetrahydro-thiopyran-4-yloxy)-nicotinamide

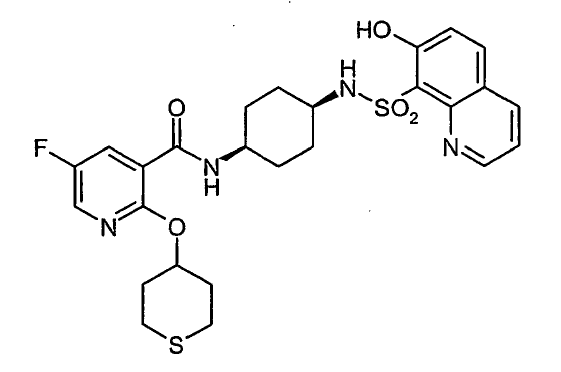

A mixture of the compound from preparation 93 (150mg, 0.26mmol) and lithium iodide (70mg, 0.52mmol) in pyridine (2ml) was heated under reflux for 3 hours. The solution was evaporated under reduced pressure, the residue triturated with methanol, and the resulting solid filtered and dried to give the title compound as an off-white solid, 119mg.
¹HNMR (DMSO-d₆, 400MHz) δ: 1.50-1.64 (m, 8H), 1.92 (m, 2H), 2.24 (m, 2H), 2.67 (m, 2H), 2.82 (m, 2H), 3.15 (m, 1H), 3.78 (m, 1H), 5.17 (m, 1H), 6.70 (d, 1H), 6.94 (m, 1H), 7.48 (d, 1H), 7.89 (m, 2H), 8.08 (d, 1H), 8.25 (d, 1H), 8.38 (m, 1H), 8.51 (m, 1H).
Microanalysis found: C, 54.15; H, 5.21; N, 9.99. C₂₆H₂₉FN₄O₅S₂;0.8H₂O equires C, 54.30; H, 5.36; N, 9.74%.

### Example 73

### Syn-N-[4-(7-hydroxy-guinoline-8-sulfonylamino)-cyclohexyl]-2-(tetrahydro-thiopyran-4-yloxy)-nicotinamide

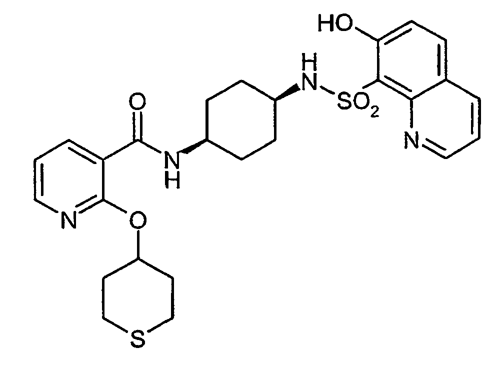

A mixture of the methyl ether from preparation 92 (67mg, 0.12mmol), and lithium iodide (48mg, 0.36mmol) in pyridine (2ml) was heated under reflux for 6 hours. The cooled mixture was concentrated under reduced pressure and the residue partitioned between water and dichloromethane. The mixture was acidified to pH 4 using 10% citric acid and the layers separated. The organic phase was evaporated under reduced pressure and the product crystallised from isopropyl acetate to give the title compound as pale yellow needles.
¹HNMR (DMSO-d₆, 400MHz) δ: 1.35-1.64 (m, 8H), 1.87 (m, 2H), 2.24 (m, 2H), 2.67-2.83 (m, 4H), 3.27 (m, 1H), 3.76 (m, 1H), 5.23 (m, 1H), 7.08 (m, 1H), 7.34 (d, 1H), 7.54 (m, 1H), 7.63 (m, 1H), 7.91 (d, 1H), 8.09 (d, 1H), 8.06 (d, 1H), 8.23 (m, 1H), 8.40 (m, 1H), 8.93 (m, 1H)
LRMS : m/z ES⁺ 565 [MNa]⁺
Microanalysis found; C, 56.76; H, 5.60; N, 10.03. C₂₆H₃₀FN₄O₅S₂; 0.3 H₂O requires C, 56.98; H, 5.63; N, 10.22%.

### Example 74

### Syn-5-Fluoro-N-[4-(5-hydroxy-benzo[1,2,5]thiadiazole-4-sulfonylamino)-cyclohexyl]-2-(tetrahydro-thiopyran-4-yloxy)-nicotinamide

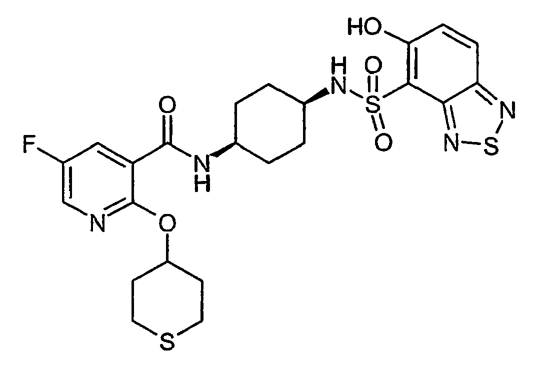

A mixture of the ether from preparation 89 (150mg, 0.26mmol) and lithium iodide (69mg, 0.52mmol) in collidine (2ml) was heated to 130°C for 1 hour. The cooled mixture was partitioned between dichloromethane and 10% citric acid and the layers separated. The organic phase was concentrated under reduced pressure and the residue purified by column chromatography on silica gel using dichloromethane:methanol (98:2) as eluant. The product was crystallised from isopropyl acetate to afford the title compound as a white crystalline solid, 81 mg.
¹HNMR (DMSO-d₆, 400MHz) δ: 1.48-1.62 (m, 6H), 1.65 (m, 2H), 1.86 (m, 2H), 2.25 (m, 2H), 2.68 (m, 2H), 2.75 (m, 2H), 3.37 (m, 1H), 3.77 (m, 1H), 5.15 (m, 1H), 7.49 (d, 1H), 7.89 (m, 1H), 7.92 (dd, 1H), 8.00 (d, 1H), 8.20 (d, 1H), 8.26 (s, 1H), 11.07 (s, 1H).
LRMS : m/z APCl⁺ 590 [MNa]⁺
Microanalysis found: C, 48.68; H, 4.63; N, 12.27. C₂₃H₂₆ FN₅O₅S₃ requires C, 48.66; H, 4.62; N, 12.34%.

### Example 75

### Syn-N-[4-(5-hydroxy-benzo[1,2,5]thiadiazole-4-sulfonylamino)-cyclohexyl]-2-(tetrahydro-thiopyran-4-yloxy)-nicotinamide

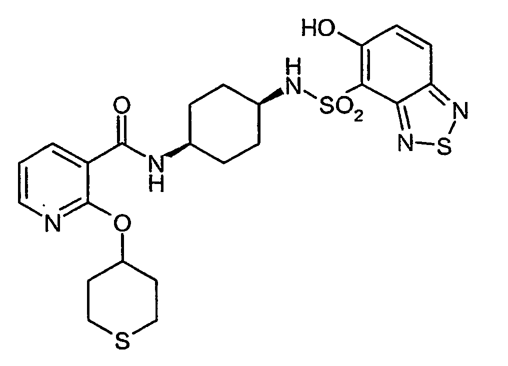

The title compound was obtained as a white crystalline solid from the ether from preparation 90, following the procedure described in example 74.
¹HNMR (DMSO-d₆, 400MHz) δ: 1.48-1.62 (m, 6H), 1.65 (m, 2H), 1.87 (m, 2H), 2.27 (m, 2H), 2.73 (m, 2H), 3.37 (m, 1H), 3.77 (m, 1H), 5.23 (m, 1H), 7.08 (m, 1H), 7.49 (d, 1H), 7.89 (m, 1H), 7.92 (m, 2H), 8.09 (d, 1H), 8.20 (d, 1H), 8.24 (m, 1H), 11.10 (brs, 1H).
Microanalysis found: C, 50.20; H, 4.95; N, 12.48. C₂₃H₂₇N₅O₅S₃ requires C, 50.26; H, 4.95; N, 12.74%.

### Preparation 1

### 2-Chloro-5-fluoro nicotinic acid

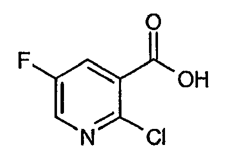

Ethyl-2-chloro-5-fluoro-nicotinoate (50.4 g, 0.247 mol) (see reference *J. Med. Chem.,* 1993, 36(18), 2676-88) was dissolved in tetrahydrofuran (350 ml) and a 2M aqueous solution of lithium hydroxide (247 ml, 0.495 mol) added. The reaction mixture was stirred at room temperature for 3 days. The pH of the solution was reduced to pH1 by addition of 6 N hydrochloric acid and then extracted with dichloromethane (3X). The combined extracts were dried (MgSO₄) and the solvent evaporated under reduced pressure to give a solid which was triturated with diethyl ether and then dried to give the title compound (40.56 g) as a white solid.
¹H NMR (400MHz, DMSO-d₆): δ 8.20 (s, 1H), 8.62 (s, 1H)
LRMS (ES⁺): m/z [MH]⁺ 174.

### Preparation 2

### Trans-N-tert-butyl (4-hydroxy-cyclohexyl)-carbamate

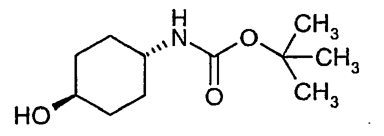

Trans-4-aminocyclohexanol (100g, 0.87mol) was added to acetonitrile (1L), with stirring followed by di-*tert*-butyl dicarbonate (208g, 0.96mol) in portions over 1 hour. The reaction was stirred at room temperature for 18 hours, the resulting precipitate filtered off, and washed with ethyl acetate:hexane (1:3, 250ml), then hexane (250ml) and dried to afford the title compound as a white solid, 166.9g.
m.p.-167-170°C

### Preparation 3

### Trans-Methanesulphonic acid 4-tert-butoxycarbonylamino-cyclohexyl ester

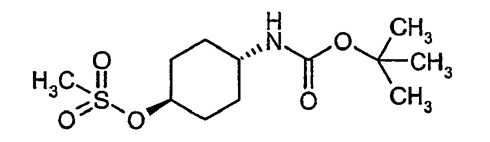

A solution of mesyl chloride (122.4g, 1.07mol) in dichloromethane (400ml) was added dropwise over 45 minutes to an ice-cooled solution of the alcohol from preparation 2 (200g, 0.93mol) and triethylamine (112.8g, 1.115mol) in dichloromethane (1L). The reaction was stirred for 15 minutes, then allowed to warm to room temperature over 1 hour. The mixture was washed with water (3x1.5L), then stirred with silica (100ml, Merck 60H). This mixture was filtered and the filtrate concentrated under reduced pressure to approx quarter volume. Hexane (500ml) was added, the mixture cooled to 0°C, the resulting solid filtered off, dried and recrystallised from ethyl acetate to give the title compound, 221.1 g.
m.p.-146-148°C

### Preparation 4

### syn-(4-Azido-cyclohexyl)-carbamic acid tert-but ester

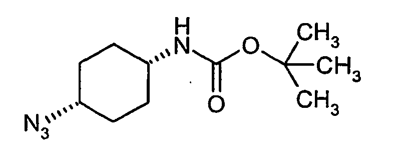

Sodium azide (25.5g, 0.39mol) was added to a solution of the mesylate from preparation 3 (100g, 0.34mol) in N,N-dimethylformamide (500ml) and the reaction slowly warmed to 80°C, and stirred for a further 24 hours at this temperature. Ice/water (1L) was added slowly to the cooled reaction, and the resulting precipitate was filtered off, washed with water and dried. The solid was dissolved in ethyl acetate (200ml), the solution washed with water, dried (MgSO₄) and evaporated under reduced pressure. The residual solid was recrystallised from hexane to afford the title compound as a white solid, 50.8g.
m.p.-79-81°C.

### Preparation 5

### Syn-tert-Butyl 4-aminocyclohexylcarbamate

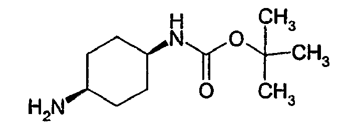

5% Palladium on charcoal (5 g) was mixed with toluene (10 ml) and was added to the azide from preparation 4 (170 g, 0.71 mol) in methanol (400 ml). The mixture was hydrogenated (80 atmospheres) at room temperature for 18 hours and then filtered. The solvent was evaporated *in-vacuo* and the residue was triturated with ethyl acetate (50 ml) and then with hexane (200 ml). The solid obtained was isolated by filtration, dissolved in ethyl acetate (600 ml) and filtered through Celite^{®}. The filtrate was concentrated *in-vacuo* to give a slush that was diluted with hexane (300 ml). The solid obtained was isolated by filtration and was washed with ethyl acetate in hexane (20:80). The mother liquors were combined and evaporated *in-vacuo,* the residue was purified by chromatography on silica gel using ethyl acetate and then methanol as eluant. The material obtained was crystallised from ethyl acetate and hexane and combined with the first crop to give the title compound as a white solid (76 g).
Mp 88-90°C

### Preparation 6

### Syn-{4-[(2-Chloro-5-fluoropyridine-3-carbonyl)amino]-cyclohexyl}-carbamic acid tert-butyl ester

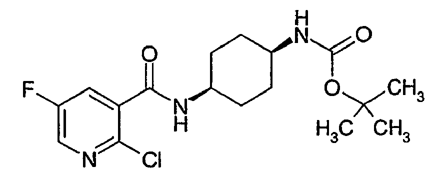

Oxalyl chloride (8ml, 90mmol) was added over 10 minutes to an ice-cooled suspension of the acid from preparation 1 (10g, 57 mmol) and N,N-dimethylformamide (5 drops) in dichloromethane (200ml). The suspension was then stirred at room temperature for 3 hours, and concentrated under reduced pressure. The residue was azeotroped with dichloromethane to give the intermediate acid chloride as a white solid.
This was dissolved in dichloromethane (200ml), the solution cooled in a water bath, then N-diisopropylethylamine (20ml, 115mmol) and the amine from preparation 5 (13.4g, 62mmol) added. The reaction mixture was stirred for 18 hours, diluted with dichloromethane (100ml) and washed sequentially with 10% citric acid solution, saturated sodium bicarbonate solution (x2), water then brine. The organic solution was dried (MgSO₄) and evaporated under reduced pressure to afford the title compound as a yellow foam, 20.2g.
¹H NMR (400MHz, CDCl₃): δ 1.27 (s, 9H), 1.76 (m, 2H), 1.86 (m, 6H), 3.64 (m, 1H), 4.16 (m, 1H), 4.54 (m, 1H), 6.67 (s, 1H), 7.80 (m, 1H), 8.33 (d, 1H).
LRMS: m/z ES⁺ 394 [MNa]⁺

### Preparation 7

### Syn-{4-[(2,5-Dichloro-pyridine-3-carbonyl)-amino]-cyclohexyl}-carbamic acid tert-butyl ester

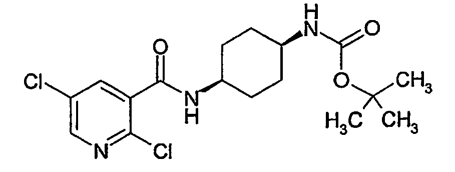

Carbonyl diimidazole (1.7g, 10.5mmol), was added to a solution of 2,5-dichloronicotinic acid (WO 95/30676, pg 19, method 1b) (2g, 10.55mmol) in N,N-dimethylformamide (20ml), and the solution stirred at room temperature for 1 hour. The amine from preparation 5 (2.46g, 11.5mmol) was added and the reaction stirred at room temperature for 3 days. The mixture was concentrated under reduced pressure and the residue partitioned between 10% citric acid solution and ether. The layers were separated, the organic washed with further 10% citric acid solution, water, saturated sodium bicarbonate solution and brine. The solution was dried (MgSO₄) and evaporated under reduced pressure to afford the title compound as a white foam, 3.61 g.
¹HNMR (CDCl₃, 400MHz) δ: 1.43 (s, 9H), 1.44-1.92 (m, 8H), 3.63 (m, 1H), 4.17 (m, 1H), 4.54 (m, 1H), 6.55 (m, 1H), 8.14 (s, 1H), 8.42 (s, 1H)
LRMS : m/z ACPl⁻ 388 [M-H]⁻

### Preparation 8

### 2-Chloro-5-methylnicotinic acid

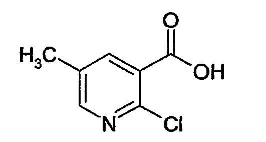

2,2,6,6-Tetramethylpiperidine (4.4ml, 26mmol) was added to a cooled (-78°C) solution of n-butyl lithium (9.4ml, 2.5M in hexane, 23.5mmol) in tetrahydrofuran (50ml), and the solution stirred for 30 minutes. 2-Chloro-5-methylpyridine (3g, 23.5mmol) was then added, and the reaction stirred at -78°C for 2.5 hours. The solution was poured onto solid carbon dioxide, and warmed to room temperature using a water bath. The solution was extracted with water, the aqueous acidified using 2N HCl, and extracted with ether. These organic extracts were washed with water and brine, then dried (MgSO₄) and evaporated under reduced pressure to afford the title compound as a yellow solid, 1.65g.
¹HNMR (CDCl₃, 400MHz) δ: 2.41 (s, 3H), 8.16 (s, 1H), 8.41 (s, 1H)
LRMS: m/z APCl⁺ 172 [MH]⁺

### Preparation 9

### Syn-{4-[(2-Chloro-5-methyl-pyridine-3-carbonyl)-amino]-cyclohexyl}-carbamic acid tert-butyl ester

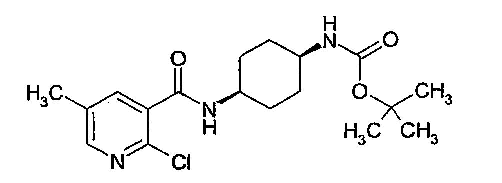

The title compound was obtained as a white foam in 82% yield from the nicotinic acid from preparation 8 and the amine from preparation 5, following the method of preparation 7.
¹HNMR (CDCl₃, 400MHz) δ: 1.45 (s, 9H), 1.68-1.88 (m, 8H), 2.38 (s, 3H), 3.62 (m, 1H), 4.08 (m, 1H), 4.52 (m, 1H), 6.55 (m, 1H), 7.97 (s, 1H), 8.27 (s, 1H)
LRMS: m/z APCl⁺ 312 [MH₂-Bu]⁺

### Preparation 10

### Syn-{4-[(2-Chloro-pyridine-3-carbonyl)-amino]-cyclohexyl}-carbamic acid tert-butyl ester

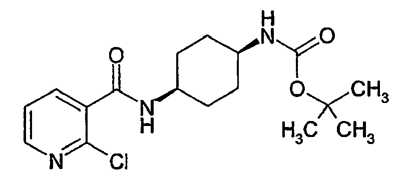

The title compound was obtained in 97% yield from 2-chloronicotinic acid and the amine from preparation 5, following the procedure described in preparation 6.
¹HNMR (CDCl₃, 400MHz) δ: 1.33-1.49 (brs, 9H), 1.52-1.94 (m, 8H), 3.63 (m, 1H), 4.17 (brs, 1H), 4.53 (brs, 1H), 6.57 (brs, 1H), 7.38 (m, 1H), 8.16 (m, 1H), 8.48 (d, 1H)
LRMS : m/z ES⁺ 376 [MNa]⁺

### Preparation 11

### Syn-(4-{[5-fluoro-2-(tetrahydrothiopyran-4-yloxy)-pyridine-3-carbonyl]-amino}-cyclohexyl)-carbamic acid tert-butyl ester

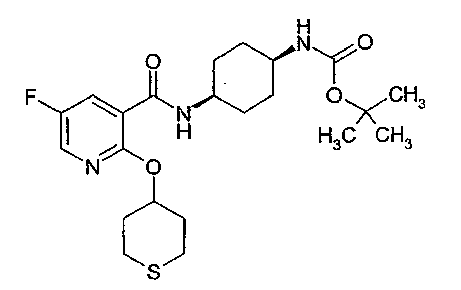

A mixture of the chloride from preparation 6 (3g, 8.1mmol), tetrahydrothiopyran-4-ol (WO 94/14793, pg 77) (2.4g, 20.3mmol) and cesium carbonate (6.5g, 20mmol) in acetonitrile (15ml) was stirred at 100°C for 24 hours. The cooled mixture was partitioned between water and ethyl acetate and the layers separated. The organic phase was washed with 10% citric acid solution, saturated sodium bicarbonate solution, water and brine, then dried (MgSO₄) and evaporated under reduced pressure to afford the title compound, 4.1g.
¹HNMR (CDCl₃, 400MHz) δ: 1.44-1.49 (s, 9H), 1.50-1.77 (m, 4H), 1.79-1.99 (m, 4H), 2.42 (m, 2H), 2.81 (m, 4H), 3.65 (m, 1H), 4.12 (m, 1H), 4.55 (m, 1H), 5.32 (m, 1H), 8.03 (m, 2H), 8.26 (m, 1H),
LRMS : m/z ACPl⁺ 476 [MNa]⁺

### Preparation 12

### Syn-(4-{[5-Chloro-2-(tetrahydrothiopyran-4-yloxy)-pyridine-3-carbonyl]-amino}-cyclohexyl)-carbamic acid tert-butyl ester

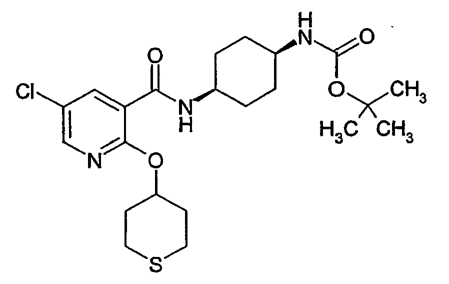

A mixture of the chloride from preparation 7 (1 g, 2.57mmol), tetrahydrothiopyran-4-ol (WO 94/14793, pg 77) (500mg, 4.23mmol) and cesium carbonate (1.4g, 4.23mmol) in acetonitrile (5ml) was stirred at reflux for 20 hours. The cooled mixture was partitioned between water (75ml) and ethyl acetate (75ml) and the layers separated. The organic phase was washed with water, 1 N HCl, saturated sodium bicarbonate solution and brine, then dried (MgSO₄) and evaporated under reduced pressure. The product was purified by column chromatography on silica gel using an elution gradient of ethyl acetate:pentane (5:95 to 70:30) to afford the title compound as a white solid, 1.02g.
¹HNMR (CDCl₃, 400MHz) δ: 1.45 (s, 9H), 1.49-2.00 (m, 10H), 2.41 (m, 2H), 2.79 (m, 4H), 3.67 (m, 1H), 4.13 (m, 1H), 4.60 (m, 1H), 5.34 (m, 1H), 7.91 (m, 1H), 8.14 (d, 1H), 8.47 (d, 1H)
LRMS: m/z APCl⁺ 470 [MH]⁺

### Preparation 13

### Syn-(4-{[5-Methyl-2-(tetrahydrothiopyran-4-yloxy)-pyridine-3-carbonyl]-amino}-cyclohexyl)-carbamic acid tert-butyl ester

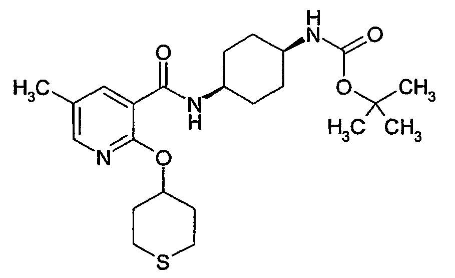

The title compound was obtained in 67% yield from the chloride from preparation 9 and tetrahydrothiopyran-4-ol (WO 94/14793, pg 77), following the procedure described in preparation 12.
¹HNMR (CDCl₃, 400MHz) δ: 1.45 (s, 9H), 1.62-1.75 (m, 4H), 1.80-1.97 (m, 6H), 2.27 (s, 3H), 2.41 (m, 2H), 2.80 (m, 4H), 3.63 (m, 1H), 4.11 (m, 1H), 4.60 (m, 1H), 5.37(m, 1H), 8.01 (s, 2H), 8.35 (m, 1H),
LRMS: m/z APCl⁺ 450 [MH]⁺

### Preparation 14

### Syn-(4-{[2-(Tetrahydrothiopyran-4-yloxy)-pyridine-3-carbonyl]-amino}-cyclohexyl)-carbamic acid tert-bulyl ester

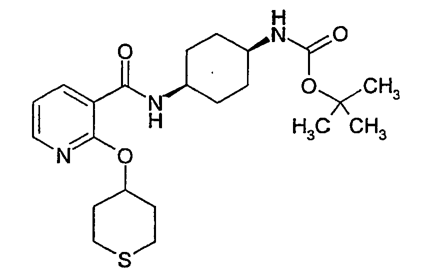

The title compound was obtained in 84% yield from the chloride from preparation 10 and tetrahydrothiopyran-4-ol (WO 94/14793, pg 77), following the procedure described in preparation 12.
¹HNMR (CDCl₃, 400MHz) δ: 1.37-1.50 (s, 9H), 1.52-2.91 (m, 16H), 3.64 (m, 1H), 4.12 (m, 1H), 4.58 (brs, 1H), 5.41 (m, 1H), 7.04 (m, 1H), 7.98 (d, 1H), 8.22 (m, 1H), 8.53 (d, 1H)
LRMS : m/z ES⁺ 436 [MH]⁺, 458 [MNa]⁺

### Preparation 15a

### Syn-N-(4-Amino-cyclohexyl)-5-fluoro-2-(tetrahydrothiopyran-4-yloxy)-nicotinamide hydrochloride

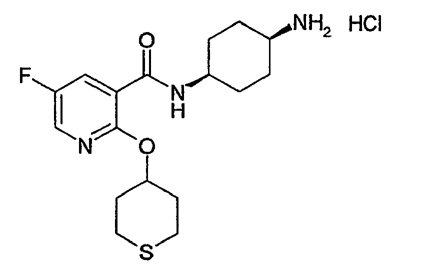

4N Hydrochloric acid in dioxan (50ml) was added to a solution of the protected amine from preparation 11 (4.1g, 9.0mmol) in dichloromethane (10ml), and the reaction stirred at room temperature for 3 hours. The mixture was evaporated under reduced pressure, the residue suspended in ether and the suspension sonicated. The mixture was filtered, the solid dried at 50°C *in vacuo* to give the title compound as an off-white solid, 2.8g.
¹HNMR (CD₃OD, 400MHz) δ: 1.64-2.02 (m, 10H), 2.42 (m, 2H), 2.78 (m, 4H), 3.30 (m, 1H), 4.10 (m, 1H), 5.30 (m, 1H), 8.04 (m, 1H), 8.18 (d, 1H)
LRMS: m/z ES⁺ 354 [MH]⁺

### Preparation 15b

### Syn-N-(4-Amino-cyclohexyl)-5-fluoro-2-(tetrahydrothiopyran-4-yloxy)-nicotinamide

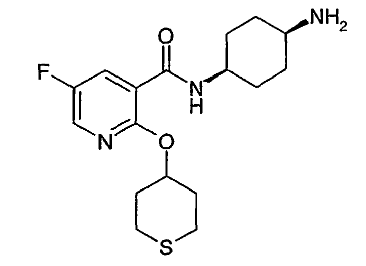

The amine hydrochloride from preparation 15a (95mg, 0.24mmol) was partitioned between dichloromethane and 1N sodium hydroxide solution, and the layers separated. The aqueous phase was extracted further with dichloromethane (2x), and the combined organic solutions dried (MgSO₄) and evaporated under reduced pressure to give the title compound, 75mg.
¹HNMR (CDCl₃, 400MHz) δ: 1.56-2.06 (m, 12H), 2.44 (m, 2H), 2.78 (m, 4H), 2.98 (m, 1H), 4.16 (m, 1H), 5.28 (m, 1H), 8.04 (m, 2H), 8.24 (m, 1H)

### Preparation 16

### Syn-N-4-Amino-cyclohexyl)-5-chloro-2-(tetrahydrothiopyran-4-yloxy)-nicotinamide hydrochloride

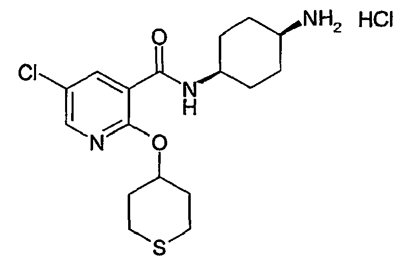

4N Hydrochloric acid in dioxan (15ml) was added to a solution of the compound from preparation 12 (980mg, 2.1mmol) in dichloromethane (5ml), and the reaction stirred at room temperature for 3 hours. Diisopropyl ether was added, the resulting suspension filtered, and the solid washed with further diisopropyl ether and dried *in vacuo* to afford the title compound, 835mg.
¹HNMR (DMSO-d₆, 400MHz) δ: 1.57-1.93 (m, 10H), 2.22-2.30 (m, 2H), 2.61-2.78 (m, 4H), 3.15 (m, 1H), 3.92 (m,1H), 5.17 (m,1H), 7.92-8.12 (m, 5H), 8.32 (s, 1H)
LRMS: m/z APCl⁺ 370 [MH]⁺
Microanalysis found; C, 49.92; H, 6.29; N, 10.09. C₁₇H₂₄ClN₃O₂S; HCl; 0.15 H₂O requires C, 49.91; H, 6.23; N, 10.27%.

### Preparation 17

### Syn-N-(4-Amino-cyclohexyl)-5-methyl-2-(tetrahydrothiopyran-4-yloxy)-nicotinamide hydrochloride

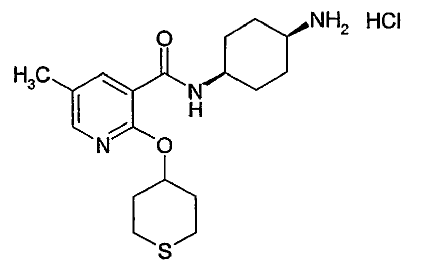

4N Hydrochloric acid in dioxan (15ml) was added to a solution of the protected amine from preparation 13 (800mg, 1.78mmol) in dichloromethane (5ml), and the reaction stirred at room temperature for 3 hours. The mixture was evaporated under reduced pressure, the residue suspended in diisopropyl ether and the suspension sonicated. The mixture was filtered, and the solid dried at 50°C *in vacuo* to give the title compound as an off-white solid, 457mg.
¹HNMR (DMSO-d₆, 400MHz) δ: 1.57-1.92 (m, 10H), 2.22-2.34 (m, 5H), 2.64-2.77 (m, 4H), 3.15 (m, 1H), 3.91 (m, 1H), 5.17 (m,1H), 7.89-8.07 (brs, 4H), 8.11 (s, 1H)
LRMS: m/z APCl⁺ 350 [MH]⁺
Microanalysis found; C, 55.52; H, 7.43; N, 10.38. C₁₈H₂₇FN₃O₂S; HCl; 0.33 H₂O requires C, 55.16; H, 7.37; N, 10.72%.

### Preparation 18

### Syn-N-(4-Amino-cyclohexyl)-2-(tetrahydrothiopyran-4-yloxy)-nicotinamide hydrochloride

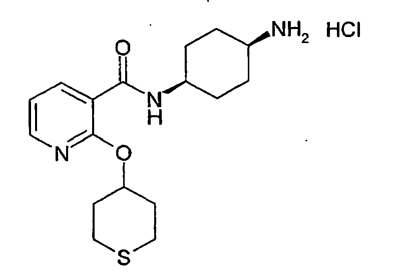

The title compound was obtained in 96% yield from the compound from preparation 14, following the procedure described in preparation 15a.
¹HNMR (DMSO-d₆, 400MHz) δ: 1.54-2.00 (m, 8H), 2.27-2.39 (m, 2H), 2.50 (s, 2H), 2.59-2.80 (m, 4H), 3.14 (brs, 1H), 3.92 (brs, 1H), 5.22 (m, 1H), 7.10 (q, 1H), 8.01 (d, 1H), 8.11 (m, 2H), 8.27 (m, 1H)
LRMS : m/z ES⁺ 336 [MH]⁺

### Preparation 19

### 2-Amino-1-(3-ethoxy-2,3-dioxopropyl)pyridinium bromide

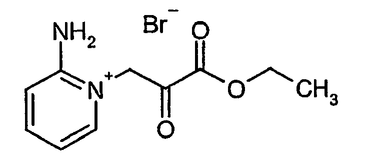

Ethyl bromopyruvate (51.9g, 266mmol) was added dropwise to a solution of 2-aminopyridine (25g, 266mmol) in ethylene glycol dimethyl ether (270ml), and the reaction then stirred at room temperature for 1 hour. The resulting precipitate was filtered off, the solid washed with ether and dried to afford the title compound as a pale yellow solid, 71.9g.
¹HNMR (CDCl₃, 300MHz) δ: 1.35 (t, 3H), 4.35 (q, 2H), 4.70 (d, 1H), 5.15 (d, 1H), 7.10-7.20 (m, 2H), 8.10 (m, 1H), 8.25 (d, 1H).

### Preparation 20

### Ethyl imidazo[1,2-a]pyridine-2-carboxylate hydrobromide

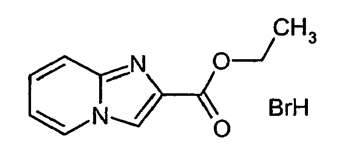

A suspension of the compound from preparation 19 (71.9g, 249mmol) in ethanol (750ml) was heated at reflux for 3 hours, then allowed to cool. The mixture was concentrated under reduced pressure, the residue triturated with ether, filtered and dried to afford the title compound as a solid, 64.17g.
¹HNMR (CD₃OD, 300MHz) δ: 1.45 (t, 3H), 4.50 (q, 2H), 7.55 (m, 1H), 7.95 (m, 1H), 8.10 (dd, 1H), 8.80 (s, 1H), 8.85 (d, 1H).

### Preparation 21

### Imidazo[1,2-a]pyridine-2-carboxylic acid hydrobromide

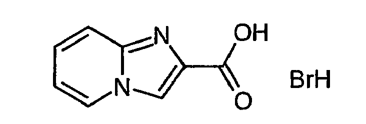

A solution of the ester from preparation 20 (5.0g, 18.4mmol) in 10% aqueous hydrobromic acid (90ml) was heated under reflux for 6 hours. The cooled mixture was concentrated under reduced pressure and the residue triturated with dioxan. The resulting solid was filtered off, washing with hexane and the filtrate evaporated under reduced pressure. The residue was again triturated with dioxan, the solid filtered and dried to afford additional compound, 3.83g in total.
¹HNMR (CD₃OD, 300MHz) δ: 7.57 (m, 1H), 7.96 (d, 1H), 8.06 (m, 1H), 8.78 (s, 1H), 8.84 (d, 1H).
LRMS : m/z ES⁺ 163 [MH]⁺

### Preparation 22

### Methyl imidazo[1,2-a]pyridine-8-carboxylate

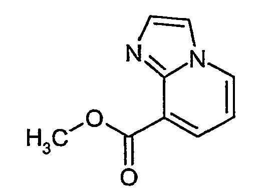

A mixture of methyl 2-aminonicotinate (WO 89/01488 pg 33, prep 17) (1 g, 6.56mmol), and chloroacetaldehyde (1.05ml, 6.56mmol) in ethanol (5ml) was heated under reflux for 18 hours. The cooled mixture was diluted with water (10ml), 0.88 ammonia (1ml) added and the solution concentrated under reduced pressure. The residue was dissolved in methanol and the dark solution treated with charcoal, the mixture filtered and the filtrate evaporated under reduced pressure. The residue was purified by column chromatography on silica gel using dichloromethane:methanol:0.88 ammonia (97:2.5:0.5) as eluant, and the product triturated with ether, to afford the title compound, 768mg.
¹HNMR (CDCl₃, 400MHz) δ : 4.02 (s, 3H), 6.83 (s, 1H), 7.63 (s, 1H), 7.79 (s, 1H), 8.00 (d, 1H), 8.31 (d, 1H).
LRMS : m/z TSP⁺ 177.2 [MH⁺]

### Preparation 23

### lmidazo[1,2-a]pyridine-8-carboxylic acid

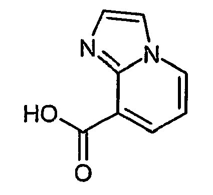

Lithium hydroxide solution (2.5ml, 1M in water) was added to a solution of the ester from preparation 22 (400mg, 2.27mmol) in methanol (5ml) and the solution stirred at room temperature for 90 minutes. The solution was concentrated under reduced pressure to remove the methanol, the aqueous solution acidified using 2N hydrochloric acid, and the mixture evaporated under reduced pressure to give the title compound as a yellow solid.
¹HNMR (DMSO-d₆, 400MHz) δ: 7.60 (dd, 1H), 8.10 (s, 1H), 8.41 (d, 1H), 8.55 (s, 1H), 9.18 (d, 1H)
LRMS : m/z TSP⁺ 163 [MH]⁺

### Preparation 24

### 7-Methoxy-imidazo[1,2-a]pyridine-8-carbonitrile

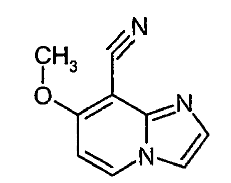

A mixture of 2-amino-4-methoxynicotinonitrile (Archiv. Der. Pharmazie 318(6); 1985; 481) (1g, 6.5mmol), and chloroacetaldehyde (1.25g, 8mmol) in ethanol (10ml) was stirred at room temperature for 1 hour, then heated at reflux for 18 hours. The cooled mixture was basified by the addition of saturated sodium bicarbonate solution, and the resulting solid filtered off, washed with water and dried *in vacuo* to afford the title compound, 1g.
¹HNMR (DMSO-d₆, 400MHz) δ: 4.03 (s, 3H), 7.11 (d, 1H), 7.51 (s, 1H), 7.91 (s, 1H), 8.82 (d, 1H).
LRMS: m/z APCl⁺ 174 [MH]⁺

### Preparation 25

### 7-Methoxy-imidazo[1,2-a]pyridine-8-carboxylic acid

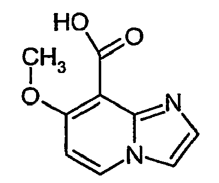

A solution of the nitrile from preparation 24 (600mg, 3.47mmol) in sulphuric acid (3ml) and water (3ml) was stirred at 60°C for 24 hours. The cooled solution was diluted with ether (20ml), then ethanol added until precipitation occurred. The resulting solid was filtered off, washed with ethanol and ether and dried *in vacuo.* The solid was dissolved in 6N hydrochloric acid, the solution stirred at 90°C for 5 hours, and concentrated under reduced pressure to afford the title compound, 110mg.

### Preparation 26

### Ethyl 3-hydroxymethyl-imidazo[1,2-a]pyridine-8-carboxylate

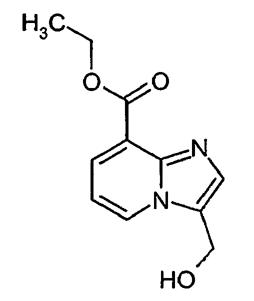

A mixture of 8-carboethoxyimidazo[1,2-a]pyridine (US 5294612, ex 114(b)) (655mg, 3.45mmol), sodium acetate (1.06g, 13mmol), formaldehyde (37% aq solution, 1.8ml, 22mmol) and acetic acid (0.75ml) was stirred at room temperature for 4 hours, then heated at reflux for 6 hours. The cooled mixture was dissolved in water (20ml), potassium carbonate added, to achieve pH 8, and the solution extracted with ethyl acetate (3x25ml). The combined organic solutions were washed with saturated sodium bicarbonate solution and brine, then dried (MgSO₄) and concentrated under reduced pressure. The crude product was purified by column chromatography on silica gel using an elution gradient of dichloromethane:methanol:0.88 ammonia (99.5:0.5:0 to 94:6:0.6) to afford the title compound.
¹HNMR (CDCl₃, 400MHz) δ: 1.45 (t, 3H), 2.47 (brs, 1H), 4.51 (q, 2H), 4.94 (s, 2H), 6.93 (m, 1H), 7.40 (s, 1H), 8.00 (d, 1H), 8.51 (d, 1H).

### Preparation 27

### 3-Hydroxymethyl-imidazo[1,2-a]pyridine-8-carboxylic acid

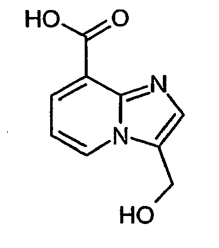

A solution of the ester from preparation 26 (200mg, 0.9mmol), 1 N sodium hydroxide (1ml) and methanol (5ml) was stirred at room temperature for 18 hours. The solution was acidified using 2N hydrochloric acid (2ml) and evaporated under reduced pressure.
¹HNMR (CD₃OD, 400MHz) δ: 5.06 (s, 2H), 7.67 (m, 1H), 8.03 (s, 1H), 8.66 (d, 1H), 9.04 (d, 1H).
LRMS: m/z ES⁺ 193 [MH]⁺

### Preparation 28

### 1H-Benzoimidazole-4-carboxylic acid

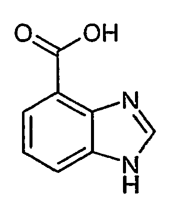

A suspension of 3-nitroanthranillic acid (J. Chem. Soc. 127; 1925; 1791) (4.0g, 22mmol) and palladium on charcoal (400mg) in ethanol was hydrogenated at room temperature using a Parr shaker for 4 hours. The mixture was filtered and the filtrate acidified with conc. hydrochloric acid. Formic acid (2.49ml, 65.9mmol) was added and the solution heated under reflux for 2 hours. The solution was concentrated under reduced pressure to low volume, cooled in ice, and the resulting precipitate filtered. Further precipitation of the filtrate occurred, this solid was filtered and the combined products were recrystallised from 0.5N hydrochloric acid to give the title compound, 2.62g.
LRMS : m/z 162.1 [MH⁺]

### Preparation 29

### Ethyl 2-amino-3-isopropylamino-benzoate

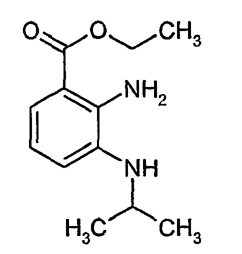

2-lodopropane (2.0ml, 20mmol) was added to a solution of ethyl 2,3-diaminobenzoate (WO 97/10219 ex51(1)) (3g, 16.67mmol) in N,N-dimethylformamide (20ml), and the solution stirred at 50°C for 3 hours. The mixture was concentrated under reduced pressure and the residue partitioned between ethyl acetate (200ml) and water (50ml), and the layers separated. The organic layer was washed with water (5x50ml), dried (MgSO₄) and evaporated under reduced pressure. The crude product was purified by column chromatography on silica gel using ethyl acetate:pentane (5:95 to 90:10) to afford the title compound as a yellow oil, 1.4g.
¹HNMR (CDCl₃, 400MHz) δ: 1.20 (d, 6H), 1.38 (t, 3H), 3.56 (m, 1H), 4.31 (q, 2H), 5.60 (brs, 2H), 6.84 (m, 1H), 6.80 (d, 1H), 7.42 (d, 1H).
LRMS: m/z ES⁺ 223 [MH]⁺

### Preparation 30

### 1-Isopropyl-1H-benzoimidazole-4-carboxylic acid

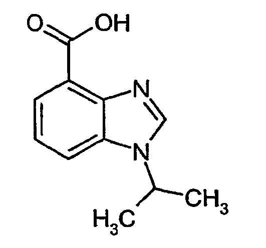

A solution of the amine from preparation 29 (1.4g, 6.31 mmol) in formic acid (15ml) was stirred at 60°C for 45 minutes. 2M Hydrochloric acid (20ml) and additional formic acid (15ml) was added and the reaction heated under reflux for 12 hours. The cooled mixture was evaporated under reduced pressure and the residue triturated initially with ethyl acetate and the solid filtered and dried. This solid was then triturated with hot ethyl acetate and the solid filtered and dried at 60°C to give the title compound as a pale pink solid, 1.16g.
¹HNMR (DMSO-d₆, 400MHz) δ: 1.61 (d, 6H), 5.10 (m, 1H), 7.72 (m, 1H), 8.13 (d, 1H), 8.39 (d, 1H), 9.75 (s, 1H).
LRMS: m/z TSP⁺ 205 [MH]⁺

### Preparation 31

### Ethyl 1-[2-(Tetrahydropyran-2-yloxy)-ethyl]-1H-indazole-3-carboxylate

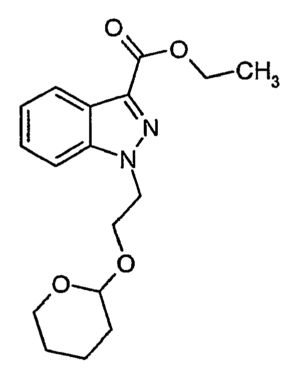

A mixture of indazole-3-carboxylic acid ethyl ester (Chem. Ber. 52; 1919; 1345) (1.9g, 10mmol), 2-(2-bromoethyloxy)tetrahydropyran (2.25g, 10.8mmol), potassium carbonate (1.43g, 10.4mmol) and lithium iodide (67mg, 0.5mmol) in 1-methyl-2-pyrrolidinone (20ml) was heated at 80°C for 17 hours. The mixture was partitioned between water (250ml) and ethyl acetate (250ml), and the layers separated. The organic phase was washed with water (3x200ml), dried (MgSO₄) and evaporated under reduced pressure. The residual oil was purified by column chromatography on silica gel using an elution gradient of pentane:ethyl acetate (91:9 to 50:50) to afford the title compound as a pale yellow oil, 1.88g.
¹HNMR (DMSOd₆, 400MHz) δ : 1.20-1.53 (m, 6H), 1.35 (t, 3H), 3.30 (m, 2H), 3.80 (m, 1H), 4.00 (m, 1H), 4.37 (q, 2H), 4.48 (m, 1H), 4.70 (m, 2H), 7.32 (m, 1H), 7.80 (d, 1H), 8.05 (d, 1H).
LRMS : m/z ES⁺ 341 [MNa⁺]

### Preparation 32

### 1-[2-(Tetrahydropyran-2-yloxy)-ethyl]-1H-indazole-3-carboxylic acid

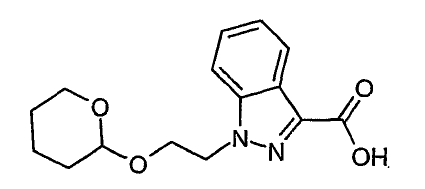

A solution of sodium hydroxide (413mg, 10.3mmol) in water (3.75ml) was added dropwise to a solution of the ester from preparation 31 (1.83g, 5.74mmol) in ethanol (14.7ml), and the reaction stirred at room temperature for 2 days. The mixture was acidifed to pH 3 using 2N hydrochloric acid, and the mixture partitioned between ethyl acetate (75ml) and water (75ml). The layers were separated, and the aqueous further extracted with ethyl acetate (3x60ml). The combined organic solutions were dried (MgSO₄) and evaporated under reduced pressure to give the title compound as a white crystalline solid, 1.44g.
¹HNMR (DMSOd₆, 400MHz) δ : 1.20-1.55 (m, 6H), 3.30 (m, 2H), 3.80 (m, 1H), 4.00 (m, 1H), 4.48 (m, 1H), 4.68 (m, 2H), 7.28 (m, 1H), 7.46 (m, 1H), 7.80 (d, 1H), 8.08 (d, 1H), 12.90 (brs, 1H).
LRMS : m/z ES⁻ 289 [M-H]

### Preparation 33

### Ethyl 5-methyl-1-[2-(tetrahydropyran-2-yloxy)ethyl]-1H-pyrazole-3-carboxylate

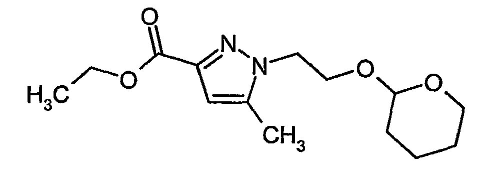

Ethyl 3-methylpyrazole-5-carboxylate (3g, 19.5mmol) was added to a suspension of sodium hydride (934mg, 60% dispersion in mineral oil, 23.35mmol) in tetrahydrofuran (50ml), and the solution stirred at room temperature for 30 minutes. 2-(2-Bromoethoxy)tetrahydro-2-pyran (3.5ml, 23.35mmol) and lithium iodide (50mg, 0.37mmol) were added and the reaction heated under reflux for 16 hours. The cooled mixture was partitioned between water and ethyl acetate and the layers separated. The organic phase was washed sequentially with 10% citric acid, water, saturated sodium bicarbonate solution, water then brine, dried (MgSO₄) and concentrated under reduced pressure. The crude product was purified by column chromatography on silica gel using an elution gradient of methanol:dichloromethane (1:99 to 5:95) to afford the title compound, 4.47g.
¹HNMR (CDCl₃, 400MHz) δ: 1.38 (t, 3H), 1.40-1.76 (m, 6H), 2.36 (s, 1H), 3.41 (m, 1H), 3.59 (m, 1H), 3.76 (m, 1H), 4.06 (m, 1H), 4.32 (t, 2H), 4.37 (q, 2H), 4.47 (m, 1H), 6.53 (s, 1H)
LRMS : m/z ES⁺ 305 [MNa]⁺

### Preparations 34 and 35

### Ethyl 5-isopropyl-2-[2-(tetrahydro-pyran-2-yloxy)ethyl]-2H-pyrazole-3-carboxylate And

### Ethyl 5-Isopropyl-1-[2-(tetrahydro-pyran-2-yloxy)-ethyl]-1H-pyrazole-3-carboxylate

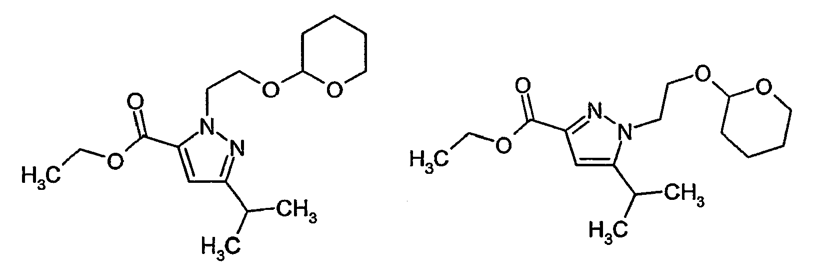

A mixture of ethyl 5-isopropyl-1H-pyrazole-3-carboxylate (Chem. and Pharm. Bull. 1984; 32(4);1568) (509mg, 2.8mmol), 2-(2-bromoethoxy)tetrahydro-2-pyran (732mg, 3.5mmol) and potassium carbonate (483mg, 3.5mmol) in 1-methyl-2-pyrrolidinone (5ml) was stirred at 80°C for 18 hours. The cooled mixture was poured into ethyl acetate and washed with water and brine, then dried (MgSO₄) and evaporated under reduced pressure. The residue was purified by column chromatography on silica gel using an elution gradient of ethyl acetate:pentane (20:80 to 40:60) to afford the title compound of preparation 34 as a clear oil, 663mg.
¹HNMR (CDCl₃, 400MHz) δ: 1.25 (d, 6H), 1.37 (t, 3H), 1.44-1.71 (m, 6H), 2.97 (m, 1H), 3.42 (m, 1H), 3.54 (m, 1H), 3.75 (m, 1H), 4.00 (m, 1H), 4.32 (q, 2H), 4.54 (t, 1H), 4.68 (m, 1H), 4.76 (m, 1H), 6.64 (s, 1H).
LRMS : m/z ACPl⁺ 311 [MH]⁺

Further elution provided the title compound of preparation 35, 242mg.
¹HNMR (CDCl₃, 400MHz) δ: 1.25 (d, 6H), 1.38 (t, 3H), 1.46-1.72 (m, 6H), 3.15 (m, 1H), 3.45 (m, 1H), 3.65 (m, 1H), 3.81 (m, 1H), 4.10 (m, 1H), 4.34 (m, 2H), 4.39 (m, 2H), 4.49 (t, 1H), 6.57 (s, 1H).
LRMS : m/z ACPl⁺ 311 [MH]⁺

### Preparation 36

### Methyl 5-ethyl-2H-pyrazole-3-carboxylate

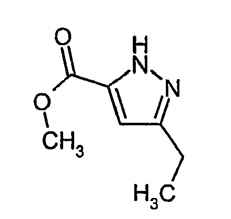

The title compound was prepared by analogy with the method described in Chem. and Pharm. Bull. 1984; 32(4);1568.
¹HNMR (DMSO-d₆, 400MHz) δ: 1.20 (t, 3H), 2.60 (q, 2H), 3.60 (s, 3H), 6.50 (s, 1H).
LRMS : m/z APCl⁺ 155 [MH]⁺

### Preparation 37 and 38

### Methyl 3-ethyl-1-[2-(tetrahydro-2H-pyran-2-yloxy)ethyl]-1H-pyrazole-5-carboxylate And

### Methyl 5-ethyl-1-[2-(tetrahydro-2H-pyran-2-yloxy)ethyl]-1H-pyrazole-3-carboxylate

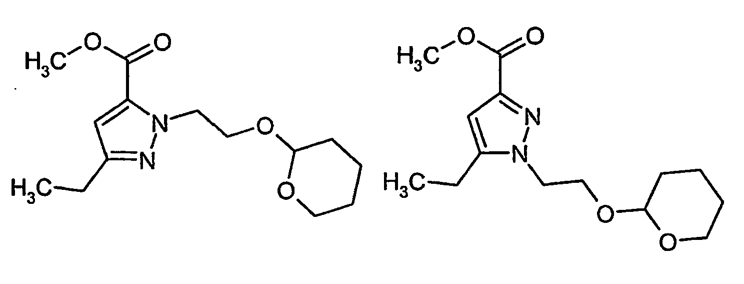

The title compounds were prepared from the ester from preparation 36 and 2-(2-bromoethoxy)tetrahydro-2-pyran by analogy with the method described for preparations 34 and 35.
Preparation 37:¹HNMR (CDCl₃, 400MHz) δ: 1.16 (t, 3H), 1.38-1.75 (m, 6H), 2.48 (m, 2H), 3.35 (m, 1H), 3.54 (m, 1H), 3.70 (m, 1H), 3.80 (s, 3H), 3.95 (m, 1H), 4.50 (m, 1H), 4.68 (m, 2H), 6.60 (s, 1H).
Preparation 38: ¹HNMR (CDCl₃, 400MHz) δ: 1.25 (t, 3H), 1.38-1.68 (m, 6H), 2.70 (t, 2H), 3.38 (m, 1H), 3.54 (m, 1H), 3.72 (m, 1H), 3.85 (s, 3H), 4.04 (m, 1H), 4.25 (m, 2H), 4.43 (m, 1H), 6.54 (s, 1H).

### Preparation 39

### 5-Methyl-1-[2-(tetrahydro-pyran-2-yloxy)ethyl]-1H-pyrazole-3-carboxylic acid

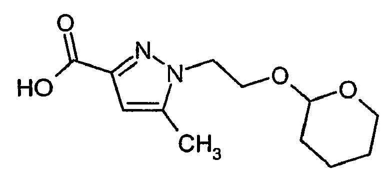

A mixture of the ester from preparation 33 (3g, 10.6mmol) and lithium hydroxide solution (50ml, 1M, 50mmol) in tetrahydrofuran (50ml) was stirred at room temperature for 24 hours. The mixture was diluted with ethyl acetate and the layers separated. The aqueous phase was acidified using 2N hydrochloric acid, and extracted with ethyl acetate. These combined organic extracts were washed with water, brine, then dried (MgSO₄) and evaporated under reduced pressure to afford the title compound, 1.8g.
¹HNMR (CDCl₃, 400MHz) δ: 1.42-1.75 (m, 6H), 2.37 (s, 3H), 3.33 (m, 1H), 3.58 (m, 1H), 3.78 (m, 1H), 4.11 (m, 1H), 4.35 (t, 2H), 4.50 (m, 1H), 6.59 (s, 1H)
LRMS : m/z ACPl⁻ 253 [M-H]⁻

### Preparation 40

### 3-Ethyl-1-[2-(tetrahydro-2H-pyran-2-yloxy)ethyl]-1H-pyrazole-5-carboxylic acid

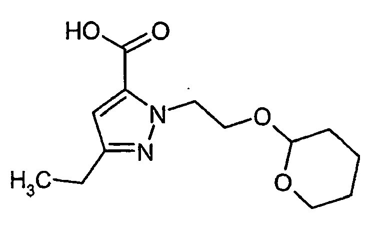

The title compound was prepared as a solid from the ester from preparation 37, following the procedure described in preparation 39.
¹HNMR (CDCl₃, 400MHz) δ: 1.24 (t, 3H), 1.41-1.85 (m, 5H), 2.64 (q, 2H), 3.42 (m, 1H), 3.60 (m, 1H), 3.76 (m, 2H), 4.02 (m, 1H), 4.57 (m, 1H), 4.65-4.81 (m, 2H), 6.73 (s, 1H)
LRMS : m/z ES⁺ 291 [MNa]⁺

### Preparation 41

### 5-Ethyl-1-[2-(tetrahvdro-2H-pyran-2-yloxy)ethyl]-1H-pyrazole-3-carboxylic acid

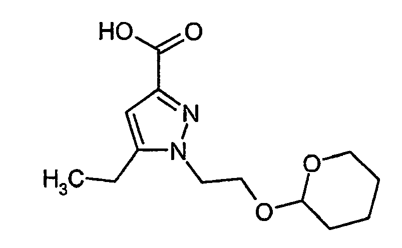

The title compound was prepared as a solid from the ester from preparation 38, following the procedure described in preparation 39.
¹HNMR (CDCl₃, 400MHz) δ: 1.27 (t, 3H), 1.41-1.89 (m, 6H), 2.71 (q, 2H), 3.38-3.64 (m, 2H), 3.7-3.85 (m, 1H), 3.97-4.12 (m, 1H), 4.30 (t, 2H), 4.48 (s, 1H), 4.93 (s, 1H), 6.73 (s, 1H)
LRMS : m/z ES⁺ 291 [MNa]⁺

### Preparation 42

### 5-Isopropyl-2-[2-(tetrahydro-pyran-2-yloxy)ethyl]-2H-pyrazole-3-carboxylic acid

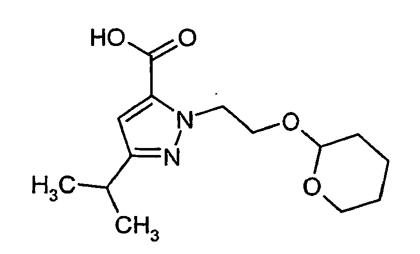

A mixture of the ester from preparation 34 (660mg, 2.13mmol), and 2M sodium hydroxide (2.5ml, 5mmol) in ethanol (10ml) was stirred at room temperature for 18 hours. The reaction mixture was diluted with ethyl acetate, washed with 0.5N citric acid and brine, dried (MgSO₄) and evaporated under reduced pressure to give the title compound as a clear oil, 570mg.
¹HNMR (CDCl₃, 400MHz) δ: 1.26 (d, 6H), 1.43-1.72 (m, 6H), 3.00 (m, 1H), 3.42 (m, 1H), 3.54 (m, 1H), 3.77 (m, 1H), 4.02 (m, 1H), 4.56 (m, 1H), 4.74 (m, 2H), 6.75 (s, 1H).
LRMS : m/z APCl⁺ 283 [MH]⁺

### Preparation 43

### 5-Isopropyl-1-[2-(tetrahydro-pyran-2-yloxy)-ethyl]-1H-pyrazole-3-carboxylic acid

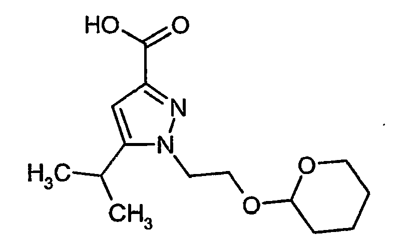

The title compound was obtained quantitatively from the ester from preparation 35 following the procedure described in preparation 42.
¹HNMR (CDCl₃, 400MHz) δ: 1.28 (d, 6H), 1.4B-1.71 (m, 6H), 3.15 (m, 1H), 3.45 (m, 1H), 3.62 (m, 1H), 3.83 (m, 1H), 4.12 (m, 1H), 4.34 (m, 2H), 4.57 (m, 1H), 6.63 (s, 1H).
LRMS : m/z APCl⁻ 281 [M-H]⁻

### Preparation 44

### Methyl 3-ethanesulphonylamino-benzoate

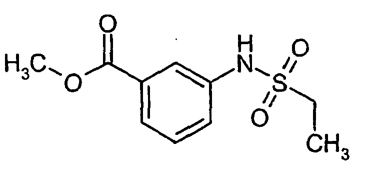

A solution of ethylsulphonyl chloride (1.25ml, 13.2mmol) in dichloromethane (10ml) was added dropwise over 5 minutes to an ice-cooled solution of methyl 3-aminobenzoate (2g, 13.2mmol) and pyridine (1.6ml, 19.8mmol) in dichloromethane (30ml). The reaction was stirred at room temperature for 18 hours, then partitioned between 2N hydrochloric acid and dichloromethane. The layers were separated, the aqueous phase extracted with dichloromethane and the combined organic solutions dried (MgSO₄) and evaporated under reduced pressure. The crude product was purified by column chromatography on silica gel using an elution gradient of dichloromethane:acetonitrile (99:1 to 90:10) to afford the title compound, 2.98g.
¹HNMR (CDCl₃, 400MHz) δ: 1.40 (t, 3H), 3.16 (q, 2H), 3.94 (s, 3H), 7.04 (s, 1H), 7.24 (m, 1H), 7.52 (m, 1H), 7.86 (m, 2H)
LRMS: m/z ES⁺ 266 [MNa]⁺

### Preparation 45

### Methyl 3-isopropylsulphonylamino-benzoate

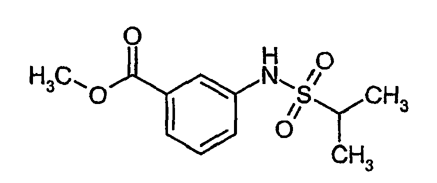

The title compound was obtained in 12% yield from methyl 3-aminobenzoate and isopropyl sulphonyl chloride following the procedure described in preparation 44.
¹HNMR (CDCl₃, 400MHz) δ: 1.40 (d, 6H), 3.32 (m, 1H), 3.94 (s, 3H), 7.20 (m, 1H), 7.40 (m, 1H), 7.56 (m, 1H), 7.80 (m, 1H), 7.88 (s, 1H)
LRMS: m/z ES⁺ 280 [MNa]⁺

### Preparation 46

### Methyl 3-methylsulphonylamino-benzoate

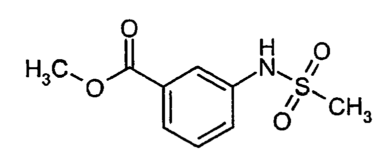

A solution of methane suphonyl chloride (1.03ml, 13.2mmol) in dichloromethane (10ml) was added dropwise to an ice-cooled solution of methyl 3-aminobenzoate (2g, 13.2mmol) and triethylamine (3.68ml, 26.4mmol) in dichloromethane (40ml), and the reaction stirred at room temperature for 18 hours. Additional triethylamine (1.84ml, 13.2mmol) and methane sulphonyl chloride (0.52ml, 6.6mmol) were added and the reaction stirred for a further 2 hours. The mixture was acidified carefully with 1N hydrochloric acid, then extracted with dichloromethane (3x). The combined organic extracts were dried (MgSO₄) and evaporated under reduced pressure. The crude product was purified by column chromatography on silica gel using dichloromethane:acetonitrile (99:1 to 94:6) to give the title compound, 1.5g.
¹HNMR (CDCl₃, 400MHz) δ: 3.04 (s, 3H), 3.94 (s, 3H), 6.84 (brs, 1H), 7.44-7.58 (m, 2H), 7.86 (m, 2H)
LRMS: m/z ES⁺ 252 [MNa]⁺

### Preparation 47

### Methyl 3-methanesuphonylmethylamino-benzoate

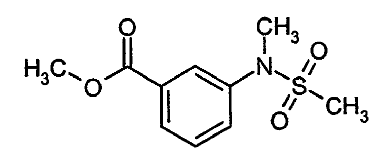

Sodium hydride (340mg, 60% in mineral oil, 8.5mmol) was added to an ice-cooled solution of the sulphonamide from preparation 46 (1.50g, 6.5mmol) in tetrahydrofuran (50ml), and the solution stirred for 90 minutes. Methyl iodide (1.21ml, 19.5mmol) was added, and the reaction stirred at room temperature for 18 hours. The mixture was acidified using 1 N hydrochloric acid, and extracted with ethyl acetate (2x). The combined organic extracts were dried (MgSO₄) and evaporated under reduced pressure. The crude product was purified by column chromatography on silica gel using an elution gradient of pentane:ethyl acetate:diethylamine (80:20:0.6 to 50:50:1) to afford the title compound as a white solid, 1.07g.
¹HNMR (CDCl₃, 400MHz) δ: 2.96 (s, 3H), 3.38 (s, 3H), 3.94 (s, 3H), 7.08 (t, 1H), 7.64 (m, 1H), 7.98 (m, 2H)
LRMS: m/z ES⁺ 266 [MNa]⁺
Microanalysis found; C, 49.48; H, 5.43; N, 5.78, C₁₀H₁₃NO₄S; requires C, 49.37; H, 5.39; N, 5.76 %.

### Preparation 48

### 3-Methanesulphonylmethylamino-benzoic acid

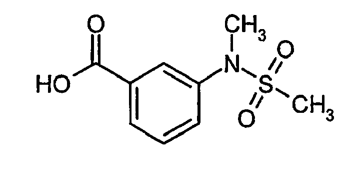

A solution of the ester from preparation 47 (1.05g, 4.3mmol), lithium hydroxide (43ml, 1M, 43mmol) in tetrahydrofuran (43ml) was stirred at room temperature for 18 hours. The mixture was concentrated under reduced pressure to remove the tetrahydrofuran and the aqueous solution acidified using 2N hydrochloric acid. The resulting precipitate was filtered off, washed with water and dried *in vacuo,* to give the title compound, 773mg.
¹HNMR (CD₃OD, 400MHz) δ: 2.90 (s, 3H), 3.45 (s, 3H), 7.50 (m, 1H), 7.70 (d, 1H), 7.90 (d, 1H), 8.10 (s, 1H).
LRMS: m/z ES⁺ 252 [MNa]⁺

### Preparation 49

### 3-Ethanesulphonylamino-benzoic acid

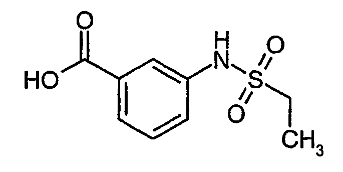

The title compound was obtained in 64% yield from the ester from preparation 44 following the procedure described in preparation 48.
¹HNMR (CD₃OD, 400MHz) δ: 1.30 (s, 3H), 3.10 (q, 2H), 7.45 (m, 2H), 7.70 (d, 1H), 7.90 (d, 1H).
LRMS: m/z ES⁺ 252 [MNa]⁺

### Preparation 50

### 3-Isopropylsulphonylamino-benzoic acid

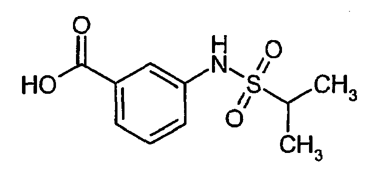

A solution of the ester from preparation 45 (398mg, 1.55mmol), and lithium hydroxide (15ml, 1M, 15mmol) in tetrahydrofuran (15ml) was stirred at room temperature for 18 hours. The mixture was concentrated under reduced pressure to remove the tetrahydrofuran and the aqueous solution acidified using 2N hydrochloric acid. This solution was extracted with ethyl acetate (x3), the combined organic extracts washed with brine, dried (MgSO₄) and evaporated under reduced pressure to give the title compound, 376mg.
¹HNMR (CD₃OD 400MHz) δ: 1.35 (d, 6H), 3.30 (m, 1H), 7.40 (m, 1H), 7.50 (d, 1H), 7.70 (d, 1H), 7.90 (s, 1H).
LRMS: m/z ES⁺ 242 [MH]⁺

### Preparations 51 to 57

Potassium carbonate (2eq) and potassium iodide (0.1 eq) were added to a solution of the appropriate phenol (1 eq) in acetonitrile (1.25mlmmol⁻¹), and the mixture warmed to 90°C. 2-(2-Bromoethoxy)tetrahydro-2H-pyran (1.3 eq) was added and the reaction stirred at 90°C for 72 hours. The cooled reaction was concentrated under reduced pressure and the residue partitioned between ethyl acetate and 10% citric acid solution, and the layers separated. The organic phase was washed with water, sodium bicarbonate solution and brine, then dried (MgSO₄) and evaporated under reduced pressure. The crude product was purified by column chromatography on silica gel using an elution gradient of ethyl acetate:pentane (5:95 to 50:50) to afford the title compounds as clear oils.

| Prep No | | Yield (%) | Data |
|---|---|---|---|
| 51¹ | 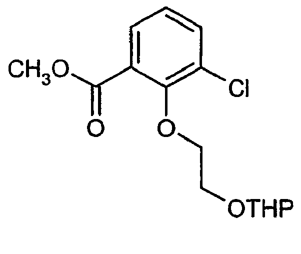 | 79 | ¹HNMR (CDCl₃, 400MHz) δ: 1.50-1.90 (m, 6H), 3.50 (m, 1H), 3.90 (m, 5H), 4.10 (m, 1H), 4.20 (m, 2H), 4.70 (t, 1H), 7.10 (m, 1H), 7.50 (dd, 1H), 7.70 (dd, 1H). |
| | | | LRMS: m/z ES⁺ 337 [MNa]⁺ |
| 52² | 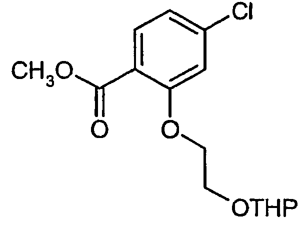 | 72 | ¹HNMR (CDCl₃, 400MHz) δ: 1.44-1.90 (m, 6H), 3.54 (m, 1H), 3.80-3.96 (m, 5H), 4.06 (m, 1H), 4.24 (m, 2H), 4.74 (m, 1H), 6.96 (m, 1H), 7.04 (d, 1H), 7.74 (d, 1H) |
| | | | LRMS: m/z ES⁺ 337 [MNa]⁺ |
| 53³ | 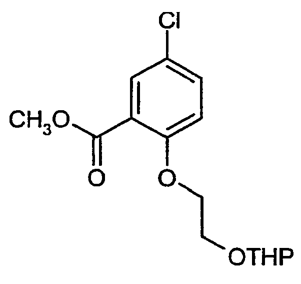 | 84 | ¹HNMR (CDCl₃, 400MHz) δ: 1.46-1.90 (m, 6H), 3.50 (m, 1H), 3.80-3.94 (m, 5H), 4.06 (m, 1H), 4.20 (m, 2H), 4.74 (m, 1H), 6.96 (d, 1H), 7.38 (m, 1H), 7.74 (d, 1H) |
| | | | LRMS: m/z ES⁺ 337 [MNa]⁺ |
| 54⁴ | 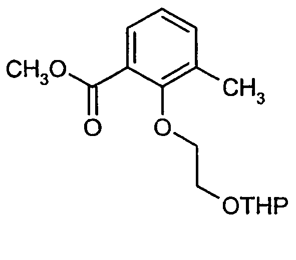 | 72 | ¹HNMR (CDCl₃, 400MHz) δ: 1.44-1.94 (m, 6H), 2.36 (s, 3H), 3.40-3.68 (m, 2H), 3.70-3.96 (m, 5H), 4.10 (m, 2H), 4.74 (m, 1H), 7.04 (m, 1H), 7.36 (m, 1H), 7.62 (m, 1H) |
| | | | LRMS: m/z ES⁺ 317 [MNa]⁺ |
| 55⁵ | 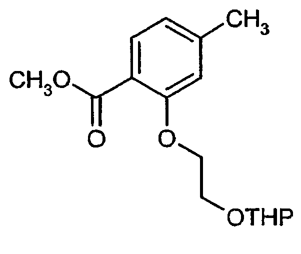 | 46 | ¹HNMR (CDCl₃, 400MHz) δ: 1.48-1.92 (m, 6H), 2.38 (m, 3H), 3.54 (m, 1H), 3.80-3.94 (m, 5H), 4.06 (m, 1H), 4.22 (m, 2H), 4.76 (m, 1H), 6.80 (m, 2H), 7.70 (d, 1H) |
| | | | LRMS: m/z ES⁺ 317 [MNa]⁺ |
| 56⁶ | 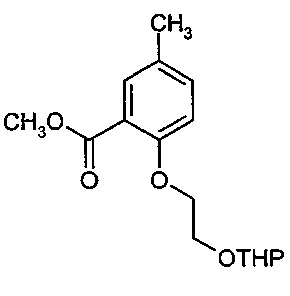 | 57 | ¹HNMR (CDCl₃, 400MHz) δ: 1.44-1.90 (m, 6H), 2.30 (m, 3H), 3.54 (m, 1H), 3.80-3.94 (m, 5H), 4.06 (m, 1H), 4.20 (m, 2H), 4.76 (m, 1H), 6.92 (d, 1H), 7.24 (m, 1H), 7.58 (m, 1H) |
| | | | LRMS: m/z ES⁺ 317 [MNa]⁺ |
| 57⁷ | 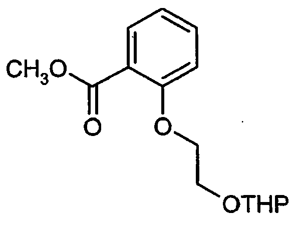 | 80 | ¹HNMR (CDCl₃, 400MHz) δ: 1.50-1.90 (m, 6H), 2.50 (t, 1H), 3.90 (m, 5H), 4.10 (m, 1H), 4.25 (m, 2H), 4.80 (m, 1H), 7.00 (m, 2H), 7.40 (dd, 1H), 7.80 (dd, 1H). |
| | | | LRMS: m/z ES⁺ 303 [MNa]⁺ |

| | | | |
|---|---|---|---|
| 1= Methyl 3-chlorosalicylate (US 4,895,860, pg 14) was the starting alcohol 2 = Methyl 4-chloro-2-hydroxybenzoate (EP 0234872, ex 2f) was the starting alcohol 3 = Methyl 5-chloro-2-hydroxybenzoate (EP 0234872 ex 2c) was used as the starting alcohol 4= Methyl 2-hydroxy-3-methylbenzoate was used as the starting alcohol 5 = Methyl 2-hydroxy-4-methylbenzoate was used as the starting alcohol 6 = Methyl 2-hydroxy-5-methylbenzoate was used as the starting alcohol 7 = Methyl salicylate was used as the starting alcohol | | | |

### Preparations 58 to 64

A mixture of the appropriate ester from preparations 51 to 57 (1 eq) and lithium hydroxide (1M aqueous) (8-12 mlmmol⁻¹) in tetrahydrofuran (5-11 mlmmol⁻¹) was stirred at room temperature for 72 hours. The reaction mixture was concentrated under reduced pressure and the residue acidified using 10% aqueous citric acid solution. The aqueous solution was extracted with ethyl acetate, and the combined organic extracts were washed with brine, dried (MgSO₄) and evaporated under reduced pressure to afford the title compounds as clear oils.

| Prep No | | Data |
|---|---|---|
| 58 | 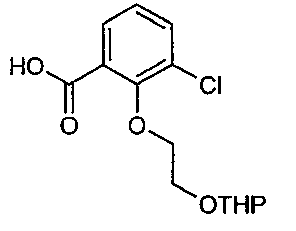 | ¹HNMR (CDCl₃, 400MHz) δ: 1.46-1.94 (m, 6H), 3.56 (m, 1H), 3.76-3.90 (m, 2H), 4.10 (m, 1H), 4.22 (m, 1H), 4.52 (m, 1H), 4.74 (m, 1H), 7.23 (t, 1H), 7.62 (m, 1H), 8.12 (m, 1H) |
| | | LRMS: m/z ES⁺ 323 [MNa]⁺ |
| 59 | 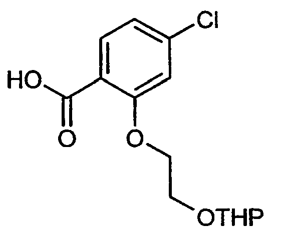 | ¹HNMR (CDCl₃, 400MHz) δ: 1.40-1.90 (m, 6H), 3.56 (m, 1H), 3.86 (m, 2H), 4.16 (m, 1H), 4.30-4.46 (m, 2H), 4.74 (m, 1H), 7.06 (s, 1H), 7.14 (m, 1H), 8.14 (d, 1H) |
| | | LRMS: m/z ES⁺ 323 [MNa]⁺ |
| 60 | 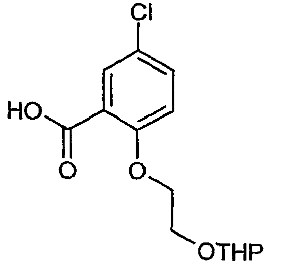 | ¹HNMR (CDCl₃, 400MHz) δ: 1.48-1.90 (m, 6H), 3.54 (m, 1H), 3.82 (m, 2H), 4.16 (m, 1H), 4.28-4.44 (m, 2H), 4.78 (m, 1H), 7.00 (d, 1H), 7.50 (m, 1H), 8.16 (d, 1H) |
| | | LRMS: m/z ES⁺ 323 [MNa]⁺ |
| 61 | 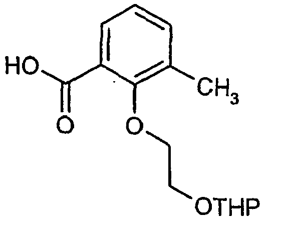 | ¹HNMR (CDCl₃, 400MHz) δ: 1.46-1.96 (m, 6H), 2.40 (s, 3H), 3.54 (m, 1H), 3.70-3.90 (m, 2H), 4.10 (m, 1H), 4.18 (m, 2H), 4.74 (m, 1H), 7.20 (m, 1H), 7.44 (d, 1H), 7.92-8.06 (m, 1H) |
| | | LRMS: m/z ES⁺ 303 [MNa]⁺ |
| 62 | 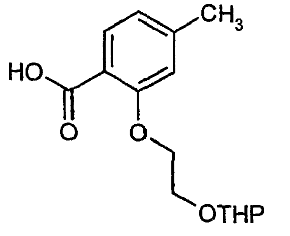 | ¹HNMR (CDCl₃, 400MHz) δ: 1.44-1.94 (m, 6H), 2.40 (s, 3H), 3.54 (m, 1H), 3.78-3.90 (m, 2H), 4.08-4.20 (m, 1H), 4.32-4.46 (m, 2H), 4.74 (m, 1H), 6.88 (s, 1H), 6.96 (m, 1H), 8.06 (d, 1H) |
| | | LRMS: m/z ES⁺ 303 [MNa]⁺ |
| 63 | 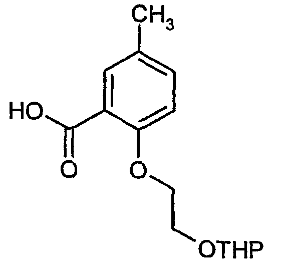 | ¹HNMR (CDCl₃, 400MHz) δ: 1.44-1.94 (m, 6H), 2.34 (s, 3H), 3.56 (m, 1H), 3.78-3.92 (m, 2H), 4.12 (m, 1H), 4.30-4.44 (m, 2H), 4.72 (m, 1H), 6.96 (d, 1H), 7.34 (m, 1H), 8.00 (s, 1H) |
| | | LRMS: m/z ES⁺ 303 [MNa]⁺ |
| 64 | 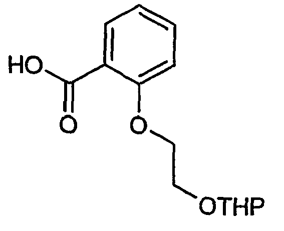 | ¹HNMR (CDCl₃, 400MHz) δ: 1.50-1.92 (m, 6H), 3.56 (m, 1H), 3.84 (m, 2H), 4.08-4.20 (m, 1H), 4.34-4.48 (m, 2H), 4.74 (m, 1H), 7.06 (m, 1H), 7.16 (m, 1H), 7.56 (m, 1H), 8.20 (m, 1H) |
| | | LRMS: m/z ES⁺ 289 [MNa]⁺ |

### Preparation 65

### 2-Hydroxy-4-hydroxymethylbenzoic acid

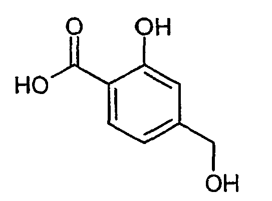

A mixture of 3-hydroxybenzylalcohol (10g, 80mmol) and potassium carbonate (33.35g, 240mmol) were stirred under carbon dioxide in a sealed vessel at 1500-2000psi and 150°C for 18 hours. The cooled residue was dissolved in water, acidified to pH 1 using concentrated hydrochloric acid and extracted with ethyl acetate. The combined organic extracts were washed with brine, dried (MgSO₄) and evaporated under reduced pressure. The product was recrystallised from cyclohexane/isopropyl acetate to afford the title compound, 740mg.
¹HNMR (CD₃OD, 400MHz) δ: 4.60 (s, 2H), 6.84 (m, 1H), 6.90 (s, 1H), 7.80 (d, 1H).

### Preparation 66

### 4-Ethyl-2-hydroxy-benzoic acid

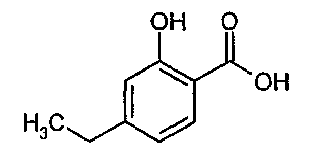

3-Ethyl phenol (10g, 82mmol) and potassium carbonate (34g, 246mmol) were heated in a sealed vessel at 150°C under an atmosphere of carbon dioxide for 18 hours. The cooled mixture was dissolved in water, the solution acidified with concentrated hydrochloric acid, and the resulting precipitate filtered and dried to afford the title compound as a white solid, 11.45g.
LRMS : m/z APCl⁻ 165 [M-H]⁻

### Preparation 67

### 2-Hydroxy-5-isopropyl-benzoic acid

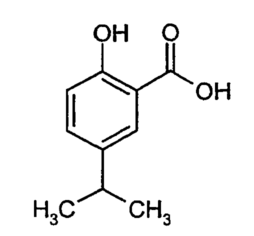

4-Isopropyl phenol (1.0g, 7.3mmol) and potassium carbonate (2.03g, 14.7mmol) were heated to 150°C under an atmosphere of carbon dioxide. The cooled residue was suspended in ethyl acetate and acidified carefully with 2N hydrochloric acid. The layers were separated, the aqueous phase extracted with ethyl acetate and the combined extracts dried (MgSO₄) and evaporated under reduced pressure to give a tan-coloured solid, 1.23g.
¹HNMR (CDCl₃, 400MHz) δ: 1.20 (d, 6H), 2.90 (m, 1H), 6.80 (d, 1H), 6.90 (s, 1H), 7.80 (d, 1H), 10.40 (s, 1H).

### Preparation 68

### 2-Hydroxy-4-isopropyl-benzoic acid

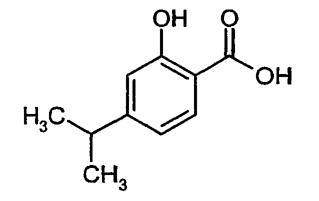

The title compound was obtained as a tan coloured solid from 3-isopropyl phenol, following the procedure described in preparation 67.
¹HNMR (CDCl₃, 400MHz) δ: 1.20 (d, 6H), 2.90 (m, 1H), 7.00 (d, 1H), 7.40 (d, 1H), 7.70 (s, 1H), 10.20 (s, 1H).

### Preparation 69

### Benzyl 4-benzyloxy-2-hydroxybenzoate

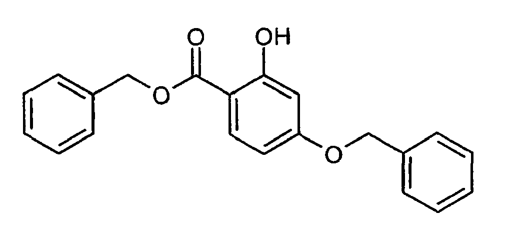

A mixture of benzyl bromide (111g, 0.65mol), potassium carbonate (90g, 0.65mol) and 2,4-dihydroxybenzoic acid (50g, 0.32mol) in N,N-dimethylformamide (250ml) was stirred at room temperature for 18 hours.
The solid was filtered off, washing with N,N-dimethylformamide. Water (125ml) was added to the filtrate, and the mixture extracted with ethyl acetate. The combined organic extracts were washed with 5% sodium hydroxide solution, dried (MgSO₄) and evaporated under reduced pressure. The crude product was recrystallised from 60/80 petroleum ether to give the title compound, 57.1g.
¹HNMR (CDCl₃, 60MHz) δ : 5.05 (s, 2H), 5.30 (s, 2H), 6.50 (m, 2H), 7.35 (m, 11H).

### Preparation 70

### 4-Benzyloxy-2-hydroxybenzoic acid

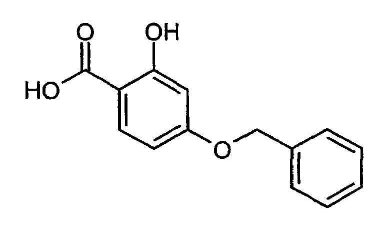

A solution of the compound from preparation 69 (9.0g, 27mmol) in 5% potassium hydroxide in ethanol was stirred under reflux for 6 hours. The mixture was concentrated under reduced pressure and the resulting solid dissolved in water and acidified using hydrochoric acid. The resulting solid was filtrered off and recrystallised from toluene to afford the title compound, 3.1 g.
m.p. 179-180.5°C

### Preparation 71

### 4-Fluoro-2-methoxy-benzonitrile

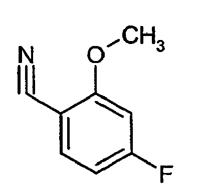

Potassium *tert*-butoxide (216ml, 1M in tetrahydrofuran , 216mmol) was added to ice-cooled methanol (8.7ml, 216mmol), and the solution stirred for 40 minutes. The resulting suspension was added dropwise to a solution of 2,4-difluorobenzonitrile (30g, 216mmol) in tetrahydrofuran at -78°C. Once addition was complete the reaction was allowed to warm to room temperature and stirred for 18 hours. The reaction was diluted with hexane (200ml) and the mixture washed with water (200ml), brine (2x200ml), then dried (MgSO₄) and evaporated under reduced pressure. The residual solid was recrystallised from ethyl acetate:hexane to give the title compound, 9.8g.
¹HNMR (CDCl₃, 300MHz) δ: 3.90 (s, 3H), 6.70 (m, 2H), 7.55 (dd, 1H).
LRMS : m/z ES⁺ 152 [MH⁺]

### Preparation 72

### 4-Benzyloxy-2-methoxy-benzonitrile

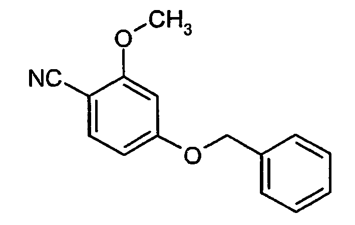

Potassium *tert*-butoxide (97ml, 1M in tetrahydrofuran , 97mmol) was added to an ice-cooled solution of benzyl alcohol (10.1ml, 97mmol) in tetrahydrofuran (50ml). This solution was then added to a solution of the compound from preparation 71 (9.8g, 65mmol) in tetrahydrofuran (50ml) and the reaction stirred at 40°C for 5 hours. The mixture was diluted with ethyl acetate, and washed with water and brine. The solution was dried (MgSO₄) and evaporated under reduced pressure. The residue was recrystallised from ethyl acetate:hexane to give the title compound, 12.73g.
¹HNMR (CDCl₃, 300MHz) δ: 3.88 (s, 3H), 5.10 (s, 2H), 6.60 (m, 2H), 7.35-7.50 (m, 6H).

### Preparation 73

### 4-Benzyloxy-2-methoxy-benzoic acid

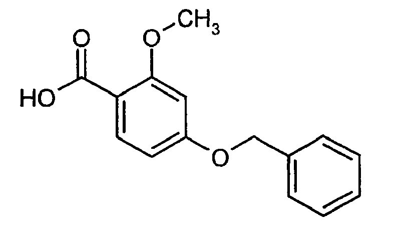

A solution of sodium hydroxide (6.7g, 170mmol) in water (50ml) was added to a suspension of the compound from preparation 72 (10g, 42mmol) in ethanol (100m1) and the reaction heated under reflux for 36 hours. Additional sodium hydroxide (2.0g, 5mmol) was added and the reaction heated for a further 24 hours. The cooled mixture was poured into ice/water (1L), and acidified with concentrated hydrochloric acid. The resulting precipitate was filtered off and dried to give the title compound.
¹HNMR (CDCl₃, 300MHz) δ: 3.98 (s, 3H), 5.10 (s, 2H), 6.80 (m, 2H), 7.40 (m, 5H), 7.52 (m, 1H).

### Preparation 74

### 4-Hydroxy-2-methoxy-benzoic acid

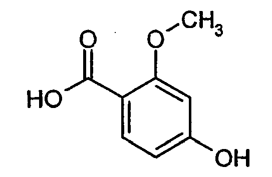

30% Palladium on charcoal (1.5g) was added to a solution of the compound from preparation 73 (11.47g, 44.4mmol) in methanol (300ml), and the mixture hydrogenated at 60psi and room temperature for 24 hours. The mixture was filtered through silica and the filtrate evaporated under reduced pressure. The residue was recrystallised from ethyl acetate:hexane to give the title compound.
¹HNMR (CD₃OD, 300MHz) δ: 3.80 (s, 3H), 6.35 (d, 1H), 6.45 (s, 1H), 7.55 (d, 1H).

### Preparation 75

### 5-Methoxy-benzo[1,2,5]thiadiazole-4-sulphonyl chloride

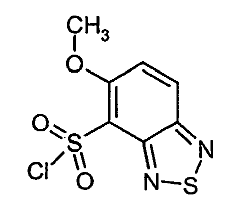

5-Methoxy-2,1,3-benzothiadiazole (500mg, 3.01mmol) was added to ice-cooled chlorosulphonic acid (1.0ml, 15mmol) and the reaction heated to 100°C for 1 hour. The cooled mixture was poured into ice-water (15ml), the resulting precipitate filtered off and dried to afford the title compound as a beige solid, 535mg.
LRMS : m/z APCl⁺ 265, 267 [MH⁺]

### Preparation 76

### syn-[4-(2-Hydroxy-4-methyl-benzoylamino)-cyclohexyl]-carbamic acid tert-butyl ester

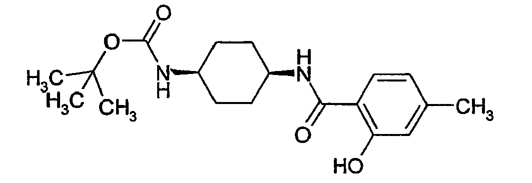

4-Methylsalicylic acid (3.5 g, 23 mmol) was added to a mixture of the amine from preparation 5 (5.35 g, 25 mmol) 1-hydroxybenzotriazole hydrate (3.88 g, 28.8 mmol) 1-(3-dimethylaminopropyl-3-ethylcarbodiimide hydrochloride (6.23 g, 32.5 mmol) and N-diisopropylethylamine (4.84 g, 37.5 mmol) in dichloromethane (65 ml). The mixture was stirred at room temperature for 72 hours and was diluted with dichloromethane (100 ml). Water (150 ml) was added and the aqueous layer was acidified to pH 3 by addition of 2M hydrochloric acid. The phases were separated and the organic phase was washed with water (2 x100 ml) and dried (MgSO₄). The organic solution was concentrated *in-vacuo* and the residue was triturated with hot ethyl acetate to give the title compound, 5.2 g.
LRMS : m/z ES⁺ 371 [MNa⁺]

### Preparation 77

### syn-N-(4-Amino-cyclohexyl)-2-hydroxy-4-methyl-benzamide hydrochloride

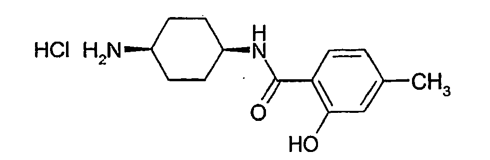

The compound from preparation 76 (5.1 g, 14.6 mmol) was suspended in dichloromethane (400 ml) and was cooled to 0°C. The mixture was purged under nitrogen and hydrogen chloride gas was bubbled into the mixture for 10 minutes to give a saturated solution. The reaction mixture was stirred at 4°C for 3 hours and then concentrated *in-vacuo.* The residue was co-evaporated with dichloromethane (2 x) and triturated with diethyl ether. The material obtained was isolated by filtration and was washed with diethyl ether to give the title compound as a white solid (4.21 g).
LRMS : m/z ES⁺249 [MH⁺]

### Preparation 78

### syn-2-Chloro-5-fluoro-N-[4-(2-hydroxy-4-methyl-benzoylamino)-cyclohexyl]-nicotinamide

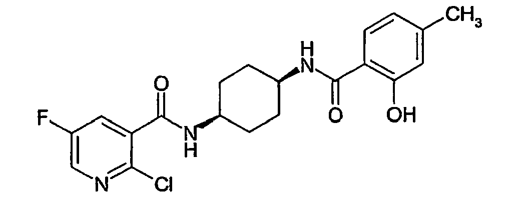

1-(3-Dimethylaminopropyl-3-ethylcarbodiimide hydrochloride (1.68 g, 5.85 mmol) was added to the compound from preparation 77 (2 g, 7.02 mmol) the acid from preparation 1 (1.03 g, 5.85 mmol), 1-hydroxybenzotriazole hydrate (0.95 g, 7.02 mmol) and N-diisopropylethylamine (4.6 ml, 26.3 mmol) in dichloromethane (50 ml) and the mixture was stirred at room temperature under a nitrogen atmosphere for 16 hours. Additional 1-(3-dimethylaminopropyl-3-ethylcarbodiimide hydrochloride (0.56 g, 2.9 mmol) was added and the mixture was stirred for a further 2 hours. The reaction mixture was partitioned between 1 N hydrochloric acid and dichloromethane. The phases were separated and the aqueous layer was extracted with dichloromethane (2 x). The combined organic solutions were dried (MgSO₄) and concentrated *in-vacuo.* The material obtained was recrystallised from isopropyl acetate to give the title compound as a white solid (1.3 g).
LRMS : m/z ES⁺ 406 [MH⁺]

### Preparation 79

### Syn-N-[4-(2-Benzyloxy-5-trifluoromethyl-benzoylamino)-cyclohexyl]-5-fluoro-2-(tetrahydro-thiopyran-4-yloxy)-nicotinamide

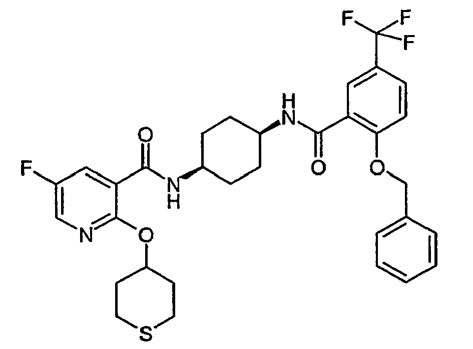

1-(3-Dimethylaminopropyl-3-ethylcarbodiimide hydrochloride (374mg, 1.95mmol) was added to a mixture of the amine from preparation 15b (530mg, 1.5mmol), 2-benzyloxy-5-trifluoromethylbenzoic acid (US 3953595, pg 9), (400mg, 1.35mmol), 1-hydroxybenzotriazole hydrate (264mg, 1.96mmol) and N-ethyldiisopropylamine (0.78ml, 4.5mmol) in N,N-dimethylformamide (30ml) and the reaction stirred at room temperature for 18 hours. The mixture was diluted with ethyl acetate (20ml), and washed with 1 N citric acid (20ml), saturated sodium bicarbonate solution (20ml), then dried (MgSO₄) and evaporated under reduced pressure. The resulting solid was triturated with ether to give the title compound as a white solid, 728mg.
¹HNMR (CD₃OD, 400MHz) δ : 1.48 (m, 2H), 1.60 (m, 2H), 1.75 (m, 4H), 1.89 (m, 2H), 2.30 (m, 2H), 2.69 (m, 4H), 3.97 (m, 2H), 5.30 (m, 3H), 7.18 (m, 1H), 7.31 (m, 2H), 7.45 (d, 1H), 7.53 (d, 2H), 7.81 (d, 1H), 8.09 (m, 1H), 8.20 (m, 2H).
LRMS : m/z ES⁺ 654 [MNa]⁺

### Preparation 80

### Syn-5-Fluoro-N-(4-{2-[2-(tetrahydro-pyran-2-yloxy)-ethoxy]-benzoylamino)-cyclohexyl)-2-(tetrahydro-thiopyran-4-yloxy)-nicotinamide

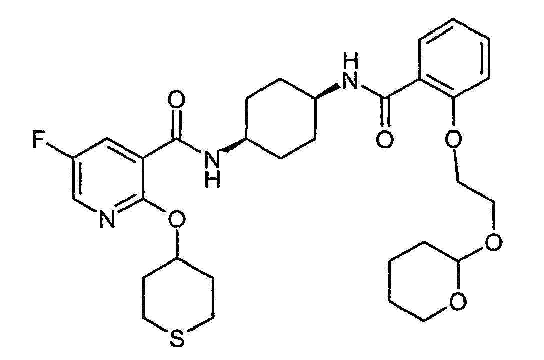

A mixture of the amine from preparation 15a (200mg, 0.51mmol), the acid from preparation 64 (150mg, 0.56mmol), 1-(3-dimethylaminopropyl-3-ethylcarbodiimide hydrochloride (150mg, 0.78mol), 1-hydroxybenzotriazole hydrate (80mg, 0.59mmol) and N-ethyldiisopropylamine (225µl, 1.29mmol) in N,N-dimethylformamide (2ml) was stirred at room temperature for 18 hours. The mixture was partitioned between ethyl acetate and 10% citric acid solution and the layers separated. The organic phase was washed with further 10% citric acid, saturated aqueous sodium bicarbonate solution, brine, then dried (MgSO₄) and evaporated under reduced pressure to give the title compound as a gum, 260mg.
¹HNMR (CD₃OD, 400MHz) δ: 1.27-2.02 (m, 15H), 2.40 (m, 2H), 2.65-2.79 (m, 4H), 3.38 (m, 1H), 3.72 (m, 2H), 3.88 (m, 1H), 4.02-4.16 (m, 4H), 4.37 (t, 3H), 4.58 (m, 1H), 5.31 (m, 1H), 7.07 (t, 1H), 7.16 (d, 1H), 7.47 (t, 1H), 7.95 (d, 1H), 8.06 (m, 1H), 8.17 (m, 1H), 8.43 (m, 1H).
LRMS: m/z APCl⁺ 518 [MH-THP]⁺

### Preparation 81

### Syn-1-[2-(Tetrahydro-pyran-2-yloxy)-ethyl]-1H-indazole-3-carboxylic acid (4-{[5-fluoro-2-(tetrahydro-thiopyran-4-yloxy)-pyridine-3-carbonyl]-amino}-cyclohexyl)-amide

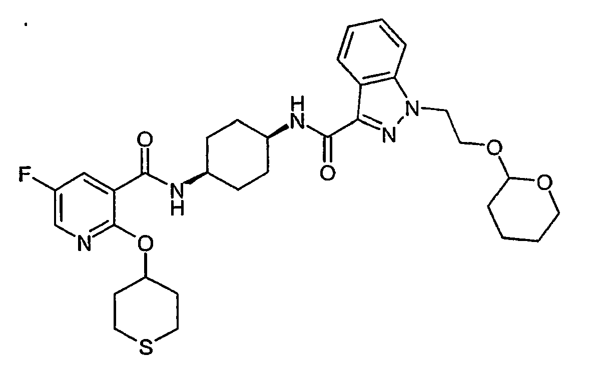

The title compound was obtained as a white solid, from the amine from preparation 15a and the acid from preparation 32, following a similar procedure to that described in preparation 80, except 1-methyl-2-pyrrolidinone was used as the reaction solvent.
¹HNMR (CD₃OD, 400MHz) δ: 1.20-1.53 (m, 6H), 1.78 (m, 8H), 1.94 (m, 2H), 2.27 (m, 2H), 2.65 (m, 2H), 2.80 (m, 2H), 3.22 (m, 1H), 3.30 (m, 1H), 3.80 (m, 1H), 3.97 (m, 3H), 4.47 (m, 1H), 4.65 (m, 2H), 5.20 (m, 1H), 7.23 (m, 1H), 7.43 (m, 1H), 7.65 (d, 1H), 7.78 (d, 1H), 7.97 (m, 1H), 8.10 (m, 2H), 8.29 (s, 1H).
LRMS : m/z ES⁺ 648 [MH⁺]

### Preparation 82

### Syn-5-Fluoro-N-[4-({5-methyl-1-[2-(tetrahydro-pyran-2-yloxy)-ethyl]-1H-pyrazole-3-carbonyl}-amino)-cyclohexyl]-2-(tetrahydro-thiopyran-4-yloxy)-nicotinamide

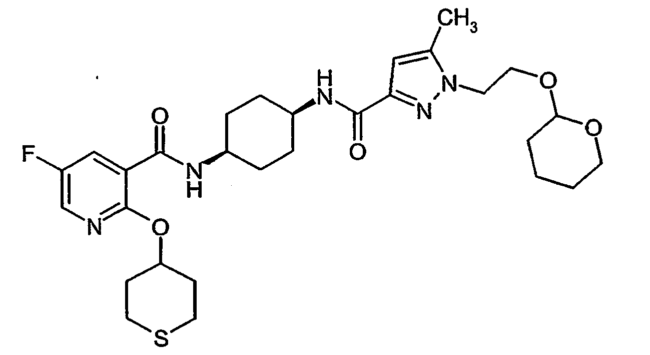

A mixture of the amine from preparation 15a (190mg, 0.48mmol), the acid from preparation 39 (125mg, 0.49mmol), 1-(3-dimethylaminopropyl-3-ethylcarbodiimide hydrochloride (140mg, 0.73mol), 1-hydroxybenzotriazole hydrate (70mg, 0.52mmol) and N-ethyldiisopropylamine (260µl, 1.44mmol) in N,N-dimethylformamide (3ml) was stirred at room temperature for 18 hours. The mixture was partitioned between ethyl acetate (50ml) and 10% citric acid solution (50ml) and the layers separated. The organic phase was washed with further 10% citric acid, saturated aqueous sodium bicarbonate solution, brine, then dried (MgSO₄) and evaporated under reduced pressure to give the title compound as a gum, 260mg.
¹HNMR (CDCl₃, 400MHz) δ: 1.42-2.04 (m, 15H), 2.32-2.48 (m, 6H), 2.81 (m, 4H), 3.41 (m, 1H), 3.55 (m, 1H), 3.77 (m, 1H), 4.00-4.29 (m, 5H), 4.50 (m, 1H), 5.31 (m, 1H), 6.57 (s, 1H), 7.07 (m, 1H), 8.01-8.13 (m, 2H), 8.26 (m, 1H)
LRMS: m/z APCl⁺ 590 [MH]⁺

### Preparation 83

### Syn-N-[4-(4-Benzyloxy-2-ethoxy-benzoylamino)-cyclohexyl]-5-fluoro-2-(tetrahydrothiopyran-4-yloxy)-nicotinamide

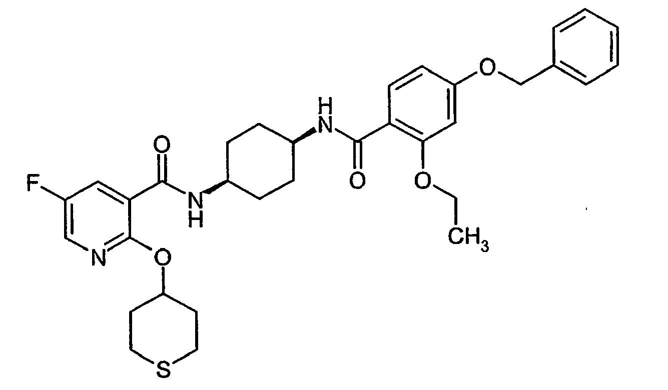

Potassium carbonate (86mg, 0.62mmol) and potassium iodide (5mg, 0.03mmol) were added to a solution of the phenol from example 79 (180mg, 0.31mmol) in acetonitrile (5ml) and N,N-dimethylformamide (1 ml). Ethyl bromide (30µl, 0.4mmol) was added and the mixture stirred at 35°C for 18 hours. The mixture was evaporated under reduced pressure and the residue partitioned between ethyl acetate and 1 N hydrochloric acid, and the layers separated. The organic phase was washed with water, sodium carbonate solution and brine, then dried (MgSO₄) and evaporated under reduced pressure. The product was purified by column chromatography on silica gel using an elution gradient of ethyl acetate:pentane (20:80 to 70:30) to afford the title compound as an oil, 174mg.
¹HNMR (CDCl₃, 400MHz) δ: 1.52 (t, 3H), 1.60-2.08 (m, 10H), 2.42 (m, 2H), 2.74 (m, 4H), 4.08-4.22 (m, 4H), 5.10 (s, 2H), 5.26 (m, 1H), 6.56 (d, 1H), 6.68 (m, 1H), 7.32-7.46 (m, 5H), 8.04 (m, 3H), 8.20 (d, 1H), 8.26 (m, 1H)
LRMS: m/z ES⁺ 608 [MH]⁺

### Preparation 84

### Syn-N-[4-(4-Benzyloxy-2-cyclopropylmethoxy-bensoylamino)-cyclohexyl]-5-fluoro-2-(tetrahydro-thiopyran-4-yloxy)-nicotinamide

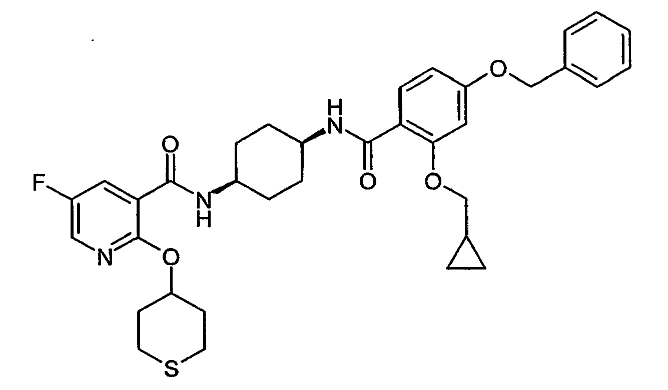

The title compound was obtained in 87% yield from the phenol from example 79 and (bromomethyl)cyclopropane, following the procedure described in preparation 83, except the reaction was performed at 90°C.
¹HNMR (CDCl₃, 400MHz) δ: 0.38 (m, 2H), 0.64 (m, 2H), 1.20-1.38 (m, 1H), 1.64-2.04 (m, 10H), 2.40 (m, 2H), 2.74 (m, 4H), 3.92 (d, 2H), 4.04-4.22 (m, 2H), 5.10 (s, 2H), 5.24 (m, 1H), 6.50 (d, 1H), 6.68 (m, 1H), 7.30-7.46 (m, 5H), 8.02 (m, 2H), 8.16-8.28 (m, 3H)
LRMS: m/z ES⁺ 656 [MNa]⁺

### Preparation 85

### Syn-N-[4-(4-Benzyloxy-2-cyclopentoxy-benzoylamino)-cyclohexyl]-5-fluoro-2-(tetrahydro-thiopyran-4-yloxy)-nicotinamide

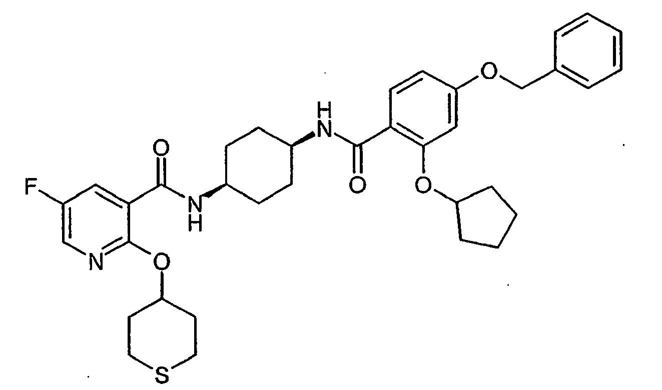

The title compound was obtained in 87% yield from the phenol from example 79 and cyclopentylbromide, following the procedure described in preparation 83, except the reaction was performed at 90°C.
¹HNMR (CDCl₃, 400MHz) δ: 1.38-2.06 (m, 18H), 2.24 (m, 2H), 2.68-2.72 (m, 4H), 4.12 (m, 2H), 4.90 (m, 1H), 5.10 (s, 1H), 5.24 (m, 1H), 6.46 (d, 1H), 6.66 (m, 1H), 7.30-7.48 (m, 5H), 7.94 (d, 1H), 8.04 (m, 2H), 8.16 (d, 1H), 8.28 (m, 1H)
LRMS: m/z ES⁺ 670 [MNa]⁺

### Preparation 86

### Syn-5-Fluoro-N-(4-{5-methyl-2-[2-(tetrahydro-pyran-2-yloxy)-ethoxy]-benzoylamino}-cyclohexyl)-2-(tetrahydro-thiopyran-4-yloxy)-nicotinamide

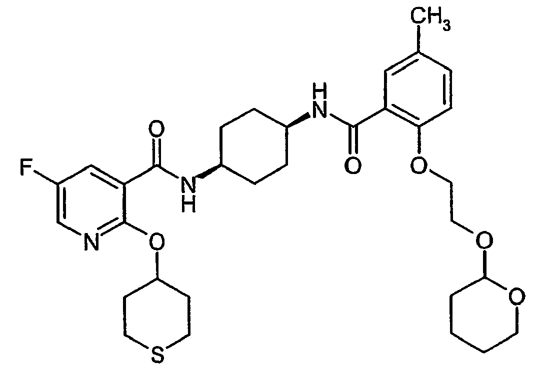

A mixture of the phenol from example 22 (1.29g, 2.65mmol), potassium carbonate (690mg, 5mmol), and 2-(2-bromoethoxy)tetrahydro-2H-pyran (840mg, 4mmol) in 1-methyl-2-pyrrolidinone (10ml), was heated at 60°C for 4 hours, followed by a further 18 hours at room temperature. The mixture was diluted with ethyl acetate and washed with water (x3), then brine, dried (MgSO₄) and evaporated under reduced pressure. The residue was purified by column chromatography on silica gel using ethyl acetate as the eluant to afford the title compound as a white foam, 1.20g.
¹HNMR (CDCl₃, 400MHz) δ: 1.37 (m, 2H), 1.46 (m, 2H), 1.61 (m, 4H), 1.76 (m, 4H), 1.92 (m, 4H), 2.33 (s, 3H), 2.43 (m, 2H), 2.72 (m, 4H), 3.39 (m, 1H), 3.72 (m, 1H), 3.86 (m, 1H), 4.13 (m, 3H), 4.30 (t, 2H), 4.54 (m, 1H), 5.24 (m, 1H), 6.87 (d, 1H), 7.21 (d, 1H), 8.04 (m, 3H), 8.13 (d, 1H), 8.26 (dd, 1H).
LRMS: m/z APCl⁻ 614 [M-H]

### Preparation 87

### 1H-indazole-7-carboxylic acid

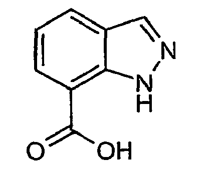

A solution of sodium nitrite (1.9g, 27.6mmol) in water (5ml) was added dropwise to an ice-cooled solution of methyl 2-amino-3-methyl benzoate (US 4,657,893 preparation II) (4.14g, 25mmol) in acetic acid (50ml). This solution was then added dropwise to a solution of *tert*-butyl mercaptan (2.26g, 25mmol) in ethanol (70ml) and stirred at room temperature. The pH of the mixture was adjusted to 5.5 using saturated sodium carbonate solution and the mixture poured into brine. This mixture was extracted with ethyl acetate, the combined organic extracts dried (Na₂SO₄), concentrated under reduced pressure and the residue azeotroped with dichloromethane and heptane. The residue was dissolved in dimethyl sulphoxide (40ml) and added dropwise to a suspension of potassium *tert*-butoxide (14.05g, 126mmol) in dimethyl sulphoxide (150ml), and the reaction stirred at room temperature for 2 hours. The reaction was poured carefully into 1N hydrochloric acid, and extracted with ethyl acetate. The combined organic extracts were washed with 1N hydrochloric acid, dried (Na₂SO₄) and evaporated under reduced pressure. The product was slurried with isopropanol, sufficient dichloromethane added for complete dissolution, and the solution allowed to evaporate. The resulting solid was filtered off, washed with isopropanol to afford the title compound as an off-white solid.
Microanalysis found: C, 59.26; H, 3.73; N, 17.28. C₈H₆N₂O₂ requires C, 59.31; H, 3.51; N, 17.42%.
Mpt. 230-233°C.

### Preparation 88

### Syn-N-[4-(4-Benzyloxy-2-hydroxy-benzoylamino)-cyclohexyl]-5-fluoro-2-(tetrahydrothiopyran-4-yloxy)-nicotinamide

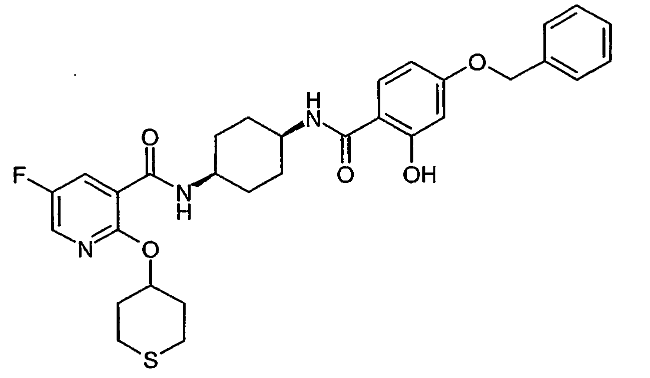

The title compound was obtained as an oil in 32% yield from the amine from preparation 15a and the acid from preparation 70, following a similar procedure to that described in examples 6 to 14, except N,N-dimethylformamide was used as the reaction solvent.
¹HNMR (CDCl₃, 400MHz) δ: 1.60-2.10 (m, 10H), 2.40 (m, 2H), 2.70-2.90 (m, 4H), 4.14 (m, 1H), 4.28 (m, 1H), 5.08 (s, 2H), 5.48 (m, 1H), 6.16 (m, 1H), 6.48 (m, 1H), 6.56 (d, 1H), 7.30-7.46 (m, 6H), 8.04-8.14 (m, 2H), 8.28 (m, 1H)
LRMS: m/z ES⁺ 602 [MNa]⁺
Microanalysis found; C, 64.09; H, 5.96; N, 7.08, C₃₁H₃₄FN₃O₅S; requires C, 64.23; H, 5.91; N, 7.25 %.

### Preparation 89

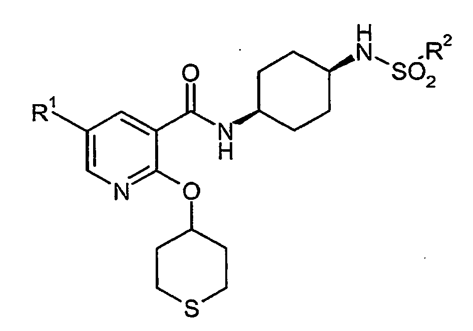

wherin R1 is F and R2 is of formula : 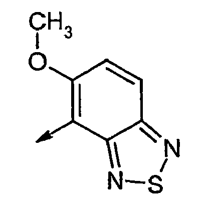 A mixture of the appropriate amine hydrochloride from preparations 15a and 18 (1eq), the appropriate sulphonyl chlorides (1.3eq) and triethylamine (3eq) in dichloromethane (25mlmmol⁻¹) was stirred at room temperature for 18 hours. The solution was washed with 10% citric acid solution then evaporated under reduced pressure. The product was crystallised from isopropyl acetate, to afford the title compounds as solids.
¹HNMR (DMSO-d₆, 400MHz) δ: 1.51 (m, 6H), 1.66 (m, 2H), 1.90 (m, 2H), 2.27 (m, 2H), 2.69 (m, 2H), 2.76 (m, 2H), 3.22 (m, 1H), 3.77 (m, 1H), 4.14 (s, 3H), 5.16 (m, 1H), 7.49 (d, 1H), 7.90 (d, 1H), 7.98 (dd, 1H), 8.03 (d, 1H), 8.28 (d, 1H), 8.35 (d, 1H).
LRMS : m/z (APCl⁺) 604 [MNa]⁺
Microanalysis found: C, 48.89; H, 5.31; N, 11.49. C₂₄H₂₈FN₅O₅S₃;0.4H₂O requires C, 48.95; H, 4.93; N, 11.89%.

### Preparation 90

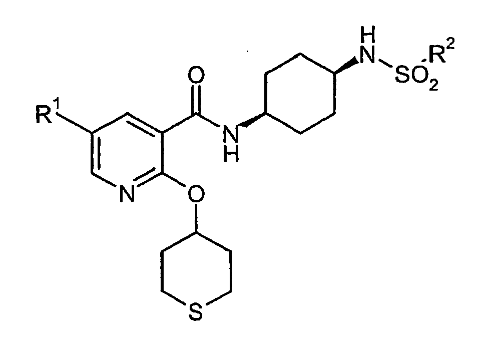

wherein R1 is H and R2 is of formula : 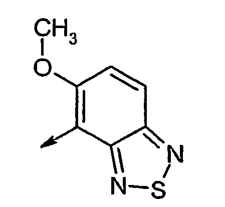 A mixture of the appropriate amine hydrochloride from preparations 15a and 18 (1eq), the appropriate sulphonyl chlorides (1.3eq) and triethylamine (3eq) in dichloromethane (25mlmmol⁻¹) was stirred at room temperature for 18 hours. The solution was washed with 10% citric acid solution then evaporated under reduced pressure. The product was crystallised from isopropyl acetate, to afford the title compounds as solids.
¹HNMR (DMSO-d₆, 400MHz) δ: 1.52 (m, 6H), 1.67 (m, 2H), 1.90 (m, 2H), 2.32 (m, 2H), 2.70-2.82 (m, 4H), 3.23 (m, 1H), 3.76 (m, 1H), 4.13 (s, 3H), 5.25 (m, 1H), 7.10 (dd, 1H), 7.49 (d, 1H), 7.90 (d, 1H), 7.93 (d, 1H), 8.11 (d, 1H), 8.25 (m, 1H), 8.35 (d,
1H).
LRMS : m/z (APCl⁺) 586 [MNa]⁺
Microanalysis found: C, 50.60; H, 5.11; N, 12.23. C₂₄H₂₉N₅O₅S₃;0.1H₂O requires C,
50.97; H, 5.20; N, 12.38%.

### Preparation 91

### Syn-N-[4-(2-methoxy-5-methyl-benzenesulfonylamino)-cyclohexyl]-2-(tetrahydro-thiopyran-4-yloxy)-nicotinamide

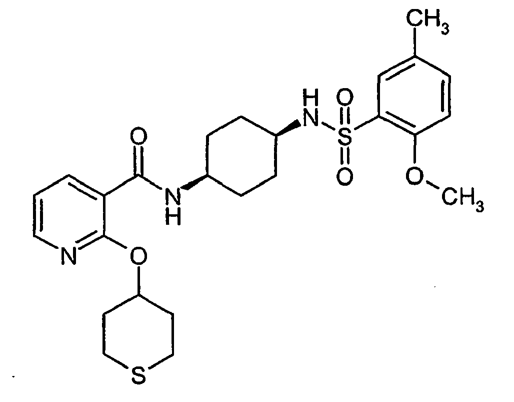

The amine hydrochloride from preparation 18 (500mg, 1.34mmol) was dissolved in dichloromethane, the solution washed with 1 N sodium hydroxide solution, then dried (MgSO₄) and evaporated under reduced pressure.
1-(3-Dimethylaminopropyl)-3-ethylcarbodiimide hydrochloride (385mg, 2.01mmol) was added to a solution of this amine, 1-hydroxybenzotriazole hydrate (181mg, 1.34mmol), 6-methoxy-m-toluenesulphonylchloride (267mg, 1.21 mmol) and N-ethyldiisopropylamine (934µl, 5.36mmol) in N,N-dimethylformamide (5ml), and the reaction stirred at room temperature for 18 hours. The mixture was evaporated under reduced pressure and the residue purified by column chromatography on silica gel using ethyl acetate:pentane (50:50) to afford the title compound as a white solid, 317mg.
¹HNMR (CDCl₃, 400MHz) δ: 1.53-1.72 (m, 6H), 1.74-1.87 (m, 2H), 1.90-2.02 (m, 2H), 2.33 (s, 3H), 2.38-2.49 (m, 2H), 2.72-2.86 (m, 4H), 3.23 (brs, 1H), 3.89-4.04 (m, 4H), 5.10 (d, 1H), 5.36 (m, 1H), 6.91 (d, 1H), 7.01 (m, 1H), 7.31 (d, 1H), 7.64 (s, 1H), 7.93 (d, 1H), 8.18 (d, 1H), 8.47 (d, 1H)
LRMS : m/z ES⁺ 542 [MNa]⁺

### Preparation 92

### Syn-N-[4-(7-methoxy-quinoline-8-sulfonylamino)-cyclohexyl]-2-(tetrahydro-thiopyran-4-yloxy)-nicotinamide

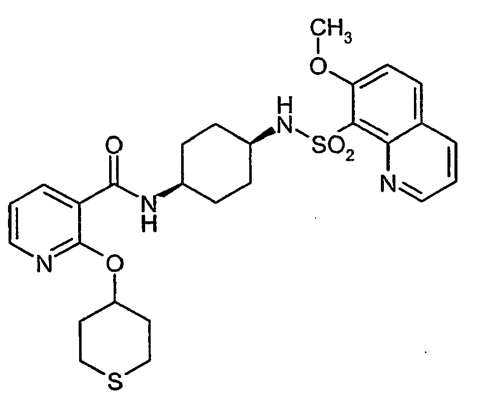

The title compound was obtained as white crystals from the amine from preparation 18 and 7-methoxyquinoline (Syn. Comm. 2000; 30(2); 367) following the procedure as follows :
Chlorosulphonic acid (0.21ml, 3.2mmol) was added dropwise to ice-cooled 7-methoxyquinoline (Syn. Comm. 2000; 30(2); 367) (100mg, 0.63mmol), and the solution then heated to 100°C for 1 hour. The cooled mixture was poured onto ice, sodium bicarbonate slowly added, followed by acetonitrile (30ml) and the amine from preparation 15a (171mg, 0.44mmol). Triethylamine (0.2ml, 1.44mmol) was then added and the solution stirred at room temperature for 18 hours. The solution was evaporated under reduced pressure and the residue partitioned between dichloromethane and water. The organic layer was evaporated under reduced pressure and the residue purified by column chromatography on silica gel using dichloromethane:methanol (98:2).
¹HNMR (DMSO-d₆, 400MHz) δ: 1.40 (m, 4H), 1.52 (m, 4H), 1.88 (m, 2H), 2.24 (m, 2H), 2.67 (m, 2H), 2.76 (m, 2H), 3.25 (m, 1H), 3.74 (m, 1H), 4.02 (s, 3H), 5.24 (m, 1H), 7.08 (dd, 1H), 7.52 (dd, 1H), 7.60 (d, 1H), 7.70 (d, 1H), 7.92 (dd, 1H), 8.03 (d, 1H), 8.25 (m, 2H), 8.45 (d, 1H), 8.99 (dd, 1H).
LRMS : m/z (APCl⁺) 579 [MNa]⁺
Microanalysis found: C, 56.88; H, 5.87; N, 9.80. C₂₇H₃₂N₄O₅S₂;0.6H₂O requires C,
57.14; H, 5.90; N, 9.87%.

### Preparation 93

### Syn-5-Fluoro-N-[4-(7-methoxy-quinoline-8-sulfonylamino)-cyclohexyl]-2-(tetrahydro-thiopyran-4-yloxy)-nicotinamide

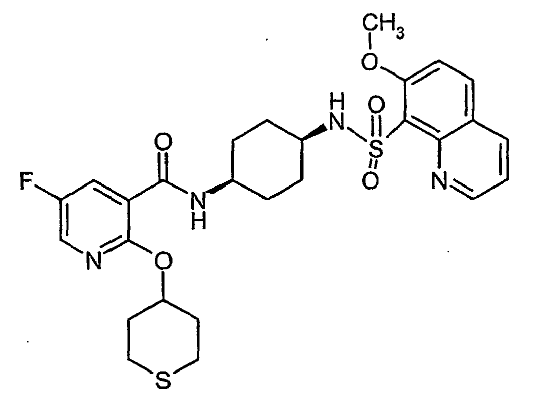

Chlorosulphonic acid (0.21ml, 3.2mmol) was added dropwise to ice-cooled 7-methoxyquinoline (Syn. Comm. 2000; 30(2); 367) (100mg, 0.63mmol), and the solution then heated to 100°C for 1 hour. The cooled mixture was poured onto ice, sodium bicarbonate slowly added, followed by acetonitrile (30ml) and the amine from preparation 15a (171mg, 0.44mmol). Triethylamine (0.2ml, 1.44mmol) was then added and the solution stirred at room temperature for 18 hours. The solution was evaporated under reduced pressure and the residue partitioned between dichloromethane and water. The organic layer was evaporated under reduced pressure and the residue purified by column chromatography on silica gel using dichloromethane:methanol (98:2) to give the title compound as a white solid, 154mg.
¹HNMR (DMSO-d₆, 400MHz) δ: 1.42 (m, 4H), 1.54 (m, 4H), 1.86 (m, 2H), 2.23 (m, 2H), 2.66 (m, 2H), 2.77 (m, 2H), 3.25 (m, 1H), 3.75 (m, 1H), 4.03 (s, 3H), 5.16 (m, 1H), 7.53 (dd, 1H), 7.60 (d, 1H), 7.70 (d, 1H), 7.89 (dd, 1H), 8.01 (d, 1H), 8.26 (m, 2H), 8.45 (d, 1H), 8.98 (dd, 1H).
LRMS : m/z (APCl⁻) 573 [M-H]⁻

### IN VITRO ACTIVITY OF THE NICOTINAMIDE DERIVATIVES (I)

The PDE4 inhibitory activity of the nicotinamide derivatives of the formula (I) is determined by the ability of compounds to inhibit the hydrolysis of cAMP to AMP by PDE4 (Thompson JW, Teraski WL, Epstein PM, Strada SJ., "Assay of nucleotidephosphodiesterase and resolution of multiple molecular forms of the isoenzyme", *Advances in cyclic nucleotides research,* edited by Brooker G, Greengard P, Robinson GA. Raven Press, New York 1979, 10, p. 69-92). Tritium labelled cAMP is incubated with PDE4. Following incubation, the radiolabelled AMP produced is able to bind yttrium silicate SPA beads. These SPA beads subsequently produce light that can be quantified by scintillation counting. The addition of a PDE4 inhibitor prevents the formation of AMP from cAMP and counts are diminished. The IC₅₀ of a PDE4 inhibitor can be defined as the concentration of a compound that leads to a 50% reduction in counts compared to the PDE4 only (no inhibitor) control wells.

The anti-inflammatory properties of the nicotinamide derivatives of the formula (I) are demonstrated by their ability to inhibit TNFα release from human peripheral blood mononuclear cells (see also Yoshimura T, Kurita C, Nagao T, Usami E, Nakao T, Watanabe S, Kobayashi J, Yamazaki F, Tanaka H, Nagai H., "Effects of cAMP-phosphodiesterase isozyme inhibitor on cytokine production by lipopolysaccharide-stimulated human peripheral blood mononuclear cells", *Gen. Pharmacol.,* 1997, 29(4), p. 63). Venous blood is collected from healthy volunteers and the mononuclear cells purified by centrifugation through Histopaque (Ficoll) cushions. TNFα production from these cells is stimulated by addition of lipopolysaccharide. After 18 hours incubation in the presence of LPS, the cell supernatant is removed and the concentration of TNFα in the supernatant determined by ELISA. Addition of PDE4 inhibitors reduces the amount of TNFα produced. An IC₅₀ is determined which is equal to the concentration of compound that gives 50% inhibition of TNFα production as compared to the LPS stimulated control wells.

All the examples were tested in the assay described above and found to have an IC₅₀ (TNFα screen) of less than 300 nM. And for most of the tested compounds, they were found to have an IC₅₀ (TNFα screen) of even less than 100 nM.

Data are presented below for the Examples in which the TNFα and PDE4 inhibition are presented as IC₅₀ values in nM.

| **Example No.** | **IC₅₀ (TNFα screen) in nM** | **IC₅₀ (PDE4 inhibition) in nM** |
|---|---|---|
| 1 | 4 | |
| 2 | 3 | |
| 3 | 2 | |
| 4 | 5 | |
| 5 | 3 | |
| 6 | 2 | |
| 7 | 4 | |
| 8 | 4 | |
| 9 | 6 | |
| 10 | 0.3 | |
| 11 | 1 | |
| 12 | 0.1 | |
| 13 | 0.6 | |
| 14 | 0.1 | |
| 15 | 0.4 | |
| 16 | 4 | |
| 17 | 7 | |
| 18 | 10 | |
| 19 | 0.8 | |
| 20 | 1 | |
| 21 | 0.6 | |
| 22 | 0.02 | |
| 23 | 0.1 | |
| 24 | 0.4 | |
| 25 | 0.8 | |
| 26 | 14 | |
| 27 | 0.7 | |
| 28 | 12 | |
| 29 | 0.4 | |
| 30 | 2 | |
| 31 | 1 | |
| 32 | 10 | |
| 33 | 0.07 | |
| 34 | 1 | |
| 35 | 0.02 | |
| 36 | 2 | |
| 37 | 0.5 | |
| 38 | 0.4 | |
| 39 | 6 | |
| 40 | 10 | |
| 41 | 0.05 | |
| 42 | 0.02 | 0.3 |
| 43 | 0.002 | |
| 44 | 0.001 | |
| 45 | 0.02 | |
| 46 | 1 | |
| 47 | 0.2 | |
| 48 | 2 | |
| 49 | 0.3 | |
| 50 | 1 | |
| 51 | 0.1 | |
| 52 | 0.03 | |
| 53 | 0.01 | |
| 54 | 0.1 | |
| 55 | 17 | |
| 56 | 20 | |
| 57 | 0.07 | |
| 58 | 1 | |
| 59 | 1 | |
| 60 | 0.06 | |
| 61 | 3 | |
| 62 | 12 | |
| 63 | 0.002 | 0.5 |
| 64 | 0.03 | |
| 65 | 0.02 | 1.8 |
| 66 | 0.1 | |
| 67 | 0.8 | |
| 68 | 3 | |
| 69 | 15 | |
| 70 | 2 | |
| 71 | 1 | |
| 72 | 0.001 | |
| 73 | 0.03 | |
| 74 | 1 | 3.5 |
| 75 | 1 | |

## Claims

1. A compound of formula (I) : 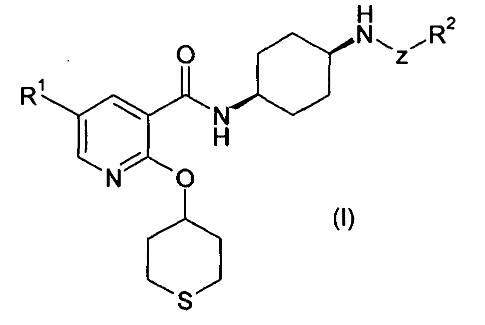 wherein :
R¹ is selected from H, halo and (C₁-C₄)alkyl;
Z is a linker group selected from CO and SO₂;
R² is selected from phenyl, benzyl, naphthyl, heteroaryl and (C₃-C₈)cycloalkyl, each of which is optionally substituted with 1 to 3 substituents each independently selected from halo, CN, CONR³R⁴, (C₁-C₆)alkyl, halo(C₁-C₆)alkyl, OH, hydroxy(C₁-C₆)alkyl, ((C₃-C₈)cycloalkyl)-(C₁-C₆)alkyl, phenyl (optionally substituted by OH and/or halo), (C₃-C₈)cycloalkyl and NR³R⁴; and
R³ and R⁴ are each independently selected from H, (C₁-C₄)alkyl, and SO₂(C₁-C₄)alkyl;
and pharmaceutically acceptable salts and solvates thereof.

2. A compound, salt or solvate according to claim 1 wherein R¹ is H, halo, CH₃ or C₂H₅.

3. A compound, salt or solvate according to claim 1 or claim 2 wherein R² is phenyl, imidazole, pyrazine, indazole, purine, quinoline, quinazoline, benzofuran, dihydrobenzofuran, benzothiadiazole, benzoxadiazole, pyrazole, imidazopyridine, benzimidazole, pyrazolopyridine, pyrazolopyrimidine, benzyl or cyclopropyl,
each of which is optionally substituted with 1 to 3 substituents each independently selected from halo, CN, CONR³R⁴, (C₁-C₆)alkyl, halo(C₁-C₆)alkyl, OH, hydroxy(C₁-C₆)alkyl, ((C₃-C₈)cycloalkyl)-(C₁-C₆)alkyl, phenyl (optionally substituted by OH and/or halo), (C₃-C₈)cycloalkyl and NR³R⁴.

4. A compound, salt or solvate according to claim 1, 2 or 3 wherein R¹ is H, F, Cl or CH₃.

5. A compound, salt or solvate according to any one of claims 1, 2, 3 or 4 wherein R² is phenyl, imidazole, indazole, quinoline, quinazoline, dihydrobenzofuran, benzothiadiazole, benzoxadiazole, pyrazole, imidazopyridine, benzimidazole, pyrazolopyridine, benzyl or cyclopropyl,
each of which is optionally substituted by one or more substituents selected from CH₃, N(CH₃)SO₂CH₃, NHSO₂CH₂CH₃, NHSO₂CH(CH₃)₂, OH, CH₂OH, Cl, F, C₂H₅, CH(CH₃)₂, C₂H₄OH, CF₃.

6. A compound, salt or solvate according to any one of claims 1 to 5 wherein R¹ is F.

7. A compound, salt or solvate according to any one of claims 1 to 6 wherein Z is CO.

8. A compound according to claim 1 which is Syn-5-Fluoro-N-[4-(2-hydroxy-4-methylbenzoylamino)-cyclohexyl]-2-(tetrahydro-thiopyran-4-yloxy)-nicotinamide of formula: 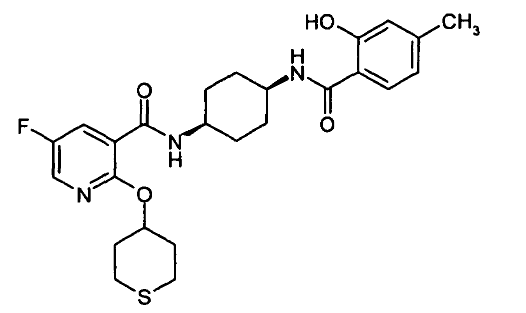 or a pharmaceutically acceptable salt or solvate thereof.

9. A pharmaceutical composition comprising a compound according to any one of claims 1 to 8, or a pharmaceutically acceptable salt or solvate thereof, and a pharmaceutically acceptable carrier, diluent or excipient.

10. A compound salt or solvate according to any one of claims 1 to 8 or pharmaceutical composition according to claim 9, for use in medicine.

11. A compound, salt or solvate according to any one of claims 1 to 8 or a pharmaceutical composition according to claim 9, for use in the treatment of a disease, disorder or condition in which PDE4 inhibition is beneficial.

12. A compound according to claim 11, wherein the disease, disorder or condition is selected from
■ asthma of whatever type, etiology, or pathogenesis, in particular asthma that is a member selected from the group consisting of atopic asthma, non-atopic asthma, allergic asthma, atopic bronchial IgE-mediated asthma, bronchial asthma, essential asthma, true asthma, intrinsic asthma caused by pathophysiologic disturbances, extrinsic asthma caused by environmental factors, essential asthma of unknown or inapparent cause, non-atopic asthma, bronchitic asthma, emphysematous asthma, exercise-induced asthma, allergen induced asthma, cold air induced asthma, occupational asthma, infective asthma caused by bacterial, fungal, protozoal, or viral infection, non-allergic asthma, incipient asthma and wheezy infant syndrome,
■ chronic or acute bronchoconstriction, chronic bronchitis, small airways obstruction, and emphysema,
■ obstructive or inflammatory airways diseases of whatever type, etiology, or pathogenesis, in particular an obstructive or inflammatory airways disease that is a member selected from the group consisting of chronic eosinophilic pneumonia, chronic obstructive pulmonary disease (COPD), COPD that includes chronic bronchitis, pulmonary emphysema or dyspnea associated therewith, COPD that is **characterized by** irreversible, progressive airways obstruction, adult respiratory distress syndrome (ARDS) and exacerbation of airways hyper-reactivity consequent to other drug therapy
■ pneumoconiosis of whatever type, etiology, or pathogenesis, in particular pneumoconiosis that is a member selected from the group consisting of aluminosis or bauxite workers' disease, anthracosis or miners' asthma, asbestosis or steam-fitters' asthma, chalicosis or flint disease, ptilosis caused by inhaling the dust from ostrich feathers, siderosis caused by the inhalation of iron particles, silicosis or grinders' disease, byssinosis or cotton-dust asthma and talc pneumoconiosis;
■ bronchitis of whatever type, etiology, or pathogenesis, in particular bronchitis that is a member selected from the group consisting of acute bronchitis, acute laryngotracheal bronchitis, arachidic bronchitis, catarrhal bronchitis, croupus bronchitis, dry bronchitis, infectious asthmatic bronchitis, productive bronchitis, staphylococcus or streptococcal bronchitis and vesicular bronchitis,
■ bronchiectasis of whatever type, etiology, or pathogenesis, in particular bronchiectasis that is a member selected from the group consisting of cylindric bronchiectasis, sacculated bronchiectasis, fusiform bronchiectasis, capillary bronchiectasis, cystic bronchiectasis, dry bronchiectasis and follicular bronchiectasis,
■ seasonal allergic rhinitis or perennial allergic rhinitis or sinusitis of whatever type, etiology, or pathogenesis, in particular sinusitis that is a member selected from the group consisting of purulent or nonpurulent sinusitis, acute or chronic sinusitis and ethmoid, frontal, maxillary, or sphenoid sinusitis,
■ rheumatoid arthritis of whatever type, etiology, or pathogenesis, in particular rheumatoid arthritis that is a member selected from the group consisting of acute arthritis, acute gouty arthritis, chronic inflammatory arthritis, degenerative arthritis, infectious arthritis, Lyme arthritis, proliferative arthritis, psoriatic arthritis and vertebral arthritis,
■ gout, and fever and pain associated with inflammation,
■ an eosinophil-related disorder of whatever type, etiology, or pathogenesis, in particular an eosinophil-related disorder that is a member selected from the group consisting of eosinophilia, pulmonary infiltration eosinophilia, Loffler's syndrome, chronic eosinophilic pneumonia, tropical pulmonary eosinophilia, bronchopneumonic aspergillosis, aspergilloma, granulomas containing eosinophils, allergic granulomatous angiitis or Churg-Strauss syndrome, polyarteritis nodosa (PAN) and systemic necrotizing vasculitis,
■ atopic dermatitis, allergic dermatitis, contact dermatitis, or allergic or atopic eczema,
■ urticaria of whatever type, etiology, or pathogenesis, in particular urticaria that is a member selected from the group consisting of immune-mediated urticaria, complement-mediated urticaria, urticariogenic material-induced urticaria, physical agent-induced urticaria, stress-induced urticaria, idiopathic urticaria, acute urticaria, chronic urticaria, angioedema, cholinergic urticaria, cold urticaria in the autosomal dominant form or in the acquired form, contact urticaria, giant urticaria and papular urticaria,
■ conjunctivitis of whatever type, etiology, or pathogenesis, in particular conjunctivitis that is a member selected from the group consisting of actinic conjunctivitis, acute catarrhal conjunctivitis, acute contagious conjunctivitis, allergic conjunctivitis, atopic conjunctivitis, chronic catarrhal conjunctivitis, purulent conjunctivitis and vernal conjunctivitis,
■ uveitis of whatever type, etiology, or pathogenesis, in particular uveitis that is a member selected from the group consisting of inflammation of all or part of the uvea, anterior uveitis, iritis, cyclitis, iridocyclitis, granulomatous uveitis, nongranulomatous uveitis, phacoantigenic uveitis, posterior uveitis, choroiditis; and chorioretinitis,
■ multiple sclerosis of whatever type, etiology, or pathogenesis, in particular multiple sclerosis that is a member selected from the group consisting of primary progressive multiple sclerosis and relapsing remitting multiple sclerosis,
■ autoimmune/inflammatory diseases of whatever type, etiology, or pathogenesis, in particular an autoimmune/inflammatory disease that is a member selected from the group consisting of autoimmune hematological disorders, hemolytic anemia, aplastic anemia, pure red cell anemia, idiopathic thrombocytopenic purpura, systemic lupus erythematosus, polychondritis, scleroderma, Wegner's granulomatosis, dermatomyositis, chronic active hepatitis, myasthenia gravis, Stevens-Johnson syndrome, idiopathic sprue, autoimmune inflammatory bowel diseases, ulcerative colitis, endocrin opthamopathy, Grave's disease, sarcoidosis, alveolitis, chronic hypersensitivity pneumonitis, primary biliary cirrhosis, juvenile diabetes or diabetes mellitus type I, keratoconjunctivitis sicca, epidemic keratoconjunctivitis, diffuse interstitial pulmonary fibrosis or interstitial lung fibrosis, idiopathic pulmonary fibrosis, cystic fibrosis, glomerulonephritis with and without nephrotic syndrome, acute glomerulonephritis, idiopathic nephrotic syndrome, minimal change nephropathy, inflammatory/hyperproliferative skin diseases, benign familial pemphigus, pemphigus erythematosus, pemphigus foliaceus, and pemphigus vulgaris,
■ allogeneic graft rejection following organ transplantation,
■ inflammatory bowel disease (IBD) of whatever type, etiology, or pathogenesis, in particular inflammatory bowel disease that is a member selected from the group consisting of collagenous colitis, colitis polyposa, transmural colitis, ulcerative colitis and Crohn's disease (CD),
■ septic shock of whatever type, etiology, or pathogenesis, in particular septic shock that is a member selected from the group consisting of renal failure, acute renal failure, cachexia, malarial cachexia, hypophysial cachexia, uremic cachexia, cardiac cachexia, cachexia suprarenalis or Addison's disease, cancerous cachexia and cachexia as a consequence of infection by the human immunodeficiency virus (HIV),
■ liver injury,
■ pulmonary hypertension of whatever type, etiology or pathogenesis including primary pulmonary hypertension / essential hypertension, pulmonary hypertension secondary to congestive heart failure, pulmonary hypertension secondary to chronic obstructive pulmonary disease, pulmonary venous hypertension, pulmonary arterial hypertension and hypoxia-induced pulmonary hypertension,
■ bone loss diseases, primary osteoporosis and secondary osteoporosis,
■ central nervous system disorders of whatever type, etiology, or pathogenesis, in particular a central nervous system disorder that is a member selected from the group consisting of depression, Alzheimers disease, Parkinson's disease, learning and memory impairment, tardive dyskinesia, drug dependence, arteriosclerotic dementia and dementias that accompany Huntington's chorea, Wilson's disease, paralysis agitans, and thalamic atrophies,
■ infection, especially infection by viruses wherein such viruses increase the production of TNF-α in their host, or wherein such viruses are sensitive to upregulation of TNF-α in their host so that their replication or other vital activities are adversely impacted, including a virus which is a member selected from the group consisting of HIV-1, HIV-2, and HIV-3, cytomegalovirus (CMV), influenza, adenoviruses and Herpes viruses including Herpes zoster and Herpes simplex,
■ yeast and fungus infections wherein said yeast and fungi are sensitive to upregulation by TNF-α or elicit TNF-α production in their host, e.g., fungal meningitis, particularly when administered in conjunction with other drugs of choice for the treatment of systemic yeast and fungus infections, including but are not limited to, polymixins, e.g. Polymycin B, imidazoles, e.g. clotrimazole, econazole, miconazole, and ketoconazole, triazoles, e.g. fluconazole and itranazole as well as amphotericins, e.g. Amphotericin B and liposomal Amphotericin B,
■ ischemia-reperfusion injury, ischemic heart disease, autoimmune diabetes, retinal autoimmunity, chronic lymphocytic leukemia, HIV infections, lupus erythematosus, kidney and ureter disease, urogenital and gastrointestinal disorders and prostate diseases,
■ scar formation in the human or animal body, such as scar formation in the healing of acute wounds, and
■ psoriasis, other dermatological and cosmetic uses, including antiphlogistic, skin-softening, skin elasticity and moisture-increasing activities.

13. The use of a compound salt or solvate according to any one of claims 1 to 8 in the manufacture of a medicament for use in the treatment of a disease, disorder, or condition in which PDE4 inhibition is beneficial.

14. The use according to claim 13 wherein the disease, disorder or condition is selected from the list as defined in claim 12.

15. A process to make a compound of formula (I) as defined in claim 1, comprising reaction of a compound of formula (VI) with a reagent of formula Y-Z-R², 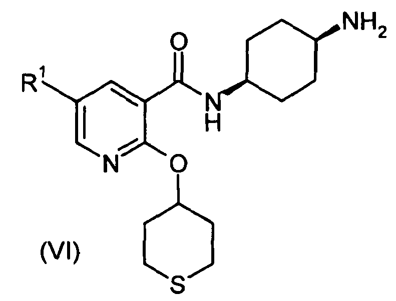 wherein R¹, R² and Z are as defined in claim 1, and Y is a leaving group.

16. A process to make a compound of formula (I) as defined in claim 1, comprising reaction of a compound of formula (IX) 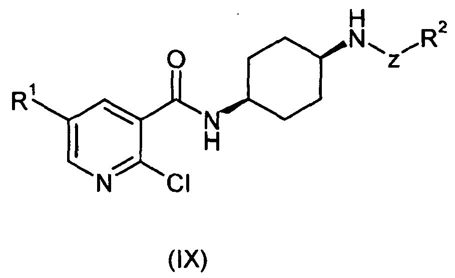 with tetrahydrothiopyran-4-ol.

17. A process to make a compound of formula (I) as defined in claim 1, comprising reaction of a compound of formula (XII) with a compound of formula (VIII) :

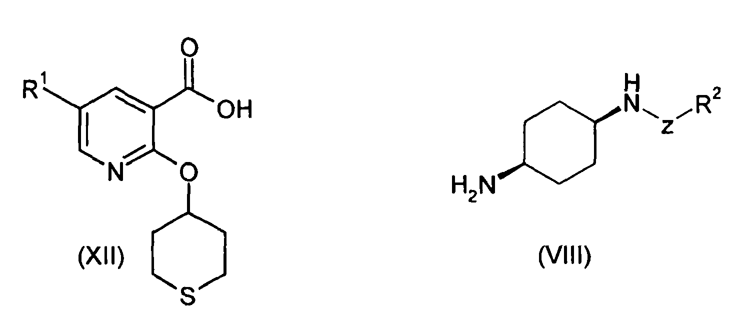

18. A compound of formula (V): 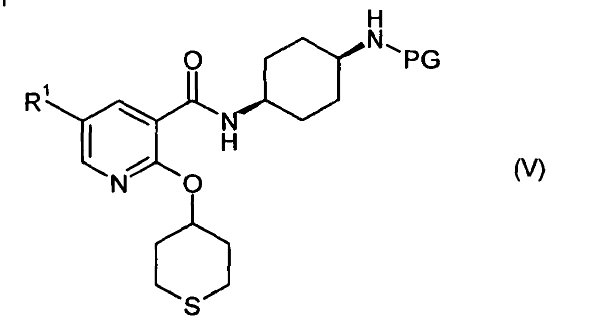 wherein R¹ is as defined in claim 1 and PG is an amine protecting group.

19. A compound of formula (VI): 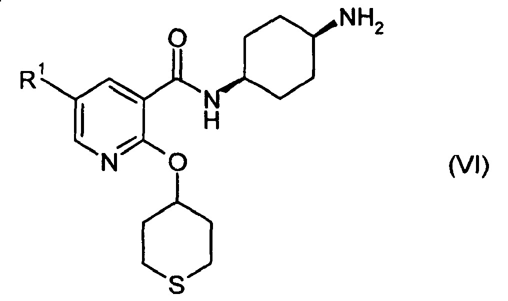 wherein R¹ is as defined in claim 1.

20. A compound of formula (XII) : 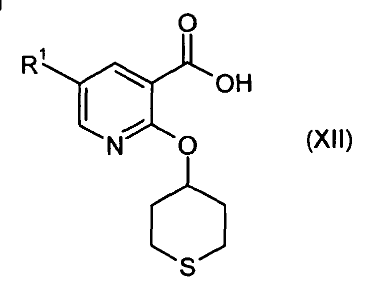 wherein R¹ is as defined in claim 1.

21. A compound of formula (XI) : 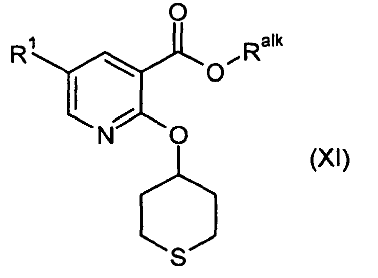 wherein R¹ is as defined in claim 1 and R^{alk} represents a (C₁-C₄)alkyl group.

22. A combination of a compound according to any one of claims 1 to 11 with other therapeutic agents selected from-:
(a) 5-Lipoxygenase (5-LO) inhibitors or 5-lipoxygenase activating protein (FLAP) antagonists,
(b) Leukotriene antagonists (LTRAs) including antagonists of LTB4, LTC4, LTD4, and LTE4,
(c) Histaminic receptor antagonists including H1, H3 and H4 antagonists,
(d) α1- and α2-adrenoceptor agonist vasoconstrictor sympathomimetic agents for decongestant use,
(e) Muscarinic M3 receptor antagonists or anticholinergic agents,
(f) β2-adrenoceptor agonists,
(g) Theophylline,
(h) Sodium cromoglycate,
(i) COX-1 inhibitors (NSAIDs) and COX-2 selective inhibitors,
(j) Oral or inhaled Glucocorticosteroids,
(k) Monoclonal antibodies active against endogenous inflammatory entities,
(l) Anti-tumor necrosis factor (anti-TNF-a) agents,
(m) Adhesion molecule inhibitors including VLA-4 antagonists,
(n) Kinin-B1 - and B2 -receptor antagonists,
(o) Immunosuppressive agents,
(p) Inhibitors of matrix metalloproteases (MMPs),
(q) Tachykinin NK1, NK2 and NK3 receptor antagonists,
(r) Elastase inhibitors,
(s) Adenosine A2a receptor agonists,
(t) Inhibitors of urokinase,
(u) Compounds that act on dopamine receptors, e.g. D2 agonists,
(v) Modulators of the NFkb pathway, e.g. IKK inhibitors,
(w) Agents that can be classed as mucolytics or anti-tussive,
(x) antibiotics, and
(y) p38 MAP kinase inhibitors.

## Patentansprüche

1. Verbindung der Formel (I): 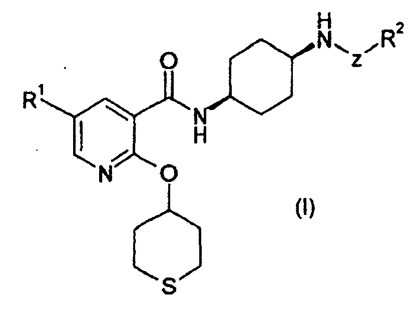 worin:
R¹ aus H, Halogen und (C₁-C₄)Alkyl ausgewählt ist;
Z für eine Verknüpfungsgruppe, die aus CO und SO₂ ausgewählt ist, steht;
R² aus Phenyl, Benzyl, Naphthyl, Heteroaryl und (C₃-C₈)Cycloalkyl ausgewählt ist, die jeweils optional mit 1 bis 3 Substituenten substituiert sind, die jeweils unabhängig voneinander aus Halogen, CN, CONR³R⁴, (C₁-C₆)Alkyl, Halogen (C₁-C₆)alkyl, OH, Hydroxy(C₁-C₆)-alkyl, ((C₃-C₈)Cycloalkyl)-(C₁-C₆)alkyl, Phenyl (das optional mit OH und/oder Halogen substituiert ist), (C₃-C₈)Cycloalkyl und NR³R⁴ ausgewählt sind; und
R³ und R⁴ jeweils unabhängig voneinander aus H, (C₁-C₄)Alkyl und SO₂(C₁-C₄)Alkyl ausgewählt sind;
und pharmazeutisch akzeptable Salze und Solvate derselben.

2. Verbindung, Salz oder Solvat nach Anspruch 1, worin R¹ für H, Halogen, CH₃ oder C₂H₅ steht.

3. Verbindung, Salz oder Solvat nach Anspruch 1 oder Anspruch 2, worin R² für Phenyl, Imidazol, Pyrazin, Indazol, Purin, Chinolin, Chinazolin, Benzofuran, Dihydrobenzofuran, Benzothiadiazol, Benzoxadiazol, Pyrazol, Imidazopyridin, Benzimidazol, Pyrazolopyridin, Pyrazolopyrimidin, Benzyl oder Cyclopropyl steht, die jeweils optional mit 1 bis 3 Substituenten substituiert sind, die jeweils unabhängig voneinander aus Halogen, CN, CONR³R⁴, (C₁-C₆)Alkyl, Halogen (C₁-C₆) alkyl, OH, Hydroxy(C₁-C₆)alkyl, ((C₃-C₈)Cycloalkyl)-(C₁-C₆)alkyl, Phenyl (das optional mit OH und/oder Halogen substituiert ist), (C₃-C₈)Cycloalkyl und NR³R⁴ ausgewählt sind.

4. Verbindung, Salz oder Solvat nach Anspruch 1, 2 oder 3, worin R¹ für H, F, Cl oder CH₃ steht.

5. Verbindung, Salz oder Solvat nach einem der Ansprüche 1, 2, 3 oder 4, worin R² für Phenyl, Imidazol, Indazol, Chinolin, Chinazolin, Dihydrobenzofuran, Benzothiadiazol, Benzoxadiazol, Pyrazol, Imidazopyridin, Benzimidazol, Pyrazolopyridin, Benzyl oder Cyclopropyl steht, die jeweils optional mit einem oder mehreren Substituenten substituiert sind, die aus CH₃, N(CH₃)SO₂CH₃, NHSO₂CH₂CH₃, NHSO₂CH(CH₃)₂, OH, CH₂OH, Cl, F, C₂H₅, CH(CH₃)₂, C₂H₄OH, CF₃ ausgewählt sind.

6. Verbindung, Salz oder Solvat nach einem der Ansprüche 1 bis 5, worin R¹ F ist.

7. Verbindung, Salz oder Solvat nach einem der Ansprüche 1 bis 6, worin Z CO ist.

8. Verbindung nach Anspruch 1, bei der es sich um syn-5-Fluor-N-[4-(2-hydroxy-4-methyl-benzoylamino)-cyclohexyl]-2-(tetrahydro-thiopyran-4-yloxy)-nicotinamid der Formel: 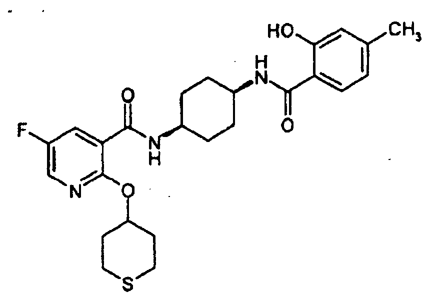 handelt, oder ein pharmazeutisch akzeptables Salz oder Solvat derselben.

9. Pharmazeutische Zusammensetzung, die eine Verbindung nach einem der Ansprüche 1 bis 8 oder ein pharmazeutisch akzeptables Salz oder Solvat derselben und einen pharmazeutisch akzeptablen Träger, ein pharmazeutisch akzeptables Verdünnungsmittel oder Streckmittel umfasst.

10. Verbindung, Salz oder Solvat nach einem der Ansprüche 1 bis 8 oder pharmazeutische Zusammensetzung nach Anspruch 9 zur Verwendung in der Medizin.

11. Verbindung, Salz oder Solvat nach einem der Ansprüche 1 bis 8 oder pharmazeutische Zusammensetzung nach Anspruch 9 zur Verwendung bei der Behandlung einer Erkrankung, Störung oder eines Zustands, wobei eine PDE4-Hemmung vorteilhaft ist.

12. Verbindung nach Anspruch 11, wobei die Erkrankung, Störung oder der Zustand ausgewählt ist aus:
Asthma jedweder Art, Ätiologie oder Pathogenese, insbesondere Asthma, das ein aus der Gruppe von atopischem Asthma, nicht-atopischem Asthma, konstitutionsallergischem Asthma, IgE-vermitteltem konstitutionsallergischem Bronchialasthma, Bronchialasthma, essentiellem/primärem Asthma, primärem/essentiellem Asthma, Intrinsic-Asthma, das durch pathophysiologische Störungen verursacht ist, Extrinsic-Asthma, das durch Umweltfaktoren verursacht ist, essentiellem Asthma unbekannter oder unklarer Ursache, nicht-atopischem Asthma, bronchitischem/katarrhalischem Asthma, emphysematösem Asthma, trainingsinduziertem Asthma, allergieninduziertem Asthma, durch kalte Luft induziertem Asthma, Asthma occupationalis, infektallergischem Asthma, das durch eine Bakterien-, Pilz-, Protozoen- oder Virusinfektion verursacht ist, nichtallergischem Asthma, beginnendem Asthma und Wheezy-Infant-Syndrom ausgewähltes Mitglied ist,
chronischer oder akuter Bronchokonstriktion, chronischer Bronchitis, Obstruktion der kleinen Luftwege und Emphysem,
obstruktiven oder entzündlichen Atemwegserkrankungen jedweder Art, Ätiologie oder Pathogenese, insbesondere einer obstruktiven oder entzündlichen Atemwegserkrankung, die ein aus der Gruppe von eosinophiler Pneumonie, chronischobstruktiver Lungenerkrankung (COPD), COPD, die damit verbundene(s) chronische Bronchitis, Lungenemphysem oder Dyspnoe umfasst, COPD, die durch eine irreversible fortschreitende Atemwegsobstruktion **gekennzeichnet** ist, Schocklunge (ARDS) und Exacerbation von Hyperreaktivität der Atemwege infolge einer anderen Arzneimitteltherapie ausgewähltes Mitglied ist,
Pneumokoniose jedweder Art, Ätiologie oder Pathogenese, insbesondere Pneumokoniose, die ein aus der Gruppe von Aluminose oder Aluminiumstaublunge, Anthrakose oder Asthma bei Anthrakose, Asbestose oder Asthma bei Asbestose, Chalikose oder Kalkstaublunge, Ptilosis, die durch Inhalation des Staubs von Straußenfedern verursacht ist, Siderose, die durch die Inhalation von Eisenpartikeln verursacht ist, Silikose oder Steinstaublunge, Byssinose oder Baumwollpneumokoniose und Talkumpneumokoniose ausgewähltes Mitglied ist,
Bronchitis jedweder Art, Ätiologie oder Pathogenese, insbesondere Bronchit'is, die ein aus der Gruppe von akuter Bronchitis, akuter laryngotrachealer Bronchitis, ArachidicBronchitis, katarrhalischer Bronchitis, kruppöser Bronchitis, trockener Bronchitis, infektiösem Asthma bronchiale, produktiver Bronchitis, Staphylokokken- oder Streptokokkenbronchitis und Vesikulärbronchitis ausgewähltes Mitglied ist,
Bronchiektase jedweder Art, Ätiologie oder Pathogenese, insbesondere Bronchiektase, die ein aus der Gruppe von zylindrischer Bronchiektase, sackförmiger Bronchiektase, fusiformer Bronchiektase, Bronchiolenerweiterung, zystischer Bronchiektase, trockener Bronchiektase und follikulärer Bronchiektase ausgewähltes Mitglied ist,
allergischer saisongebundener Rhinitis oder perennialer allergischer Rhinitis oder Sinusitis jedweder Art, Ätiologie oder Pathogenese, insbesondere Sinusitis, die ein aus der Gruppe von eitriger oder nichteitriger Sinusitis, akuter oder chronischer Sinusitis und Sinusitis ethmoidalis, frontalis, maxillaris oder sphenoidalis ausgewähltes Mitglied ist,
rheumatoider Arthritis jedweder Art, Ätiologie oder Pathogenese, insbesondere rheumatoider Arthritis, die ein aus der Gruppe von akuter Arthritis, akuter Gichtarthritis, progredienter/primär chronischer Polyarthritis, degenerativer Gelenkentzündung, Infektarthritis, Lyme-Krankheit, proliferativer Arthritis, Arthritis psoriatica und Arthritis vertebralis ausgewähltes Mitglied ist, Gicht und Fieber und Schmerz in Verbindung mit einer Entzündung,
einer mit Eosinophilen in Verbindung stehenden Störung jedweder Art, Ätiologie oder Pathogenese, insbesondere einer mit Eosinophilen in Verbindung stehenden Störung, die ein aus der Gruppe von Eosinophilie, eosinophilem Lungeninfiltrat, Löffler-Syndrom, eosinophiler Pneumonie, tropischer pulmonaler Eosinophilie, bronchopulmonaler Aspergillose, Aspergillom, Eosinophile enthaltenden Granulomen, allergischer granulomatöser Angiitis oder Churg-Strauss-Syndrom, Polyarteritis nodosa (PAN) und systemischer nekrotisierender Angiitis/Vaskulitis ausgewähltes Mitglied ist,
atopischer Dermatitis, allergischer Kontaktdermatitis, Kontaktdermatitis oder atopischem/endogenem/exsudativem/neuropathischem/konstitutionellem Enzym,
Urtikaria jedweder Art, Ätiologie oder Pathogenese, insbesondere Urtikaria, die ein aus der Gruppe von immunvermittelter Urtikaria, komplementvermittelter Urtikaria, durch ein Urtikaria hervorrufendes Material induzierter Urtikaria, durch ein physikalisches Mittel induzierter Urtikaria, stressinduzierter Urtikaria, idiopathischer Urtikaria, akuter Urtikaria, chronischer Urtikaria, Angioödem, cholinergischer Urtikaria, Kälteurtikaria in der autosomalen dominanten Form oder in der erworbenen Form, Kontakturtikaria, Quincke-Ödem und Urtikaria papulosa chronica ausgewähltes Mitglied ist,
Konjunktivitis jedweder Art, Ätiologie oder Pathogenese, insbesondere Konjunktivitis, die ein aus der Gruppe von Konjunktivitis actinica, Konjunktivitis acuta, akuter kontagiöser Konjunktivitis, allergischer Konjunktivitis, atopischer Konjunktivitis, chronischer katarrhalischer Konjunktivitis, eitriger Konjunktivitis und Konjunktivitis vernalis ausgewähltes Element ist,
Uveitis jedweder Art, Ätiologie oder Pathogenese, insbesondere Uveitis, die ein aus der Gruppe von einer Entzündung der gesamten oder eines Teils der Uvea, Uveitis anterior, Iritis, Zyklitis, Iridozyklitis, granulomatöser Uveitis, nicht-granulomatöser Uveitis, phakoantigener Uveitis, Uveitis posterior, Choroiditis und Chorioretinitis ausgewähltes Mitglied ist,
multipler Sklerose jedweder Art, Ätiologie oder Pathogenese, insbesondere multipler Sklerose, die ein aus der Gruppe von primärer progressiver multipler Sklerose und rezidivierender multipler Sklerose ausgewähltes Mitglied ist,
Autoimmun/entzündlichen Erkrankungen jedweder Art, Ätiologie oder Pathogenese, insbesondere einer Autoimmun/entzündlichen Erkrankung, die ein aus der Gruppe von hämatologischen Autoimmunerkrankungen, hämolytischer Anämie, aplastischer Anämie, aregenerativer Anämie, idiopathischer thrombozytopenischer Purpura, systemischem Lupus erythematodes, Polychrondritis, Skleroderm, Wegener-Granulomatose, Dermatomyositis, chronischaktiver Hepatitis, Erb-Goldflam-Syndrom, Stevens-Johnson-Syndrom, idiopathischer Sprue, entzündlichen Autoimmundarmerkrankungen, ulzeröser Kolitis, endokriner Ophthalmopathie, Morbus Basedow, Sarkoidose, Alveolitis, Pneumonitis bei chronischer Überempfindlichkeit, primärbiliärer Zirrhose, insulinabhängigem Diabetes oder Typ-I-Diabetes mellitus, Keratokonjunktivitis sicca, Keratokonjunktivitis epidemica, diffuser interstitieller Lungenfibrose oder interstitieller Lungenfibrose, Lungenzirrhose, Muskoviszidose, Glomerulonephritis mit und ohne nephrotischem Syndrom, akuter Glomerulonephritis, idiopathischem nephrotischem Syndrom, Minimal-Change-Nephropathie, entzündlichen/hyperproliferativen Hauterkrankungen, Hailey-Hailey-Syndrom, Pemphigus erythematosus, Pemphigus foliaceus und Pemphigus vulgaris ausgewähltes Mitglied ist,
Allotransplantatabstoßung nach Organtransplantation,
einer entzündlichen Darmerkrankung (IBD) jedweder Art, Ätiologie oder Pathogenese, insbesondere einer entzündlichen Darmerkrankung, die ein aus der Gruppe von Collagenkolitis, Kolitis polyposa, Kolitis regionalis, ulzeröser Kolitis, und Morbus Crohn (CD) ausgewähltes Mitglied ist,
septischem Schock jedweder Art, Ätiologie oder Pathogenese, insbesondere septischem Schock, der ein aus der Gruppe von Nierenversagen, akutem Nierenversagen, Kachexie, chronischer Malaria, Simmonds-Kachexie, urämischer Kachexie, kardialer Kachexie, Kachexie suprarenalis oder Morbus Addison, Kachexie bei Malignomerkrankung und Kachexie infolge einer Infektion durch das Humanimmunschwächevirus (HIV) ausgewähltes Mitglied ist,
Lebertrauma,
pulmonaler Hypertonie jedweder Art, Ätiologie oder Pathogenese, die primäre pulmonale Hypertonie/essentielle Hypertonie, pulmonale Hypertonie infolge dekompensierter Herzinsuffizienz, pulmonale Hypertonie infolge chronischer obstruktiver Lungenerkrankung, Lungenvenenhypertonie, Pulmonalarterienhypertonie und hypoxieinduzierte pulmonale Hypertonie umfasst,
Knochenschwunderkrankungen, primärer Osteoporose und sekundärer Osteoporose,
Störungen des Zentralnervensystems jedweder Art, Ätiologie oder Pathogenese, insbesondere einer Störung des Zentralnervensystems, die ein aus der Gruppe von Depression, Alzheimer-Krankheit, Parkinson-Krankheit, Lern- und Gedächtnisschwäche, Dyskinesia tardive, Drogenabhängigkeit, atherosklerotischer Demenz und Demenzerkrankungen, die Chorea Huntington, Wilson-Krankheit, Paralysis agitans und Thalamusatrophien begleiten, ausgewähltes Mitglied ist,
einer Infektion, insbesondere einer Infektion durch Viren, wobei derartige Viren die Produktion von TNF-α in ihrem Wirt erhöhen oder wobei derartige Viren zur Hochregulierung von TNF-α in ihrem Wirt derart, dass deren Replikation oder andere Vitalaktivitäten nachteilig beeinflusst werden, empfindlich sind, wobei diese ein Virus umfassen, das ein aus der Gruppe von HIV-1, HIV-2 und HIV-3, Cytomegalovirus (CMV), Influenza, Adenoviren und Herpesviren, die Herpes zoster und Herpes simplex umfassen, ausgewähltes Mitglied ist,
Hefe- und Pilzinfektionen, wobei die Hefen und Pilze zur Hochregulation durch TNF-α oder Auslösung einer TNF-α-Produktion in ihrem Wirt empfindlich sind, beispielsweise Pilzmeningitis, insbesondere bei Verabreichung in Verbindung mit anderen Arzneistoffen der Wahl zur Behandlung systemischer Hefe- und Pilzinfektionen, wobei diese, ohne hierauf beschränkt zu sein, Polymixine, beispielsweise Polymycin B, Imidazole, beispielsweise Clotrimazol, Econazol, Miconazol und Ketoconazol, Triazole, beispielsweise Fluconazol und Itranazol, sowie Amphotericine, beispielsweise Amphotericin B und liposomales Amphotericin B, umfassen,
einer Ischämie-Reperfusionsläsion, ischämischen Herzkrankheit, Autoimmundiabetes, retinaler Autoimmunität, chronischer lymphatischer Leukämie, HIV-Infektionen, Lupus erythematosus, Nieren- und Harnleitererkrankung, Urogenital- und Gastrointestinalerkrankungen und Prostataerkrankungen,
Narbenbildung im Menschen- oder Tierkörper, beispielsweise Narbenbildung bei der Heilung akuter Wunden, und
Psoriasis, anderen dermatologischen und kosmetischen Verwendungszwecken, die antiphlogistische, die Haut weich machende, die Hautelastizität und Feuchtigkeit erhöhende Aktivitäten umfassen.

13. Verwendung einer Verbindung, eines Salzes oder Solvats nach einem der Ansprüche 1 bis 8 bei der Herstellung eines Medikaments zur Verwendung bei der Behandlung einer Erkrankung, Störung oder eines Zustands, wobei eine PDE4-Hemmung vorteilhaft ist.

14. Verwendung nach Anspruch 13, wobei die Erkrankung, Störung oder der Zustand aus der in Anspruch 12 angegebenen Liste ausgewählt ist.

15. Verfahren zur Herstellung einer Verbindung der Formel (I) gemäß der Definition in Anspruch 1, das die Umsetzung einer Verbindung der Formel (VI) mit einem Reagens der Formel Y-Z-R² umfasst, 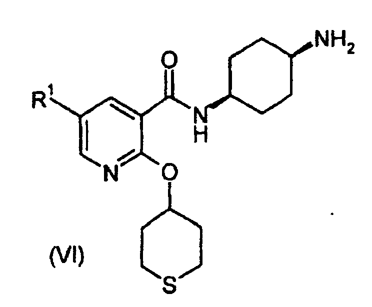 worin R¹, R² und Z wie in Anspruch 1 definiert sind und Y eine Abgangsgruppe ist.

16. Verfahren zur Herstellung einer Verbindung der Formel (I) gemäß der Definition in Anspruch 1, das die Umsetzung einer Verbindung der Formel (IX) 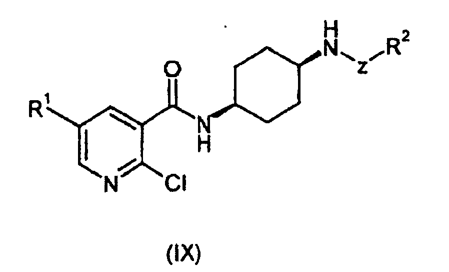 mit Tetrahydrothiopyran-4-ol umfasst.

17. Verfahren zur Herstellung einer Verbindung der Formel (I) gemäß der Definition in Anspruch 1, das die Umsetzung einer Verbindung der Formel (XII) mit einer Verbindung der Formel (VIII) umfasst:

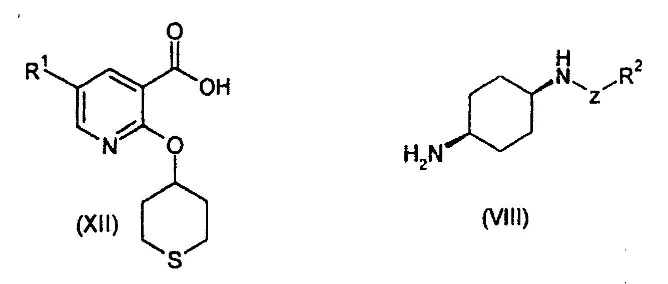

18. Verbindung der Formel (V): 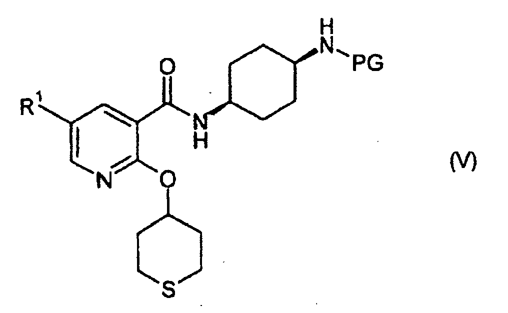 worin R¹ wie in Anspruch 1 definiert ist und PG eine Aminschutzgruppe ist.

19. Verbindung der Formel (VI) 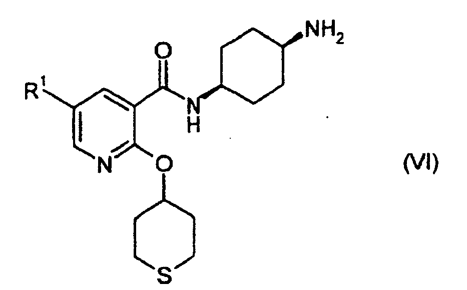 worin R¹ wie in Anspruch 1 definiert ist.

20. Verbindung der Formel (XII) 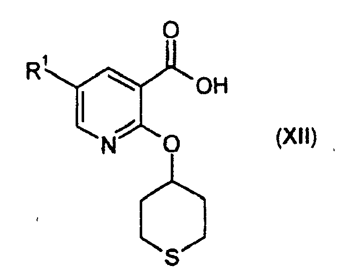 worin R¹ wie in Anspruch 1 definiert ist.

21. Verbindung der Formel (XI) 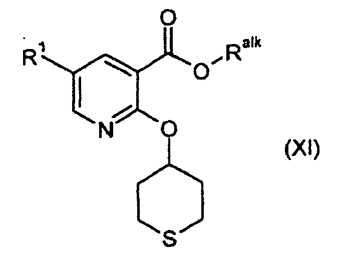 worin R¹ wie in Anspruch 1 definiert ist und R^{alk} für eine (C₁-C₄) Alkylgruppe steht.

22. Kombination von einer Verbindung gemäß einem der Ansprüche 1 bis 11 mit anderen Therapeutika, die ausgewählt sind aus:
(a) 5-Lipoxygenase (5-LO)-Inhibitoren oder Antagonisten des 5-Lipoxygenase Activating Protein (FLAP),
(b) Leukotrienantagonisten (LTRAs), die die Antagonisten von LTB4, LTC4, LTD4 und LTE4 umfassen,
(c) Histaminrezeptorantagonisten, die H1-, H3- und H4-Antagonisten umfassen,
(d) α1- und α2-Adrenozeptoragonist-Vasokonstriktor-Sympathomimetika zur Dekongestionsverwendung,
(e) Muscarin-M3-Rezeptorantagonisten oder Anticholinergika,
(f) β-Adrenozeptoragonisten,
(g) Theophyllin,
(h) Natriumcromoglycat,
(i) COX-1-Inhibitoren (NSAIDs) und COX-2 selektive Inhibitoren,
(j) orale oder inhalierte Glucocorticosteroide,
(k) monoklonale Antikörper, die gegen endogene entzündliche Einheiten aktiv sind,
(l) Anti-Tumornekrosefaktor (anti-TNF-a)-Mittel,
(m) Adhäsionsmolekülinhibitoren, die VLA-4-Antagonisten umfassen,
(n) Kinin-B1- und -B2-Rezeptorantagonisten,
(o) Immunsuppresiva,
(p) Inhibitoren von Matrixmetalloproteasen (MMPs),
(q) Tachykinin-NK1-, -NK2- und -NK3-Rezeptorantagonisten,
(r) Elastaseinhibitoren,
(s) Adenosin-A2a-Rezeptoragonisten,
(t) Inhibitoren von Urokinase,
(u) Verbindungen, die auf Dopaminrezeptoren wirken, beispielsweise D2-Agonisten,
(v) Modulatoren des NFkb-Pfades, beispielsweise IKK-Inhibitoren,
(w) Mittel, die als Mukolytika oder Antitussiva klassifiziert werden können,
(x) Antibiotika und
(y) p38-MAP-Kinaseinhibitoren.

## Revendications

1. Composé de formule (I) : 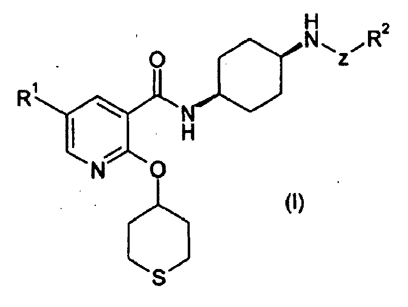 dans laquelle :
R¹ est sélectionné parmi H, halogéno et alkyle en C₁-C₄ ;
Z est un groupe de liaison sélectionné parmi CO et SO₂ ;
R² est sélectionné parmi phényle, benzyle, naphtyle, hétéroaryle et cycloalkyle en C₃-C₈,
chacun de ceux-ci étant facultativement substitué par de 1 à 3 substituants qui sont chacun indépendamment sélectionnés parmi halogéno, CN, CONR³R⁴, alkyle en C₁-C₆, halogéno(alkyle en C₁-C₆), OH, hydroxy(alkyle en C₁-C₆), (cycloalkyle en C₃-C₈)-(alkyle en C₁-C₆), phényle (facultativement substitué par OH et/ou halogéno), cycloalkyle en C₃-C₈ et NR³R⁴ ; R³ et R⁴ étant chacun indépendamment sélectionnés parmi H, alkyle en C₁-C₄ et SO₂ (alkyle en C₁-C₄) ;
et sels et solvates pharmaceutiquement acceptables correspondants.

2. Composé, sel ou solvate selon la revendication 1, dans lequel R¹ représente H, halogéno, CH₃ ou C₂H₅.

3. Composé, sel ou solvate selon la revendication 1 ou la revendication 2, dans lequel R² représente phényle, imidazole, pyrazine, indazole, purine, quinoline, quinazoline, benzofurane, dihydrobenzofurane, benzothiadiazole, benzoxadiazole, pyrazole, imidazopyridine, benzimidazole, pyrazolopyridine, pyrazolopyrimidine, benzyle ou cyclopropyle,
chacun de ceux-ci étant facultativement substitué par de 1 à 3 substituants qui sont chacun indépendamment sélectionnés parmi halogéno, CN, CONR³R⁴, alkyle en C₁-C₆, halogéno(alkyle en C₁-C₆), OH, hydroxy(alkyle en C₁-C₆), (cycloalkyle en C₃-C₈)-(alkyle en C₁-C₆), phényle (facultativement substitué par OH et/ou halogéno), cycloalkyle en C₃-C₈ et NR³R⁴.

4. Composé, sel ou solvate selon la revendication 1, 2 ou 3, dans lequel R¹ représente H, F, Cl ou CH₃.

5. Composé, sel ou solvate selon l'une quelconque des revendications 1, 2, 3 ou 4, dans lequel R² représente phényle, imidazole, indazole, quinoline, quinazoline, dihydrobenzofurane, benzothiadiazole, benzoxadiazole, pyrazole, imidazopyridine, benzimidazole, pyrazolopyridine, benzyle ou cyclopropyle,
chacun de ceux-ci étant facultativement substitué par un ou plusieurs substituants sélectionnés parmi CH₃, N(CH₃)SO₂CH₃, NHSO₂CH₂CH₃, NHSO₂CH(CH₃)₂, OH, CH₂OH, Cl, F, C₂H₅, CH(CH₃)₂, C₂H₄OH, CF₃.

6. Composé, sel ou solvate selon l'une quelconque des revendications 1 à 5, dans lequel R¹ représente F.

7. Composé, sel ou solvate selon l'une quelconque des revendications 1 à 6, dans lequel Z représente CO.

8. Composé selon la revendication 1, qui est le syn-5-fluoro-N-[4-(2-hydroxy-4-méthyl-benzoylamino)-cyclohexyl]-2-(tétrahydro-thiopyran-4-yloxy)-nicotinamide de formule : 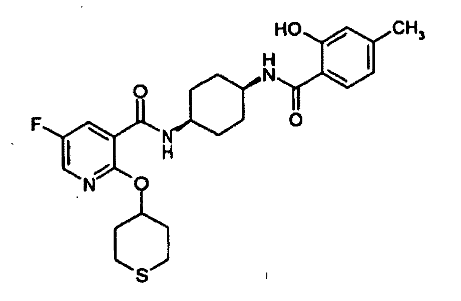 ou sel ou solvate pharmaceutiquement acceptable correspondant.

9. Composition pharmaceutique comprenant un composé selon l'une quelconque des revendications 1 à 8, ou un sel ou solvate pharmaceutiquement acceptable correspondant, et un support, diluant ou excipient pharmaceutiquement acceptable.

10. Composé, sel ou solvate selon l'une quelconque des revendications 1 à 8, ou composition pharmaceutique selon la revendication 9, pour une utilisation en médecine.

11. Composé, sel ou solvate selon l'une quelconque des revendications 1 à 8, ou composition pharmaceutique selon la revendication 9, pour une utilisation dans le traitement d'une maladie, d'un trouble ou d'un état dans laquelle/lequel l'inhibition de la PDE4 est bénéfique.

12. Composé selon la revendication 11, dans lequel la maladie, le trouble ou l'état est sélectionné(e) parmi :
• l'asthme quels qu'en soient le type, l'étiologie ou la pathogénèse, en particulier, un asthme qui fait partie du groupe consistant en l'asthme atopique, l'asthme non-atopique, l'asthme allergique, l'asthme bronchique atopique induit par IgE, l'asthme bronchique, l'asthme essentiel, l'asthme vrai, l'asthme intrinsèque causé par des troubles pathophysiologiques, l'asthme extrinsèque causé par des facteurs environnementaux, l'asthme essentiel sans cause apparente ou de cause inconnue, l'asthme non-atopique, l'asthme bronchitique, l'asthme emphysémateux, l'asthme induit par l'exercice, l'asthme induit par les allergènes, l'asthme induit par l'air froid, l'asthme occupationnel, l'asthme infectieux causé par une infection bactérienne, fongique, à protozoaires ou virale, l'asthme non-allergique, l'asthme du nourrisson et le syndrome asthmatique du nourrisson,
• la bronchoconstriction chronique ou aiguë, la bronchite chronique, l'obstruction des petites voies respiratoires et l'emphysème,
• les maladies des voies respiratoires obstructives ou inflammatoires quels qu'en soient le type, l'étiologie ou la pathogénèse, en particulier, une maladie des voies respiratoires obstructive ou inflammatoire qui fait partie du groupe consistant en la pneumonie à éosinophiles chronique, la maladie pulmonaire obstructive chronique (COPD), la COPD y compris la bronchite chronique, l'emphysème pulmonaire ou la dyspnée qui leur est associée, la COPD qui est **caractérisée par** l'obstruction progressive et irréversible des voies respiratoires, le syndrome de détresse respiratoire de l'adulte (ARDS) et l'aggravation de l'hyper-réactivité des voies respiratoires à la suite d'une autre thérapie médicamenteuse,
• la pneumoconiose quels qu'en soient le type, l'étiologie ou la pathogénèse, en particulier, une pneumoconiose qui fait partie du groupe consistant en l'aluminose ou maladie des travailleurs de la bauxite, l'anthracose ou asthme des mineurs, l'asbestose ou asthme des installateurs de conduites de vapeur, la chalicose ou maladie des tailleurs de pierre, la ptilose provoquée par l'inhalation de poussière de plumes d'autruche, la sidérose provoquée par l'inhalation de particules de fer, la silicose ou maladie des rémouleurs, la byssinose ou asthme de la poussière de coton et la pneumoconiose due au talc ;
• la bronchite quels qu'en soient le type, l'étiologie ou la pathogénèse, en particulier, une bronchite qui fait partie du groupe consistant en la bronchite aiguë, la bronchite aiguë laryngotrachéenne, la bronchite d'allergie aux arachides, la bronchite catarrhale, la bronchite de type croup, la bronchite sèche, la bronchite asthmatique infectieuse, la bronchite à toux productive, la bronchite à staphylocoques ou streptocoques et la bronchite à murmure vésiculaire modifié,
• la bronchiectasie quels qu'en soient le type, l'étiologie ou la pathogénèse, en particulier une bronchiectasie qui fait partie du groupe consistant en la bronchiectasie cylindrique, la bronchiectasie sacculiforme, la bronchiectasie fusiforme, la bronchiectasie capillaire, la bronchiectasie kystique, la bronchiectasie sèche et la bronchiectasie folliculaire,
• la rhinite allergique saisonnière ou la rhinite allergique pérenne ou la sinusite quels qu'en soient le type, l'étiologie ou la pathogénèse, en particulier une sinusite qui fait partie du groupe consistant en la sinusite purulente ou non purulente, la sinusite aiguë ou chronique et la sinusite ethmoïde, frontale, maxillaire ou sphénoïde,
• l'arthrite rhumatoïde quels qu'en soient le type, l'étiologie ou la pathogénèse, en particulier une arthrite rhumatoïde qui fait partie du groupe consistant en l'arthrite aiguë, l'arthrite aiguë goutteuse, l'arthrite inflammatoire chronique, l'arthrite dégénérative, l'arthrite infectieuse, l'arthrite de Lyme, l'arthrite proliférative, l'arthrite psoriatique, et l'arthrite vertébrale,
• la goutte ainsi que la fièvre et la douleur associées à l'inflammation,
• les troubles liés aux éosinophiles quels qu'en soient le type, l'étiologie ou la pathogénèse, en particulier un trouble lié aux éosinophiles qui fait partie du groupe consistant en l'éosinophilie, l'éosinophilie à infiltration pulmonaire, le syndrome de Loffler, la pneumonie éosinophile chronique, l'éosinophilie pulmonaire tropicale, l'aspergillose bronchopneumonique, l'aspergillome, les éosinophiles contenant des granulomes, l'angéite granulomateuse allergique ou syndrome de Churg-Strauss, la polyartérite noueuse (PAN) et la vasculite nécrosante systémique,
• la dermatite atopique, la dermatite allergique, la dermatite de contact ou l'eczéma allergique ou atopique,
• l'urticaire quels qu'en soient le type, l'étiologie ou la pathogénèse, en particulier une urticaire qui fait partie du groupe consistant en l'urticaire immunoinduite, l'urticaire induite par un complément, l'urticaire induite par un matériau urticariogène, l'urticaire induite par un agent physique, l'urticaire induite par le stress, l'urticaire idiopathique, l'urticaire aiguë, l'urticaire chronique, l'angioedème, l'urticaire cholinergique, l'urticaire froide dans la forme dominante autosome ou la forme acquise, l'urticaire de contact, l'urticaire géante ou l'urticaire papulaire,
• la conjonctivite quels qu'en soient le type, l'étiologie ou la pathogénèse, en particulier une conjonctivite qui fait partie du groupe consistant en la conjonctivite actinique, la conjonctivite catarrhale aiguë, la conjonctivite contagieuse aiguë, la conjonctivite allergique, la conjonctivite atopique, la conjonctivite catarrhale chronique, la conjonctivite purulente et la conjonctivite vernale,
• l'uvéite quels qu'en soient le type, l'étiologie ou la pathogénèse, en particulier une uvéite qui fait partie du groupe consistant en l'inflammation de tout ou partie de l'uvée, l'uvéite antérieure, l'iritite, la cyclite, l'iridocyclite, l'uvéite granulomateuse, l'uvéite non granulomateuse, l'uvéite phacoantigénique, l'uvéite postérieure, la choroïdite et la choriorétinite,
• la sclérose en plaques quels qu'en soient le type, l'étiologie ou la pathogénèse, en particulier une sclérose en plaques qui fait partie du groupe consistant en la sclérose en plaques progressive primaire et la sclérose en plaques à poussées/rémissions,
• les maladies auto-immunes/inflammatoires quels qu'en soient le type, l'étiologie ou la pathogénèse, en particulier une maladie auto-immune/inflammatoire qui fait partie du groupe consistant en les troubles hématologiques auto-immuns, l'anémie hémolytique, l'anémie aplastique, la dysérythropoïèse pure, le purpura thrombocytopénique idiopathique, le lupus érythémateux systémique, la polychondritite, la sclérodermie, la granulomatose de Wagner, la dermatomyosite, l'hépatite active chronique, la myasthénie grave, le syndrome de Stevens-Johnson, la sprue idiopathique, les maladies inflammatoires de l'intestin auto-immunes, la colite ulcérante, l'ophtalmopathie endocrine, la maladie de Grave, la sarcoïdose, l'alvéolite, la pneumonite chronique d'hypersensibilité, la cirrhose biliaire primaire, le diabète juvénile ou le diabète sucré de type 1, la kératoconjonctivite sèche, la kératoconjonctivite épidémique, la fibrose pulmonaire interstitielle diffuse ou la fibrose pulmonaire interstitielle, la fibrose pulmonaire idiopathique, la fibrose kystique, la glomérulonéphrite avec ou sans syndrome néphrotique, la glomérulonéphrite aiguë, le syndrome néphrotique idiopathique, la néphropathie à changement minimal, les maladies de la peau inflammatoires/hyperprolifératives, le pemphigus familial bénin, le pemphigus érythémateux, le pemphigus foliacé et le pemphigus vulgaris,
• le rejet de greffon allogène à la suite d'une transplantation d'organe,
• la maladie inflammatoire de l'intestin (IBD) quels qu'en soient le type, l'étiologie ou la pathogénèse, en particulier une maladie inflammatoire de l'intestin qui fait partie du groupe consistant en la colite collagéneuse, la colite polypeuse, la colite transmurale, la colite ulcérante et la maladie de Crohn (CD),
• le choc septique quels qu'en soient le type, l'étiologie ou la pathogénèse, en particulier un choc septique qui fait partie du groupe consistant en la défaillance rénale, la défaillance rénale aiguë, la cachexie, la cachexie malariale, la cachexie hypophysaire, la cachexie urémique, la cachexie cardiaque, la cachexie surrénale ou maladie d'Addison, la cachexie cancéreuse ou la cachexie en tant que conséquence de l'infection par le virus d'immunodéficience humaine (HIV),
• une lésion du foie,
• l'hypertension pulmonaire quels qu'en soient le type, l'étiologie ou la pathogénèse, en particulier l'hypertension pulmonaire primaire/l'hypertension essentielle, l'hypertension pulmonaire secondaire à une défaillance cardiaque congestive, l'hypertension pulmonaire secondaire à une maladie pulmonaire obstructive chronique, l'hypertension pulmonaire veineuse, l'hypertension pulmonaire artérielle et l'hypertension pulmonaire induite par une hypoxie,
• les maladies de perte osseuse, l'ostéoporose primaire et l'ostéoporose secondaire,
• les troubles du système nerveux central quels qu'en soient le type, l'étiologie ou la pathogénèse, en particulier un trouble du système nerveux central qui fait partie du groupe consistant en la dépression, la maladie d'Alzheimer, la maladie de Parkinson, l'altération des capacités d'apprentissage et de la mémoire, la dyskinésie tardive, la dépendance aux médicaments, la démence artériosclérotique, et les démences qui accompagnent la chorée de Huntington, la maladie de Wilson, la paralysie agitante et les atrophies thalamiques,
• l'infection, en particulier les infections causées par des virus dans lesquelles de tels virus augmentent la production de TNF-α chez leur hôte ou dans lesquelles de tels virus sont sensibles à une sur-régulation du TNF-α chez leur hôte de sorte que leur réplication ou autre activité vitale est affectée négativement, y compris un virus qui est un qui fait partie du groupe consistant en le HIV-1, le HIV-2 et le HIV-3, le cytomégalovirus (CMV), le virus de la grippe, les adénovirus et les virus Herpès, y compris Herpès zoster et Herpès simplex,
• les infections à levures et champignons, dans lesquelles lesdites levures et champignons sont sensibles à' une sur-régulation par le TNF-α ou provoquent une production de TNF-α chez leur hôte, par exemple la méningite fongique, en particulier en cas d'administration conjointement avec d'autres médicaments de choix pour le traitement d'infections systémiques à levures et champignons, y compris, sans y être limités, les polymixines, par exemple la Polymicine B, les imidazoles, par exemple le clotrimazole, l'éconazole, le miconazole et le kétoconazole, les triazoles, par exemple le fluconazole et l'itranazole, ainsi que les amphotéricines, par exemple l'amphotéricine B et l'amphotéricine B liposomique,
• les lésions de reperfusion d'ischémie, la maladie cardiaque ischémique, le diabète auto-immun, l'auto-immunité rétinienne, la leucémie lymphocytique chronique, les infections à HIV, le lupus érythémateux, les maladies des reins et de l'uretère, les troubles urogénitaux et gastro-intestinaux et les maladies de la prostate,
• la cicatrisation du corps humain ou animal, telle que la cicatrisation dans la guérison de plaies aiguës, et
• le psoriasis, d'autres utilisations dermatologiques et cosmétiques, y compris les activités antiphlogistiques, d'assouplissement de la peau, et d'augmentation de l'élasticité et de l'humidité de la peau.

13. Utilisation d'un composé, sel ou solvate selon l'une quelconque des revendications 1 à 8, dans la fabrication d'un médicament pour le traitement d'une maladie, d'un trouble ou d'un état dans laquelle/lequel l'inhibition de la PDE4 est bénéfique.

14. Utilisation selon la revendication 13, dans laquelle la maladie, le trouble ou l'état est sélectionné(e) dans la liste telle que définie dans la revendication 12.

15. Procédé de fabrication d'un composé de formule (I) tel que défini dans la revendication 1, comprenant la réaction d'un composé de formule (VI) 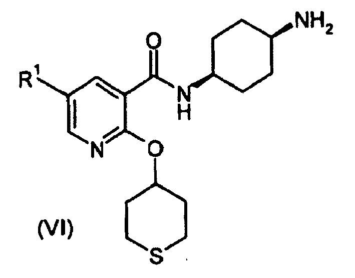 avec un réactif de formule Y-Z-R²,
formules dans lesquelles R¹, R² et Z sont tels que définis dans la revendication 1, et Y est un groupe labile.

16. Procédé de fabrication d'un composé de formule (I) tel que défini dans la revendication 1, comprenant la réaction d'un composé de formule (IX) 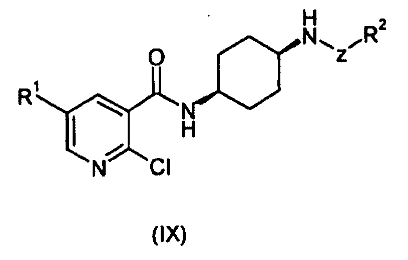 avec le tétrahydrothiopyran-4-ol.

17. Procédé de fabrication d'un composé de formule (I) tel que défini dans la revendication 1, comprenant la réaction d'un composé de formule (XII) avec un composé de formule (VIII) :

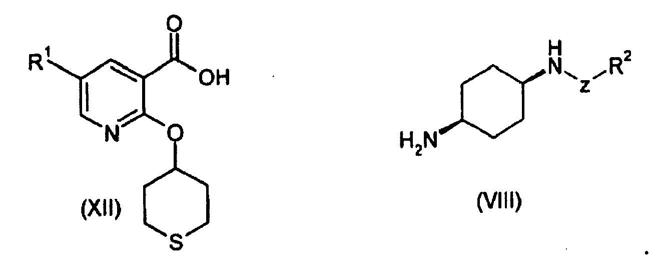

18. Composé de formule (V) : 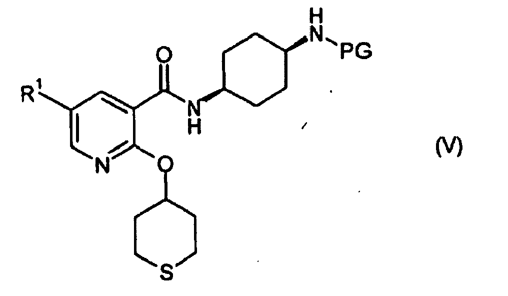 dans laquelle R¹ est tel que défini dans la revendication 1 et PG est un groupe protecteur de l'amine.

19. Composé de formule (VI) : 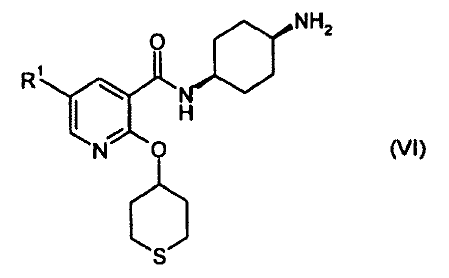 dans laquelle R¹ est tel que défini dans la revendication 1.

20. Composé de formule (XII) : 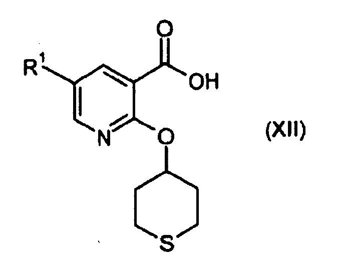 dans laquelle R¹ est tel que défini dans la revendication 1.

21. Composé de formule (XI) : 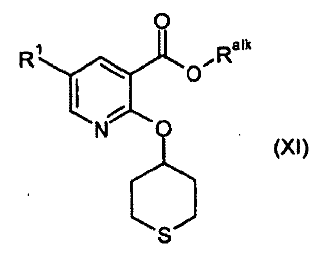 dans laquelle R¹ est tel que défini dans la revendication 1 et R^{alk} représente un groupe alkyle en C₁-C₄.

22. Combinaison d'un composé selon l'une quelconque des revendications 1 à 11 avec d'autres agents thérapeutiques sélectionnés parmi :
(a) les inhibiteurs de la 5-lipoxygénase (5-LO) ou les antagonistes de la protéine activant la 5-lipoxygénase (FLAP),
(b) les antagonistes de leucotriènes (LTRA), y compris les antagonistes de LTB4, LTC4, LTD4 et LTE4,
(c) les antagonistes de récepteurs histaminiques, y compris les antagonistes de H1, H3 et H4,
(d) les agents sympathomimétiques vasoconstricteurs agonistes des adrénocepteurs α1 et α2 pour une utilisation décongestionnante,
(e) les antagonistes du récepteur muscarinique M3 ou les agents anticholinergiques,
(f) les agonistes de l'adrénocepteur β2,
(g) la théophylline,
(h) le cromoglycate de sodium,
(i) les inhibiteurs de la COX-1 (NSAID) et les inhibiteurs sélectifs de la COX-2,
(j) les glucocorticostéroïdes oraux ou inhalés,
(k) les anticorps monoclonaux actifs contre les entités inflammatoires endogènes,
(l) les agents anti-facteur de nécrose tumorale (anti-TNF-a),
(m) les inhibiteurs de molécules d'adhésion, y compris les antagonistes de VLA-4,
(n) les antagonistes des récepteurs de la kinine B1 et B2,
(o) les agents immunosuppresseurs,
(p) les inhibiteurs de métalloprotéases de la matrice (MMP),
(q) les antagonistes des récepteurs de la tachykinine NK1, NK2 et NK3,
(r) les inhibiteurs d'élastase,
(s) les agonistes du récepteur de l'adénosine A2a,
(t) les inhibiteurs de l'urokinase,
(u) les composés qui agissent sur les récepteurs de la dopamine, par exemple les agonistes D2,
(v) les modulateurs de la voie NFkb, par exemple les inhibiteurs d'IKK,
(w) les agents qui peuvent être classés comme mucolytiques ou antitussifs,
(x) les antibiotiques et
(y) les inhibiteurs de la p38 MAP kinase.
